# EUROPÄISCHE PATENTSCHRIFT

(11) **EP 2 843 360 B1**
(45) Veröffentlichungstag und Bekanntmachung des Hinweises auf die Patenterteilung: **11.05.2022**
(21) Anmeldenummer: 14003048.7
(22) Anmeldetag: 03.09.2014
(51) Int. Cl.: G01B 11/24, G01B 9/02, A61B 5/00

(54) **Robustes One-Shot-Interferometer und OCT- Verfahren, insbesondere zur Materialmessung und auch Tumorzellen-Erkennung**
Robust one shot interferometer and OCT method, in particular for material measurement and tumour cell detection
Interféromètre à mission unique robuste et procédé OCT, en particulier pour mesurer le matériau et également de détection de cellules tumorales

(30) Priorität: 03.09.2013 DE 102013015031; 02.10.2013 DE 102013016752
(43) Veröffentlichungstag der Anmeldung: 04.03.2015
(73) Patentinhaber: Universität Stuttgart, 70174 Stuttgart (DE)
(72) Erfinder: Körner, Klaus, 10367 Berlin (DE); Osten, Wolfgang, 70569 Stuttgart (DE)
(74) Vertreter: Müller-Boré & Partner Patentanwälte PartG mbB

(56) Entgegenhaltungen:
- EP-A1- 2 447 663
- DE-B3-102010 006 239
- DE-B4-102010 046 907

## Beschreibung

Die vorliegende Erfindung betrifft ein Verfahren und eine Anordnung zur robusten One-shot-Interferometrie, insbesondere zur optischen Kohärenz-Tomografie nach dem Spatial-domain-Ansatz (SD-OCT) und/oder nach dem Lichtfeld-Ansatz. Das Verfahren und die Anordnung können zur Messung an Material und lebendem Gewebe, zur Abstandsmessung, zur 2D- oder 3D-Messung mit einer feinstrukturierten, auf das Objekt beugungsbegrenzt abgebildeten Lichtquelle oder Spots derselben eingesetzt werden.

In der Offenlegungsschrift DE 10 2006 015 387 A1 [1] von M. Hering u. a. ist eine interferometrische Messvorrichtung auf der Basis der Weißlicht-Interferometrie, auch als Kurzkohärenz- Interferometrie bekannt, beschrieben, bei der die Wellenfronten des reflektierten Objektstrahls und die des reflektierten Referenzstrahls mittels einer Neigungsvorrichtung um einen bestimmten Winkelbetrag zueinander geneigt sind, so dass ein räumliches Interferogramm als Single-Shot-Datensatz entstehen kann. Beispielsweise ist dieser Winkelbetrag hier in einer stark modifizierten Linnik-Interferometer-Anordnung, die auch Merkmale eines Mach-Zehnder-Interferometers aufweist, durch einen Kippspiegel, der vom Licht auf dem Weg zur Detektion nur einmalig passiert wird, realisiert. Mit dieser optischen Anordnung können ein oder mehrere räumliche Interferogramme, auch als Linienstapel auf einer Matrixkamera, als Single-Shot-Datensätze in der Zeitdauer einer Bildaufnahme vollständig zur Verfügung gestellt werden.

Von besonderem Vorteil ist bei diesem Ansatz, dass die Ortsfrequenz für die Schwerpunktwellenlänge, beziehungsweise die Schwerpunktwellenzahl, im räumlichen Interferogramm am Ausgang des Interferometers in erster Näherung nicht von der Neigung der Objektoberfläche in Relation zum Interferometer beeinflusst wird.

Eine gezielte Veränderung des Winkelbetrages mittels Neigungsvorrichtung, beispielsweise um die Ortsfrequenz für die Schwerpunktwellenlänge im räumlichen Interferogramm vorbestimmt zu verändern, kann zu einem unerwünschten Lateralversatz von Objektwellenfront und Referenzwellenfront bei der Detektion führen, der nur aufwendig oder durch eine Justierung in einigen Fällen gar nicht zu kompensieren ist und eine Quelle von Messfehlern darstellen oder den Tiefenmessbereich erheblich einschränken kann. Das Interferometer ist in der Regel nicht sehr langzeitstabil.

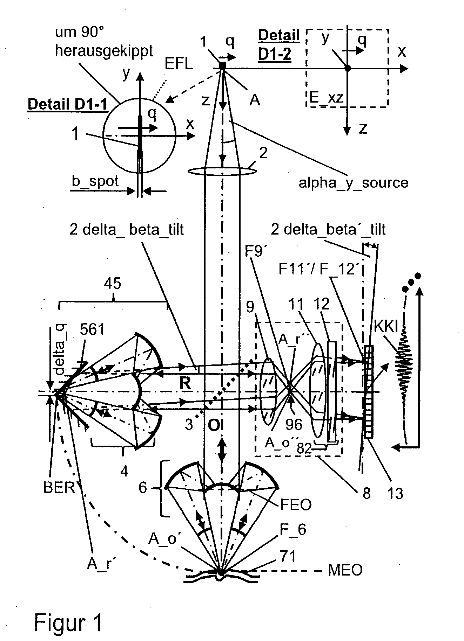

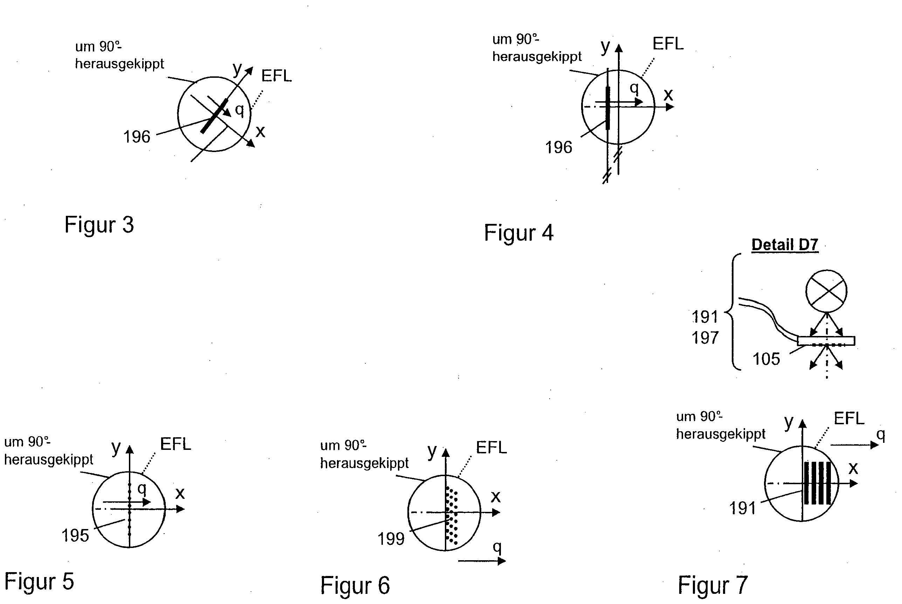

In der Veröffentlichung von M. Hering u. a. in Applied Optics, Vol. 48, Nummer 3, Seite 525 bis 538 vom 20. Januar 2009 [2] zeigen die gemessenen räumlichen Interferogramme in Abbildung 3 das Potenzial dieses Ansatzes gemäß [1]. Die in der Abbildung 1 präsentierte One-Shot-Interferometer-Messanordnung stellt einen Experimentalaufbau zu Studienzwecken dar und ist für die wirtschaftliche Umsetzung eher zu komplex und zu voluminös. Typische Messergebnisse auf der Grundlage dieses Ansatzes wurden von M. Hering u. a. bereits im Jahr 2006 in den Proceedings of SPIE, Vol. 6188, 61880E-1 bis 61880E-11 in der Figur 7 dargestellt [3].Dagegen sind Michelson-Typ-Interferometer mit einem planem Referenzspiegel, bei denen die Ortsfrequenz für die Schwerpunktwellenlänge in einem räumlichen Interferogramm am Ausgang des Interferometers durch Kippung des Referenzspiegels oder durch Kippung des Objekts in Bezug zum Interferometer oder des Interferometers in Bezug zum Objekt zu verändern ist, für Messobjekte mit variierender und unbekannter Oberflächenneigung hinsichtlich der Auswertung von räumlichen Interferogrammen hier überhaupt nicht von Interesse. Deshalb werden Ansätze auf dieser Grundlage nicht als Stand der Technik in Bezug zu dieser Erfindung angesehen und werden deshalb hier auch nicht weiter betrachtet.

Das Gewinnen räumlicher Interferogramme für die One-Shot-Messtechnik mittels Lateral-Shear zwischen Objekt- und Referenzwellenfronten am Ausgang eines Zweistrahl-Interferometers stellt grundsätzlich eine weitere Möglichkeit der Erzeugung räumlicher Interferogramme für die One-Shot-Messtechnik, beispielsweise zur Erfassung des Abstands, dar. Denn Lateral-Shear kann in einer optischen Anordnung als Grundlage zur Generierung von Interferenzen zueinander geneigter Wellenfronten genutzt werden. Einen ganz klassischen Ansatz stellt dafür eine Michelson-Interferometer-Anordnung mit zwei Dachkant-Reflektoren und einer lateral ausgedehnten Lichtquelle dar, um die erforderliche Lateral-Shear zu erzeugen. Dieser Ansatz mit zwei Dachkant-Reflektoren ist den Fachleuten gut bekannt, s. a. D. Malacara, Optical Shop Testing, John Wiley & Sons, Inc., 1992, S. 140 -141, Figur 4.16 [4] und auch W. H. Steel, Interferometry, Cambridge University Press, 1967, S. 83 letzter Absatz bis S. 84 oben [5]. Um diesen Interferometer-Ansatz mit zwei Dachkant-Reflektoren für die Abstandsmessung und Profilmessung nutzen zu können, muss demzufolge im Objektarm des Interferometers der Objektoberfläche ein zusätzlicher Planspiegel zugeordnet werden, wobei dieser Planspiegel mit der Objektoberfläche dann eine Dachkante bildet. Im Referenzarm ist hierbei der zweite Dachkantreflektor angeordnet. Diese Anordnung liefert bei entsprechender Justierung eine Lateral-Shear zwischen den Wellenfronten und vermeidet dabei eine Wellenfront-Inversion, weist aber in der Regel eindeutige Nachteile wegen des benötigten Bauvolumens im Objektarm auf, beispielsweise bei der Messung in Innenräumen.

Bekannt ist auch der von D. Kelsall im Jahr 1959 in Proc. Phys. Society, 73, S. 470, Fig. 1 [6] veröffentlichte Ansatz mit zwei Tripelreflektoren als Endreflektoren eines Michelson-Interferometers. Die Querverschiebung eines Tripelreflektors erzeugt ebenfalls eine Lateral-Shear zwischen Objekt- und Referenzwellenfronten am Ausgang eines Michelson-Interferometers. Der Einsatz eines Tripelreflektors im Referenzarm eines Michelson-Interferometers geht ja nach bestem Wissen jedoch bereits auf F. Twyman und A. Green zurück, s. a. US-Patent 1 565 533, Figur 6, [7] zurück.

Diesen Interferometer-Ansatz als Anordnung für einen interferometrischen Sensor zur Abstandsmessung u. a. zu nutzen, bei dem ein Planspiegel des Tripelreflektors in Form einer Würfelecke, auch als Corner Cube bekannt, durch die Objektoberfläche ersetzt ist, führt dazu, dem Objekt oder der Objektoberfläche im Objektarm noch einen Dachkant-Reflektor oder zwei Planspiegel beiordnen zu müssen, da die unerwünschte Wellenfrontinversion zwischen Objekt- und Referenzstrahlung für eine flächenhafte Messung vermieden werden muss, da sonst der Interferenzkontrast null ist. Dieser Ansatz vergrößert das Sensorvolumen ganz erheblich, was für viele Applikationen sehr nachteilig ist oder dies schließt den Einsatz einer derartigen Lösung völlig aus.

In der Schrift DE 10 2010 006 239 B3 [8] ist ein Ansatz beschriebenen, bei dem aufgrund des Einsatzes eines Dreifach-Spiegels als Endspiegel im Referenzarm des Interferometers der Nachteil besteht, dass kein fokussiertes Bündel mit einer sehr hohen numerischen Apertur über diesen Referenzarm geführt werden kann, wodurch ggf. der Messbereich begrenzt sein kann, da bei hoher NA, z.B. oberhalb von 0,7, das vom Referenzarm zurückkommende Licht im Aperturwinkel etwas limitiert ist und somit nicht das volle Winkelspektrum im Vergleich zum Objektlicht aufweisen kann. Das kann den lateralen Bereich des Entstehens von auswertbaren Interferenzen auf einem Kamera-Chip merklich einschränken.

Die Verfahren und Anordnung nach DE 10 2010 046 907 B4 [9] haben den Vorteil, einen zweiten Interferometerausgang nutzen zu können und die Objektpunkte können nahezu lateral beliebig angeordnet werden. Anordnungen nach [9] sind aufgrund des Tripelreflektors im Referenzarm hochstabil hinsichtlich des Interferometer-Justierzustandes, aber in der optischen Schaltung auch recht aufwendig. Darüber hinaus kann es bei diesem Verfahren bei einem ausgedehnten Messfeld Probleme mit einem sphärischen Phasenterm im Wavelet geben, da die effektiven Spiegelebenen im Referenz- und Objektarm nicht zwingend zusammenfallen, so dass eine größere Anzahl von Interferenzen gleicher Neigung, bekannt auch als Haidingersche Ringe, im Feld entstehen kann. Dies kann zu einer Verletzung des Sampling-Theorems bei der Detektion führen.

Ein Verfahren nach DD 268771 A1 [10] ermöglicht die zeitaufgelöste Objekterfassung. Dieser Ansatz ermöglicht jedoch eher keine Kurzkohärenz-Interferometrie für einzelne Messpunkte oder entlang einzelner Messlinien und erfordert ein erhebliches Sensorvolumen aufgrund der sehr großen Lateral-Shear.

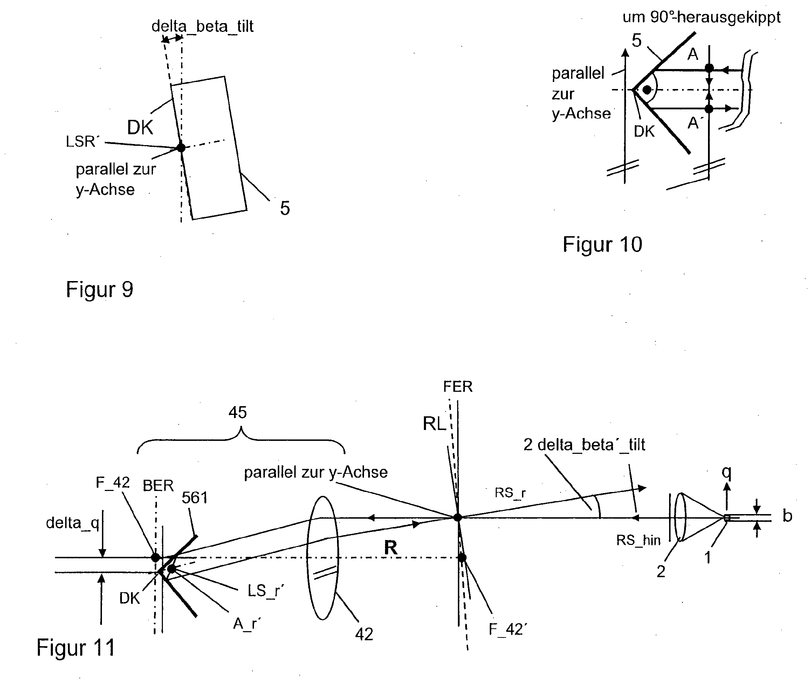

Von K. Gastinger u. a. wird in Proc. of SPIE 7389, 73891J1 bis -73891J12, 2009, [11] und auch in der PCT-Schrift WO2010/139764 A1 [12] der Einsatz von Mikro-Mirau-Interferometern und Twyman-Green-Interferometern in Array-Form auch mit Kurzkohärenz-Lichtquelle zur parallelisierten Inspektion von MEMS und MOEMS beschrieben. Für die Kurzkohärenz-Technik wird hierbei jedoch die optische Weglänge über der Zeit gescannt, so dass hier kein One-shot-Verfahren gegeben ist. Selbst moderate Vibrationen im Umfeld sind hierbei im hohen Maße schädlich für im Scan zu detektierenden Wavelet-Signale, gemäß Abb. 9 oben in [11], die im Extremfall mit üblichen Algorithmen gar nicht mehr auswertbar sind oder stark verfälschte Messergebnisse liefern.

Von J. Schwider wurde bereits 1996 in DE 196 32 594 A1 [13] für die Zweistrahl-Interferometrie ein Mach-Zehnder-Interferometer oder ein Michelson-Interferometer mit einem mikrooptischen Array in Form eines Mikrolinsen-Arays im Objektstrahlengang zwecks konfokaler Beleuchtung des Objekts und konfokaler Diskriminierung vorgeschlagen.

Erstmalig wird nach bestem Wissen von W. Emer und J. Schwider in Applied Optics, 38, No. 16, S. 3516-3522, 1999 [14] die Anwendung eines Schwarzschild-Spiegelobjektivs in einer Mirau-Anordnung für die Phasenschiebe-Interferometrie im UV-Bereich beschrieben. Der optische Gangunterschied wird hierbei zeitsequenziell verändert, um das Phasenschiebe-Verfahren anwenden zu können. Es handelt sich somit nicht um ein One-shot- und auch kein Kurzkohärenz-Verfahren.

Joseph W. Goodman beschreibt im Aufsatz "Holography Viewed from the Perspective of the Light Field Camera" Goodman [15] Ansätze zur Fourier- und Fresnel-Holografie mit einer Maske in der Bildebene. Diese werden von der Lichtfeld-Kamera für die Holografie abgeleitet. Dieser Aufsatz findet sich in den Tagungsunterlagen zur Fringe 2013, S. 3 bis 15, 7th International Workshop on Advanced Optical Imaging and Metrology, Editor Wolfgang Osten, ISBN 978-3-642-36358-0 ISBN 978-3-642-36359-7 (eBook), DOI 10.1007/978-3-642-36359-7, Springer Heidelberg New York Dordrecht London. Mittels Video-Konferenzschaltung erfolgte die Video-Präsentation dieses Aufsatzes auf der Fringe 2013 in Nürtingen bei Stuttgart am 11.9.2013. Die dabei von Joseph W. Goodmann präsentierten Ansatze zur Holografie stellt jedoch keine Lehre dar, um für die One-shot-Zweistrahl-Interferometrie oder die für die optische Kohärenz-Tomografie benötigten Zweistrahl-Interferogramme, insbesondere auch Kurzkohärenz-Interferogramme, zu generieren, welche sich im Vergleich zu Hologrammen in der Regel vergleichsweise sehr schnell numerisch auswerten lassen. Es wurde hierbei keine Lehre für eine strukturierte . Beleuchtung des Objekts beim Lichtfeld-Ansatz für die Zweistrahl-Interferometrie gegeben.

Von Ren Ng u.a. wird im Stanford Tech Report CTSR 2005-02 [16] in der Abbildung 1 auf Seite 2 die Lichtfeld-Fotografie mit einer Kamera nach dem Plenoptic-Ansatz beschrieben, die ein Mikrolinsen-Array in der Bildebene aufweist. Diese Anordnung ist in dieser Form jedoch nicht für die One-shot-Zweistrahl-Interferometrie und auch nicht für die optische Kohärenz-Tomografie anwendbar, da mit dieser Anordnung in der Regel keine Zweistrahl-Interferenzen erzeugt werden können.

Von Peter J. deGroot wird in der Patentschrift US 7,177,029 B2 [17] ein stroboskopisches Interferometer beschrieben, das jedoch die Interferogramm-Daten zeitseriell mit einer Serie von Lichtpulsen detektiert, also auf der Time-domain-Methode basiert. Mit diesem Ansatz kann demzufolge keine One-Shot-Messung durchgeführt werden.

Von J. Niehues und Peter Lehmann wird in der Offenlegungsschrift DE 10 2011 000 213 A1 [18] eine Anordnung zur Weißlicht-Interferenzmikroskopie beschrieben, bei der eine konfokale Beleuchtung mittels eines räumlichen Lichtmodulators erzeugt wird. Auch mit diesem Ansatz kann keine One-Shot-Messung durchgeführt werden, da es sich um einen Time-domain-Ansatz handelt.

EP 2 447 663 beschreibt ein Mirau Interferometer, bei welchem sowohl die Referenz- als auch die Objektwellenfront gekrümmt sind. Das Interferometer umfasst Mittel zur Korrektur der durch Temperatur bedingten Änderungen in den optischen Gängen des Referenzlichts und des Objektlichts, so dass der Unterschied zwischen der optischen Länge des Referenzlichts A und der optischen Länge des Objektlichts B ungefähr Null ist.

### Ziele der Erfindung

Ein Ziel der Erfindung besteht darin, vor allem robuste One-Shot-Messtechnik mit einer vergleichsweise hohen lateralen Auflösung zur Erfassung von Abstand, Tiefe, Profil, Form, Welligkeit und/oder Rauheit oder der optischen Weglänge in oder an technischen oder biologischen Objekten, auch in Schichtenform oder auch zur optischen Kohärenz-Tomografie (OCT), insbesondere auch mit One-Shot-Multi-Punkt-Antastung, bei der die Signale in der Regel in Wavelet-Form entstehen, der wirtschaftlichen Nutzung zuzuführen. Die Messung soll vorzugsweise in einer vergleichsweise kurzen Messzeit ausgeführt werden können. Weiterhin soll die Verkippung und/oder die Neigung der Objektoberfläche oder deren Form, bzw. Mikroform innerhalb gewisser Grenzen, vorzugsweise keinen oder nur einen vernachlässigbar geringen Einfluss auf die Signalform, insbesondere die räumliche Frequenz des Wavelets, aufweisen.

Insbesondere sollen vorzugsweise Messungen auch bei extremen mechanischen Stoßbelastungen im Messgebiet möglich sein.

Ein weiteres Ziel besteht darin, für die Koordinatenmesstechnik eine "fliegende" Multipunkt-Abstands-Messung, eine "fliegende" 3D-Messung oder eine "fliegende" Linienprofilmessung zu ermöglichen.

### Aufgaben der Erfindung

Damit ist also eine erfinderische Aufgabe zu lösen, beim optischen Antasten der Objektoberfläche, eines Objektpunktes oder eines Objektvolumens mit einem Zweistrahl-Interferometer, optische Signale in möglichst gut auswertbarer Signalform für ein punktförmiges, ein linienhaftes Messfeld oder für ein flächiges Messfeld mit vielen einzelnen Messpunkten ohne einen mechanischen, zeitseriell erfolgenden Tiefen-Scan bereitzustellen, welche zumindest bei nicht oder wenig lichtstreuenden Messobjekten möglichst eine Phasenauswertung gestatten.

Es besteht wegen der Forderung nach einer hohen lateralen Auflösung auch die Aufgabe, das Messsystem mit einer vergleichsweise hohen numerischen Apertur bis in den Grenzbereich des technisch Machbaren, einschließlich auch in Immersionstechnik, auszubilden. Durch eine große und gegebenenfalls auch sehr große numerische Apertur soll ein vergleichsweise hoher Anteil des auf das Objekt eingestrahlten Lichts zur Detektion gebracht werden, so dass die Messzeit gemäß der Aufgabenstellung vergleichsweise kurz gewählt werden kann und Messungen auch in einer vibrationsbelasteten Umgebung, an bewegten Komponenten oder bei der In-Vivo-Diagnostik von Geweben oder Zellen am lebenden Menschen möglich sind. Dabei sollen insbesondere auch Tumorzellen anhand ihrer geometrischen Struktur sicher erkannt werden können.

Es sollen dabei in einer Messung viele lateral benachbarte Objektelemente oder Objektpunkte gleichzeitig angemessen werden können. Das heißt, es besteht die Aufgabe, beim Stand der verfügbaren Auswerte-Algorithmen in der Kurzkohärenz-Interferometrie und OCT gut auswertbare und vergleichsweise robuste optische Signale bei der optischen Antastung von Objekten durch das erfindungsgemäße Verfahren und die erfindungsgemäße Anordnung mittels eines das Objekt anmessenden Interferometers, auch in der Form eines hochaperturigen Interferenzmikroskops, insbesondere mit Messobjektiv im Objektarm, möglichst schnell bereitzustellen.

Die Ortsfrequenz für die Schwerpunktwellenlänge der Signale in Wavelet-Form, beziehungsweise die Schwerpunktwellenzahl kS in einem räumlichen Interferogramm, soll dabei in hohem Maße vorbestimmt einstellbar sowie von der Verkippung und Neigung der Objektoberfläche unabhängig sein.

Dabei ist im Einzelnen die Aufgabe zu lösen, den Betrag der Neigung interferierender Wellenfronten, die auf einem Detektor ein räumliches Interferogramm bilden, mit einfachen Mitteln, zumindest näherungsweise vorbestimmt zu realisieren.

Dabei ist im Einzelnen weiterhin die Aufgabe zu lösen, den Einfluss der Dispersion bei der Anwendung refraktiver Komponenten insbesondere im interferometrischen Strahlengang, vernachlässigbar klein oder numerisch beherrschbar zu halten, um eine weitgehend konstante Mittenfrequenz im entstehenden Interferenz-Wavelet zu erreichen. Dies wiederum begünstigt eine vergleichsweise einfache und zuverlässige Signalverarbeitung der aus räumlichen Interferogrammen abgeleiteten Signale. Der Einfluss der Dispersion des Objekts ist natürlich stets gegeben und ist auch bei der Signalverarbeitung zu beachten und gegebenenfalls durch eine geeignete Algorithmik zu bearbeiten.

Eine hoch-aperturige, interferometrische 2D- oder auch 3D-Objekterfassung soll im Extremfall auf der Basis der Daten eines einzigen Kamera-Frames durchgeführt werden, weil One-shot-Daten gewonnen mittels Kurzpuls-Technik, auch bei Vibrationen in sich konsistent sind. Insbesondere soll dazu kurzkohärentes Licht eingesetzt werden.

Es sollen auch Informationen über das Winkelspektrum für jeden einzelnen Objektpunkt gewonnen werden können.

Die obigen Aufgaben werden durch die Merkmale der unabhängigen Ansprüche gelöst. Bevorzugte Ausführungsformen sind Gegenstand der abhängigen Ansprüche.

### Einsatzgebiete der erfinderischen Lösung

Einsatzgebiete der erfinderischen Lösung sollen sein: Die Mikroform- und die Mikroprofilmessung, die Messung der Rauheit sowie auch die Miniform-Messung, die Form-Messung an nicht oder nur wenig kooperativen Oberflächen, wie auch z.B. menschliches Lebergewebe. Ein weiteres Anwendungsgebiet stellt auch die Messung von polierten und auch nichtpolierten optischen Asphären und Freiformflächen dar. Hier kann ein optisches Materialbearbeitungssystem der erfinderischen Anordnung beigeordnet sein. Dieses Bearbeitungssystem soll auf den Erkenntnissen der neuesten Materialforschung beruhen und ist in seinen Details und der Parametrisierung ebenfalls nicht Bestandteil dieser Erfindung.

Ein Beispiel für die Anwendung der Erfindung ist hier auch die Erfassung der Mikroform von lebenden Zellen, insbesondere auch Tumorzellen am lebenden Organismus.

Ein besonderes Anwendungsgebiet stellt auch die intraorale Vermessung der 3D-Form eines Zahns, eines Zahnbereiches zumindest in Teilbereichen dar.

Die Messanordnung und das Verfahren sollen auch zur optischen Kohärenz-Tomografie mit räumlichen Interferogrammen, auch als Optische Kohärenz-Tomografie nach dem Spatial domain-Ansatz bekannt (SD OCT), insbesondere auch mit schneller Multi-Punkt-Antastung für technische oder biologische Objekte eingesetzt werden.

Ein spezielles Anwendungsgebiet kann die biologische und medizinische Grundlagenforschung darstellen. Beispielsweise können die erfinderischen Verfahren und Anordnungen durch die Ankopplung, insbesondere spektral höchstauflösender Sensorsysteme zur Aufklärung der Entstehung und des Wachstums, einschließlich der Stoffwechselprozesse von lebenden Zellen, eingesetzt werden.

Ein anderes Einsatzgebiet ist die Messung an optisch rauen Objektoberflächen wo die Höhenwerte von Messpunkt zu benachbartem Messpunkt um mehr als ein Viertel der Wellenlänge differieren können.

Ein weiteres Anwendungsgebiet stellt auch die In-line-Vermessung von mikro-elektro-mechanischen Systemen (MEMS) und mikro-opto-elektro-mechanischen Systemen (MOEMS) dar.

Eine besondere Motivation für die Anwendung der Erfindung ist die Nutzbarmachung der interferometrischen Verstärkung eines schwachen Objektsignals von einem eher wenig kooperativen Messobjekt.

### Beschreibung

Für die nachstehende Beschreibung wird auch der Begriff Lichtfeld-Interferometrie (Light Field Interferometry) in Analogie zur Lichtfeld-Fotografie (Light Field Photography) verwendet. Dieser Ansatz wird hier auf der Basis der Zweistrahl-Interferometrie und mit strukturierter und konfokaler Beleuchtung beschrieben. Dabei soll der Fall der Zweistrahl-Interferometrie zumindest angenähert sein, da bei lichtstreuenden Objekten der Übergang zur Vielstrahl-Interferenz fließend gegeben sein kann. Es werden Zweistrahl-Interferometer-Anordnungen eingesetzt. Beim Einsatz eines Fizeau-Interferometers, welches in der Regel Interferenzen - je nach gegebenen Reflektivitäten - mit einer gewissen Vielstrahl-Charakteristik erzeugt, entstehen durch die hierbei stets gegebene Strahlführung Zweistrahl-Interferenzen. Die Beleuchtung des Objekts erfolgt hierbei - im Gegensatz zur Holografie - stets strukturiert und zumindest näherungsweise auch konfokal.

Hier wird der Begriff Licht stets als Synonym für elektromagnetische Strahlung vom Terahertz-, über den Infrarot- bis zum tiefen UV-Bereich verwendet.

Der Unterschied des holografischen Ansatzes nach J. W. Goodman [15] mit der Lichtfeld-Kamera zum erfinderischen Ansatz besteht darin, dass es sich hierbei um Zweistrahl-Interferometrie mit konfokaler, also feiner oder sehr feiner strukturierter Objektbeleuchtung handelt. Es werden Messpunkte vom Objekt erfasst und ausgewertet, die sich zumindest näherungsweise im wellenoptischen Schärfentiefebereich der Beleuchtung befinden. Darüber hinaus wird mit eher kurzkohärentem Licht beleuchtet, wodurch sich Kurzkohärenz-Interferogramme ergeben.

Die Auswertung der hier im Weiteren beschriebenen Zweistrahlinterferenzen erfordert in der Regel einen geringeren Aufwand als die numerische Rekonstruktion von Hologrammen.

**Ausführungsbeispiel1.** Ausführungsbeispiel 1 betrifft ein Messverfahren mit einem einzelnen Endreflektor im Referenzarm eines Zweistrahl-Interferometers.

Es handelt sich um ein Verfahren zur robusten One-shot-Interferometrie, insbesondere auch zur optischen Kohärenz-Tomografie nach dem Spatial-domain-Ansatz (SD-OCT), insbesondere zur Materialmessung und auch Tumorzellen-Erkennung. Dieses kann zur Abstandsmessung, insbesondere auch zur Messung in Verbindung mit einer 3D-Koordinaten-Messmaschine, zur zwei- oder dreidimensionalen Profilmessung, Rauheits-, Welligkeits- und Ebenheitsmessung an technischen und biologischen Objekten, insbesondere dort auch zur Tumorzellen-Erkennung mittels Erfassung des 3D-Profils von Zellen, und zur Schichtenmessung eingesetzt werden.

Dazu wird ein Zweistrahl-Interferometer mit Referenz- und Objektarm, insbesondere auch in Form eines Interferenzmikroskops, eingesetzt. Im Referenzarm ist mindestens ein Endreflektor angeordnet. Das Zweistrahl-Interferometer ist insbesondere in Freistrahlausbildung und insbesondere auch als Michelson-, Twyman-Green-, Mirau-, Linnik-, Hybrid-Linnik- oder Mach-Zehnder-Interferometer ausgebildet. Das Zweistrahl-Interferometer weist folgende Komponenten auf:
- entweder mindestens eine, in mindestens einer Achsen-Richtung schmal ausgebildete Linien-Lichtquelle
- oder ein Linien-Lichtquellen-Array, ausgebildet mit einer Vielzahl von in einer Achsen-Richtung schmal gebildeten, zumindest näherungsweise parallelen Linienspots.

Die Achsenrichtung der schmalen Ausbildung wird hier jeweils als Quer-Achsen-Richtung (q) bezeichnet.

Weiterhin weist dieses Zweistrahl-Interferometer folgende Komponenten auf:
- ein der Linien-Lichtquelle oder dem Linien-Lichtquellen-Array nachfolgendes Lichtquellen-Objektiv zur zumindest näherungsweise beugungsbegrenzten Abbildung derselben,
- ein das Objekt abbildendes Objektiv,
- ein Detektor-Objektiv, wobei das Objektiv, welches das Objekt abbildet, und das Detektor-Objektiv auch als ein einziges Objektiv ausgebildet sein können,
- ein gerasterter Detektor mit Empfängerelementen für elektromagnetische Strahlung,
- und mindestens ein Digitalrechner mit mindestens einem Auswerteprogramm für detektierte Interferogramme zur schnellen Gewinnung von Informationen über das Objekt.

Ferner ist im Referenzarm:
- entweder die Linien-Lichtquelle in der Quer-Achsen-Richtung (q) jeweils so schmal ausgebildet,
- oder sind die Linienspots des Linien-Lichtquellen-Arrays in der Quer-Achsen-Richtung (q) jeweils so schmal ausgebildet,
dass diese für das nachfolgende Lichtquellen-Objektiv in dieser Quer-Achsen-Richtung (q) zumindest näherungsweise gleich oder unterhalb dessen beugungsbegrenzter Lateral-Auflösung ist/sind, welche durch die numerische Apertur dieses Lichtquellen-Objektivs und der eingesetzten Lichtwellenlänge oder der eingesetzten Lichtwellenlängen der Linien-Lichtquelle oder des Linien-Lichtquellen-Arrays bestimmt ist.

Es wird eine zumindest näherungsweise beugungsbegrenzte Abbildung der Linien-Lichtquelle oder der Linienspots des Linien-Lichtquellen-Arrays auf das Objekt mittels Objektiv durchgeführt. Die Airy-Breite in dieser Abbildungssituation übersteigt also zumindest näherungsweise die der schmal ausgebildeten Linien-Lichtquelle oder die Breite der schmalen Linienspots ist dieser Airy-Breite zumindest näherungsweise gleichgemacht. Die Airy-Breite in dieser Abbildungssituation ergibt sich aus der numerischen Apertur der nachfolgenden Abbildungsoptik und der eingesetzten Wellenlänge.

Weiterhin ist:
(i) Entweder zum einen
   - entweder ein Hybrid-Retro-Referenz-Endreflektor (z.B. als der Endreflektor bzw. als eine Komponente des Endreflektors) vollständig im Referenzarm angeordnet, welcher mit einem Referenzobjektiv ausgebildet ist,
   - oder ein Hybrid-Retro-Referenz-Endreflektor-System gebildet, welches mit einem Objektiv ausgebildet ist, das außerhalb des Referenzarms angeordnet ist. Dabei ist der Endreflektor selbst - als eine Komponente des Hybrid-Retro-Referenz-Endreflektor-Systems -jedoch im Referenzarm angeordnet.
      Diese Hybrid-Retro-Referenz-Endreflektoren sind jeweils in Kombination mit mindestens einem 90°-Dachkant-Endreflektor im Referenzarm ausgebildet und die Dachkante ist jeweils zumindest näherungsweise in der Brennebene des Referenzobjektivs oder des Objektivs, welches sich außerhalb des Referenzarms befindet, angeordnet. Die Brennebene von beiden Objektiven ist dabei stets im Referenzraum lokalisiert. Die Dachkante des 90°-Dachkant-Endreflektors ist jeweils zumindest näherungsweise senkrecht zur Quer-Achsen-Richtung (q) und parallel zur Längsrichtung der Linien-Lichtquelle angeordnet.
      Mittels dieses Hybrid-Retro-Referenz-Endreflektors bzw. Hybrid-Retro-Referenz-Endreflektor-Systems wird durch einen Querversatz delta_q der Dachkante (DK) zumindest näherungsweise parallel zur Richtung der Quer-Achsen-Richtung (q)
         - entweder in der Brennebene (BER) des dem Hybrid-Retro-Referenz-Endreflektor zugeordneten Referenzobjektivs
         - oder in der Brennebene (BER) eines sowohl dem Referenz- als auch dem Objektarm zugeordneten Objektivs, welches sich außerhalb des Referenzarms befindet,
      in der dem Detektorobjektiv vorgeordneten Brennebene (F_91_r , F_9') ein Querversatz 2 delta_q_strich von zueinander kohärenten Lichtspots (A_r", A_o") aus dem Referenz- und Objektarm eingeführt.
      Der Querversatz delta_q dieser Hybrid-Retro-Referenz-Mikro-Endreflektoren ist maximal gleich dem zehnten Teil oder kleiner der Brennweite dieses Referenzobjektivs oder dieses Objektivs, welches sich außerhalb des Referenzarms befindet, und mindestens gleich oder größer als der objektivbezogene Airy-Scheibchen-Radius für die Schwerpunktwellenlänge im detektierten räumlichen Interferogramm gemacht.
(ii) Oder zum anderen ist weiterhin:
   ein Hybrid-Retro-Referenz-Endreflektor im Referenzarm angeordnet,
      - welcher entweder als Zylinder-Optik-Hybrid-Retro-Referenz-Endreflektor mit einem Zylinder-Objektiv mit einer Zylinderachse und einem Planspiegel in der Brennebene des Zylinder-Objektivs ausgebildet und die Zylinderachse jeweils senkrecht zur Längsrichtung der Linien-Lichtquelle ist,
      - oder welcher als Dachkantreflektor ausgebildet ist und die Dachkante jeweils senkrecht zur Längsrichtung der Linien-Lichtquelle ist.
   Durch eine Kippung (delta_beta_tilt) des Hybrid-Retro-Referenz-Endreflektors wird,
      wobei der Kippwinkel maximal gleich oder kleiner 6 Altgrad (6°) und mindestens gleich oder größer als eine halbe Schwerpunktwellenlänge im detektierten räumlichen Interferogramm, bezogen auf den ausgeleuchteten Pupillendurchmesser des Objektivs am Interferometerausgang (62, 91, 9) gemacht ist, jeweils um eine Kipp-Achse, die zumindest näherungsweise sowohl senkrecht zur Quer-Achsen-Richtung (q) als auch senkrecht zur Richtung der Lichtausbreitung ist, jeweils bezogen auf einen entfalteten Strahlengang des Zweistrahl-Interferometers,
   in der dem Detektorobjektiv vorgeordneten Brennebene ein Querversatz 2 delta_q oder 2 delta_q_strich von zueinander kohärenten Lichtspots (A_r", A_o") aus dem Referenz- und Objektarm eingeführt.

In beiden Fällen werden in der Ebene des gerasterten Detektors durch diesen gerasterten Detektor vorzugsweise vorgeordnete optische Mittel zur Einführung von Astigmatismus in den Detektionsstrahlengang, welche dem Objektiv zur Abbildung des Objekts zugeordnet oder nachgeordnet sind, jeweils zwei zueinander gekippte interferierende Wellenfronten, je eine vom Objekt- (0) und je eine vom Referenzarm (R), welche vorzugsweise zumindest näherungsweise eine Zylinderform aufweisen, gebildet und zur Interferenz gebracht. Dabei wird mindestens ein räumliches Interferogramm (KKI) gebildet und mittels des gerasterten Detektors detektiert.

Dabei ist die Richtung der Längsachsen der Zylinder-Wellenfronten jeweils zumindest näherungsweise mit der Quer-Achsen-Richtung (q) - im entfalteten Strahlengang des Zweistrahl-Interferometers - übereinstimmend gemacht und die Kippachse der mindestens einen Referenz-Zylinder-Wellenfront ist jeweils zumindest näherungsweise senkrecht zur Quer-Achsen-Richtung (q).

**Ausführungsbeispiel 2.** Ausführungsbeispiel 2 betrifft ein Messverfahren mit einem Endreflektor-Array im Referenzarm eines Zweistrahl-Interferometers.

Es handelt sich um ein Verfahren zur robusten One-shot-Interferometrie, insbesondere auch zur optischen Kohärenz-Tomografie nach dem Spatial-domain-Ansatz (SD-OCT), insbesondere zur Materialmessung und auch Tumorzellen-Erkennung. Dieses kann zur Abstandsmessung, insbesondere auch zur Messung in Verbindung mit einer 3D-Koordinaten-Messmaschine, zur zwei- oder dreidimensionalen Profilmessung, Rauheits-, Welligkeits- und Ebenheitsmessung an technischen und biologischen Objekten, insbesondere dort auch zur Tumorzellen-Erkennung mittels Erfassung des 3D-Profils von Zellen, und zur Schichtenmessung eingesetzt werden.

Dazu wird ein Zweistrahl-Interferometer mit Referenz- und Objektarm, insbesondere auch in Form eines Interferenzmikroskops, eingesetzt. Im Referenzarm ist mindestens ein Endreflektor angeordnet. Das Zweistrahl-Interferometer ist insbesondere in Freistrahlausbildung und insbesondere auch als Michelson-, Twyman-Green-, Mirau-, Linnik-, Hybrid-Linnik- oder Mach-Zehnder-Interferometer ausgebildet. Das Zweistrahl-Interferometer weist folgende Komponenten auf:
- entweder mindestens eine, in mindestens einer Achsen-Richtung q schmal ausgebildete Linien-Lichtquelle
- oder ein Linien-Lichtquellen-Array, ausgebildet mit einer Vielzahl von in einer Achsen-Richtung q schmal gebildeten, zumindest näherungsweise parallelen Linienspots.

Diese Achsenrichtung der schmalen Ausbildung wird hier jeweils als Quer-Achsen-Richtung (q) bezeichnet.

Weiterhin weist dieses Zweistrahl-Interferometer folgende Komponenten auf:
- ein der Linien-Lichtquelle oder dem Linien-Lichtquellen-Array nachfolgendes Lichtquellen-Objektiv zur zumindest näherungsweise beugungsbegrenzten Abbildung derselben,
- ein das Objekt abbildendes Objektiv,
- ein Detektor-Objektiv, wobei das Objektiv, welches das Objekt abbildet, und das Detektor-Objektiv auch als ein einziges Objektiv ausgebildet sein können,
- ein gerasterter Detektor mit Empfängerelementen für elektromagnetische Strahlung,
- und mindestens ein Digitalrechner mit einem Auswerteprogramm für detektierte Interferogramme zur schnellen Gewinnung von Informationen über das Objekt.

Ferner ist im Referenzarm
- entweder die Linien-Lichtquelle in der Quer-Achsen-Richtung (q) jeweils so schmal ausgebildet,
- oder sind die Linienspots des Linien-Lichtquellen-Arrays in der Quer-Achsen-Richtung (q) jeweils so schmal ausgebildet,
dass diese für das nachfolgende Lichtquellen-Objektiv in dieser Quer-Achsen-Richtung (q) zumindest näherungsweise gleich oder unterhalb dessen beugungsbegrenzter Lateral-Auflösung ist/sind, welche durch die numerische Apertur dieses Lichtquellen-Objektivs und der eingesetzten Lichtwellenlänge oder der eingesetzten Lichtwellenlängen der Linien-Lichtquelle oder des Linien-Lichtquellen-Arrays bestimmt ist.

Es wird eine zumindest näherungsweise beugungsbegrenzte Abbildung der Linien-Lichtquelle oder der Linienspots des Linien-Lichtquellen-Arrays auf das Objekt mittels Objektiv durchgeführt. Die Airy-Breite in dieser Abbildungssituation übersteigt also zumindest näherungsweise die der schmal ausgebildeten Linien-Lichtquelle oder die Breite der schmalen Linienspots ist dieser Airy-Breite zumindest näherungsweise gleichgemacht. Die Airy-Breite in dieser Abbildungssituation ergibt sich aus der numerischen Apertur der nachfolgenden Abbildungsoptik und der eingesetzten Wellenlänge.

Ferner ist:
- zum einen entweder ein Hybrid-Retro-Referenz-Endreflektor-System im Referenzarm R angeordnet, welches mit einem mit einem Referenzobjektiv ausgebildet ist,
- oder zum anderen ein Hybrid-Retro-Referenz-Endreflektor-System angeordnet, das mit einem Objektiv ausgebildet ist, welches sich außerhalb des Referenzarms befindet. Dieses Hybrid-Retro-Referenz-Endreflektor-System ist jeweils in Kombination mit
   - entweder einem Hybrid-Referenz-Mikro-Endreflektor-Array mit Hybrid-Retro-Referenz-Mikro-Endreflektoren vom 90°-Dachkant-Spiegel-Typ oder 90°-Dachkant-Prismen-Typ;
   - oder einem Referenz-Mikro-Endreflektor-Array mit Hybrid-Retro- Referenz-Mikro-Endreflektoren vom Zylinder-Linsen-Cat' eye-Typ oder vom Zylinder-Spiegel-Cat' eye-Typ im Referenzarm ausgebildet.

Dieses Hybrid-Retro-Referenz-Endreflektor-System ist jeweils zumindest näherungsweise in der Brennebene des Referenzobjektivs oder des Objektivs, welches sich außerhalb des Referenzarms befindet, angeordnet.

Mittels des Hybrid-Retro-Referenz-Endreflektor-Systems wird jeweils durch einen Querversatz delta_q dieser Hybrid-Retro- Referenz-Mikro-Endreflektoren vom Zylinder-Cat' eye-Typ oder vom 90°-Dachkant-Typ zumindest näherungsweise parallel zur Richtung der Quer-Achsen-Richtung (q)
- entweder in der Brennebene (BER) des dem Hybrid-Retro-Referenz-Endreflektor zugeordneten Referenzobjektivs (4, 42)
- oder in der Brennebene (BER) eines sowohl dem Referenz- als auch dem Objektarm zugeordneten Objektivs,
eingeführt.

Damit ist auch in der dem Detektorobjektiv vorgeordneten Brennebene (F_91_r, F_9 ') ein Querversatz 2 delta_q_strich von zueinander kohärenten Lichtspots (A_r", A_o") aus dem Referenz- und Objektarm eingeführt.

Der Querversatz delta_q dieser Hybrid-Retro-Referenz-Mikro-Endreflektoren ist maximal gleich dem zehnten Teil oder kleiner der Brennweite dieses Referenzobjektivs oder dieses Objektivs, welches sich außerhalb des Referenzarms befindet, und mindestens gleich oder größer als der objektivbezogene Airy-Scheibchen-Radius für die Schwerpunktwellenlänge im detektierten räumlichen Interferogramm gemacht.

In beiden Fällen werden in der Ebene des gerasterten Detektors durch diesen gerasterten Detektor vorzugsweise vorgeordnete optische Mittel zur Einführung von Astigmatismus in den Detektionsstrahlengang, welche dem Objektiv zur Abbildung des Objekts zugeordnet oder nachgeordnet sind, eine Vielzahl von jeweils zwei zueinander gekippten interferierenden Wellenfronten, je eine vom Objekt- (O) und je eine vom Referenzarm (R), welche vorzugsweise zumindest näherungsweise eine Zylinderform aufweisen, gebildet und zur Interferenz gebracht. Dabei wird eine Vielzahl von räumlichen Interferogrammen (KKI) gebildet und mittels gerastertem Detektor detektiert.

Dabei ist die Richtung der Längsachsen der Zylinder-Wellenfronten jeweils zumindest näherungsweise mit der Quer-Achsen-Richtung (q) - im entfalteten Strahlengang des Zweistrahl-Interferometers - übereinstimmend gemacht und die Kippachse der mindestens einen Referenz-Zylinder-Wellenfront ist jeweils zumindest näherungsweise senkrecht zur Quer-Achsen-Richtung (q).

**Ausführungsbeispiel 3.** Ausführungsbeispiel 3 betrifft ein Messverfahren mit einem Endreflektor-Array im Referenzarm eines Zweistrahl-Interferometers.

Es handelt sich um ein Verfahren zur robusten One-shot-Interferometrie, insbesondere auch zur optischen Kohärenz-Tomografie nach dem Spatial-domain-Ansatz (SD-OCT), insbesondere zur Materialmessung und auch Tumorzellen-Erkennung. Dieses kann zur Abstandsmessung, insbesondere auch zur Messung in Verbindung mit einer 3D-Koordinaten-Messmaschine, zur zwei- oder dreidimensionalen Profilmessung, Rauheits-, Welligkeits- und Ebenheitsmessung an technischen und biologischen Objekten, insbesondere dort auch zur Tumorzellen-Erkennung mittels Erfassung des 3D-Profils von Zellen, und zur Schichtenmessung eingesetzt werden.

Dazu wird ein Zweistrahl-Interferometer mit Referenz- und Objektarm, insbesondere auch in Form eines Interferenzmikroskops, eingesetzt. Im Referenzarm ist mindestens ein Endreflektor angeordnet. Das Zweistrahl-Interferometer ist insbesondere in Freistrahlausbildung und insbesondere auch als Michelson-, Twyman-Green-, Mirau-, Linnik-, Hybrid-Linnik- oder Mach-Zehnder-Interferometer ausgebildet. Das Zweistrahl-Interferometer weist folgende Komponenten auf:
- entweder mindestens eine fein ausgebildete Punkt-Lichtquelle
- oder eine fein ausgebildete Punkt-Lichtquellen-Matrix, ausgebildet mit einer Vielzahl von fein gebildeten Spots,
- ein der Lichtquelle nachfolgendes Lichtquellen-Objektiv zur zumindest näherungsweise beugungsbegrenzten Abbildung derselben oder der Spots derselben,
- ein das Objekt abbildendes Objektiv,
- ein Detektor-Objektiv, wobei das Objektiv, welches das Objekt abbildet, und das Detektor-Objektiv auch als ein einziges Objektiv ausgebildet sein können.
- ein gerasterter Detektor mit Empfängerelementen für elektromagnetische Strahlung,
- und mindestens ein Digitalrechner mit einem Auswerteprogramm für detektierte Interferogramme zur schnellen Gewinnung von Informationen über das Objekt.

Ferner
- ist im Referenzarm entweder die fein ausgebildete Punkt-Lichtquelle jeweils so fein ausgebildet, oder sind im Referenzarm die Spots der fein ausgebildeten PunktLichtquellen-Matrix jeweils so fein ausgebildet, dass diese für das nachfolgende Lichtquellen-Objektiv zumindest näherungsweise gleich oder unterhalb dessen beugungsbegrenzter Lateral-Auflösung ist/sind, welche durch numerische Apertur dieses Lichtquellen-Objektivs und der eingesetzten Lichtwellenlänge oder der eingesetzten Lichtwellenlängen der fein ausgebildeten Lichtquelle (198) oder der fein ausgebildeten Punkt- Lichtquellen-Matrix bestimmt ist.
- Entweder mittels eines Voll-Retro-Referenz-Mikro-Endreflektor-Systems mit einem Referenzobjektiv im Referenzarm R und mit Voll-Retro-Referenz-Mikro-Endreflektoren in jeweils
   - entweder einem Mikro-Endreflektor-Array mit Voll-Retro-Referenz-Mikro-Endreflektoren vom rotationssymmetrischen Linsen-Cat'eye-Typ
   - oder einem Mikro-Endreflektor-Array mit Voll-Retro-Referenz-Mikro-Endreflektoren vom rotationssymmetrischen Spiegel-Cat'eye-Typ
   - oder einem Tripelprismenspiegel-Mikro-Endreflektor-Array mit Tripelprismenspiegel-Mikro-Endreflektoren
   - oder einem Hohl-Tripelspiegel-Mikro-Endreflektor-Array mit Tripelspiegel-Mikro-Endreflektoren
      und bei den Mikro-Endreflektoren jeweils ein Querversatz delta_q in der Brennebene des zugeordneten Referenzobjektivs besteht, der maximal gleich dem zehnten Teil oder kleiner der Brennweite dieses Referenzobjektivs und mindestens gleich oder größer als der objektivbezogene Airy-Scheibchen-Radius für die Schwerpunktwellenlänge im detektierten räumlichen Interferogramm gemacht ist,
   wird in der dem Detektorobjektiv vorgeordneten Brennebene ein Querversatz 2 delta_q oder 2 delta_q_strich von zueinander kohärenten Lichtspots (A_r", A_o") aus dem Referenz- und Objektarm eingeführt. Dieser ist mindestens gleich oder größer als der objektivbezogene Airy-Scheibchen-Radius für die Schwerpunktwellenlänge im detektierten räumlichen Interferogramm gemacht.

In der Ebene des gerasterten Detektors werden
durch diesen gerasterten Detektor vorzugsweise vorgeordnete optische Mittel zur Einführung von Astigmatismus in den Detektionsstrahlengang, welche dem Objektiv zur Abbildung des Objekts zugeordnet oder nachgeordnet sind, jeweils zwei zueinander gekippte interferierende Wellenfronten, welche vorzugsweise zumindest näherungsweise eine Zylinderform aufweisen, je eine vom Objekt- (O) und je eine vom Referenzarm (R) gebildet und zur Interferenz gebracht. Dabei wird eine Vielzahl von räumlichen Interferogrammen (KKI) gebildet und mittels gerastertem Detektor detektiert.

Dabei ist die Richtung der Längsachsen der Zylinder-Wellenfronten jeweils zumindest näherungsweise mit der Quer-Achsen-Richtung (q) - im entfalteten Strahlengang des Zweistrahl-Interferometers - übereinstimmend gemacht und die Kippachse der mindestens einen Referenz-Zylinder-Wellenfront ist jeweils zumindest näherungsweise senkrecht zur Quer-Achsen-Richtung (q).

**Ausführungsbeispiel 4.** Weiterhin ist vorzugsweise bei dem Verfahren zur robusten One-shot-Interferometrie gemäß einem oder mehrerer der obigen Ausführungsbeispiele die Richtung der Längsachsen der Zylinder-Wellenfronten jeweils zumindest näherungsweise mit der Quer-Achsen-Richtung (q) - im entfalteten Strahlengang des Zweistrahl-Interferometers - übereinstimmend gemacht und die Kippachse der mindestens einen Referenz-Zylinder-Wellenfront ist jeweils zumindest näherungsweise senkrecht zur Quer-Achsen-Richtung (q) angeordnet. Bei einer Linien-Lichtquelle fällt diese mit der Längsausdehnungs-Richtung der Linien-Lichtquelle oder der Längsausdehnungs-Richtung von länglichen Lichtspots einer Lichtquelle zusammen.

**Ausführungsbeispiel 5.** Weiterhin wird vorzugsweise bei dem Verfahren zur robusten One-shot-Interferometrie gemäß einem oder mehrerer der obigen Ausführungsbeispiele im Schnitt, der die Quer-Achsen-Richtung (q) enthält, die Fourier-Ebene (FEO) des objektabbildenden Objektivs auf den gerasterten Detektor zumindest näherungsweise scharf mit optischen Mitteln abgebildet und im Schnitt senkrecht dazu, der im entfalteten Strahlengang parallel zur Längsrichtung der Linienquelle ist, wird die Messebene des Objekts (MEO) scharf mit optischen Mitteln auf den gerasterten Detektor abgebildet, so dass im Detektionsstrahlengang eine astigmatische Abbildung durchgeführt wird.

Die Wellenfront-Inversion stellt hier keinen expliziten Vorteil dar. Vielmehr muss man das in Kauf nehmen. Diese ist unerwünscht, aber durch beugungsbegrenzte Abbildung von schmalen Spots der Lichtquelle tolerierbar.

**Ausführungsbeispiel 6.** Bei dem Verfahren zur robusten One-shot-Interferometrie gemäß einem oder mehrerer der obigen Ausführungsbeispiele wird vorzugsweise bei der Abbildung in einer Zwischenbildebene ZBE zumindest für das Objektlicht eine konfokale Diskriminierung mittels optischer Mittel zur Tiefpassfilterung durchgeführt. Damit wird bekannter Weise das Streulicht aus der Tiefe des Objekts verringert. Außerdem dient diese zur Tiefpassfilterung des Objektlichts, was zu einer Verbesserung der Signalqualität bei den detektierten räumlichen Interferogrammen führen kann.

Bei dem Verfahren zur robusten One-shot-Interferometrie wird vorzugsweise im Objektarm eine chromatische Tiefenaufspaltung mittels über der Wellenlänge brechkraftvariabler Mittel durchgeführt. Damit kann der wellenoptisch begründete Tiefenmessbereich bei dem Messverfahren wesentlich vergrößert werden.

**Ausführungsbeispiel 7.** Weiterhin wird bei dem Verfahren zur robusten One-shot-Interferometrie gemäß einem oder mehrerer der obigen Ausführungsbeispiele vorzugsweise bei der Abbildung in einer Zwischenbildebene ZBE für das Objektlicht eine Tiefpassfilterung mittels optischer Mittel durchgeführt.

Bei dem Verfahren zur robusten One-shot-Interferometrie wird vorzugsweise weiterhin eine spektrale Aufspaltung im Detektionsstrahlengang zur Vergrößerung der Kohärenzlänge und somit eine Separierung der Interferogramme unterschiedlicher Wellenlängen oder Wellenlängenbereiche auf dem gerasterten Empfänger durchgeführt.

Die spektrale Aufspaltung im Detektionsstrahlengang bewirkt eine Vergrößerung der Kohärenzlänge, was wiederum eine Vergrößerung der Fläche mit kontrastreichen auswertbaren Interferenzen auf dem Detektor bewirkt und somit auch die Möglichkeit der Durchführung einer Multiwellenlängen-Auswertung mit Phasenauswertung gibt. Diese Phasenauswertung liefert den optischen Gangunterschied null zumindest näherungsweise, auch wenn nur Teile des Interferogramms vorliegen oder auch eine Information über die Nichtauswertbarkeit des Messpunktes, beispielsweise wegen zu starkem Specklings im Interferogramm.

Bei dem Verfahren zur robusten One-shot-Interferometrie wird vorzugsweise eine rechnergesteuerte Lateral-Verschiebung des oder der Linienspots der Linien-Lichtquelle oder des Linien-Lichtquellen-Arrays durchgeführt. Somit kann das Messfeld voll erfasst werden.

Bei dem Verfahren zur robusten One-shot-Interferometrie wird vorzugsweise eine rechnergesteuerte Lateral-Verschiebung des oder der Endreflektoren oder des Endreflektor-Arrays durchgeführt. Somit kann das Messfeld voll erfasst werden.

Bei dem Verfahren zur robusten One-shot-Interferometrie wird vorzugsweise eine rechnergesteuerte Lateral-Verschiebung des oder der Linienspots der Linien-Lichtquelle oder des Linien-Lichtquellen-Arrays durchgeführt. Somit kann das Messfeld voll erfasst werden.

Bei dem Verfahren zur robusten One-shot-Interferometrie wird vorzugsweise die rechnergesteuerte Lateral-Verschiebung des oder der Endreflektoren oder des Endreflektor-Arrays und die rechnergesteuerte Lateral-Verschiebung des oder der Linienspots der Linien-Lichtquelle oder des Linien-Lichtquellen-Array zueinander synchronisiert durchgeführt.

Bei dem Verfahren zur robusten One-shot-Interferometrie wird vorzugsweise eine Immersionstechnik im Objektarm eingesetzt. Damit können auch Objekte in flüssiger Umgebung angemessen werden.

Bei dem Verfahren zur robusten One-shot-Interferometrie wird vorzugsweise eine Immersionstechnik im Objektarm und im Referenzarm eingesetzt.

Bei dem Verfahren zur robusten One-shot-Interferometrie wird vorzugsweise der Schwerpunkt der Einhüllenden eines kurzkohärenten, räumlichen Interferogramms (KKI) ausgewertet.

Bei dem Verfahren zur robusten One-shot-Interferometrie wird vorzugsweise die Phase eines räumlichen Interferogramms (KKI) ausgewertet.

Weitere Ausführungsbeispiele betreffen eine Messanordnung mit einem einzelnen Endreflektor im Referenzarm eines Zweistrahl-Interferometers.

**Ausführungsbeispiel 8.** Ausführungsbeispiel 8 betrifft r eine Messanordnung mit einem einzelnen Endreflektor im Referenzarm eines Zweistrahl-Interferometers.

Es handelt sich um eine Anordnung zur robusten One-shot-Interferometrie, insbesondere auch zur optischen Kohärenz-Tomografie nach dem Spatial-domain-Ansatz (SD-OCT), insbesondere zur Materialmessung und auch Tumorzellen-Erkennung. Dieses kann zur Abstandsmessung, insbesondere auch zur Messung in Verbindung mit einer 3D-Koordinaten-Messmaschine, zur zwei- oder dreidimensionalen Profilmessung, Rauheits-, Welligkeits- und Ebenheitsmessung an technischen und biologischen Objekten, insbesondere dort auch zur Tumorzellen-Erkennung mittels Erfassung des 3D-Profils von Zellen, und zur Schichtenmessung eingesetzt werden.

Dazu wird ein Zweistrahl-Interferometer mit Referenz- und Objektarm, insbesondere auch in Form eines Interferenzmikroskops, eingesetzt. Im Referenzarm ist mindestens ein Endreflektor angeordnet. Das Zweistrahl-Interferometer ist insbesondere in Freistrahlausbildung und insbesondere auch als Michelson-, Twyman-Green-, Mirau-, Linnik-, Hybrid-Linnik- oder Mach-Zehnder-Interferometer ausgebildet. Das Zweistrahl-Interferometer weist folgende Komponenten auf:
- entweder mindestens eine, in mindestens einer Achsen-Richtung schmal ausgebildete Linien-Lichtquelle
- oder ein Linien-Lichtquellen-Array, ausgebildet mit einer Vielzahl von in einer Achsen-Richtung schmal gebildeten, zumindest näherungsweise parallelen Linienspots.

Diese Achsenrichtung der schmalen Ausbildung wird hier jeweils als Quer-Achsen-Richtung (q) bezeichnet.

Weiterhin weist das Zweistrahl-Interferometer folgende Komponenten auf:
- ein der Linien-Lichtquelle oder dem Linien-Lichtquellen-Array nachfolgendes Lichtquellen-Objektiv zur zumindest näherungsweise beugungsbegrenzten Abbildung derselben,
- ein das Objekt abbildendes Objektiv,
- ein Detektor-Objektiv, wobei das Objektiv, welches das Objekt abbildet, und das Detektor-Objektiv auch als ein einziges Objektiv ausgebildet sein können,
- ein gerasterter Detektor mit Empfängerelementen für elektromagnetische Strahlung,
- und mindestens ein Digitalrechner mit einem Auswerteprogramm für detektierte Interferogramme zur schnellen Gewinnung von Informationen über das Objekt.

Ferner ist im Referenzarm:
- entweder die Linien-Lichtquelle in der Quer-Achsen-Richtung (q) jeweils so schmal ausgebildet ist,
- oder die Linienspots des Linien-Lichtquellen-Arrays in der Quer-Achsen-Richtung (q) jeweils so schmal ausgebildet sind,
dass diese für das nachfolgende Lichtquellen-Objektiv in dieser Quer-Achsen-Richtung (q) zumindest näherungsweise gleich oder unterhalb dessen beugungsbegrenzter Lateral-Auflösung ist/sind, welche durch die numerische Apertur dieses Lichtquellen-Objektivs und der eingesetzten Lichtwellenlänge oder der eingesetzten Lichtwellenlängen der Linien-Lichtquelle oder des Linien-Lichtquellen-Arrays bestimmt ist.

Es wird eine zumindest näherungsweise beugungsbegrenzte Abbildung der Linien-Lichtquelle oder der Linienspots des Linien-Lichtquellen-Arrays auf das Objekt mittels eines Objektivs durchgeführt. Die Airy-Breite in dieser Abbildungssituation übersteigt also zumindest näherungsweise die der schmal ausgebildeten Linien-Lichtquelle oder die Breite der schmalen Linienspots ist dieser Airy-Breite zumindest näherungsweise gleichgemacht. Die Airy-Breite in dieser Abbildungssituation ergibt sich aus der numerischen Apertur der nachfolgenden Abbildungsoptik und der eingesetzten Wellenlänge. Die Airy-Breite in dieser Abbildungssituation übersteigt also zumindest näherungsweise die der schmal ausgebildeten Linien-Lichtquelle oder die Breite der schmalen Linienspots ist dieser Airy-Breite zumindest näherungsweise gleichgemacht.

Weiterhin ist
(i) entweder zum einen
   - entweder ein Hybrid-Retro-Referenz-Endreflektor vollständig im Referenzarm angeordnet, welcher mit einem Referenzobjektiv ausgebildet ist,
   - oder ein Hybrid-Retro-Referenz-Endreflektor-System gebildet, welches mit einem Objektiv ausgebildet ist, das außerhalb des Referenzarms angeordnet ist. Dabei ist der Endreflektor selbst - als eine Komponente des Hybrid-Retro-Referenz-Endreflektor-Systems -jedoch im Referenzarm angeordnet.
      Diese Hybrid-Retro-Referenz-Endreflektoren sind jeweils in Kombination mit mindestens einem 90°-Dachkant-Endreflektor im Referenzarm ausgebildet und die Dachkante ist jeweils zumindest näherungsweise in der Brennebene des Referenzobjektivs oder des Objektivs, welches sich außerhalb des Referenzarms befindet, angeordnet. Die Brennebene von beiden Objektiven ist dabei stets im Referenzraum lokalisiert. Die Dachkante des 90°-Dachkant-Endreflektors ist jeweils zumindest näherungsweise senkrecht zur Quer-Achsen-Richtung (q) und parallel zur Längsrichtung der Linien-Lichtquelle angeordnet.
      Mittels dieses Hybrid-Retro-Referenz-Endreflektors wird durch einen Querversatz delta_q der Dachkante (DK) zumindest näherungsweise parallel zur Richtung der Quer-Achsen-Richtung (q)
         - entweder in der Brennebene (BER) des dem Hybrid-Retro-Referenz-Endreflektor zugeordneten Referenzobjektivs
         - oder in der Brennebene (BER) eines sowohl dem Referenz- als auch dem Objektarm zugeordneten Objektivs, welches sich außerhalb des Referenzarms befindet,
      in der dem Detektorobjektiv vorgeordneten Brennebene (F_91_r, F_9') ein Querversatz 2 delta_q_strich von zueinander kohärenten Lichtspots (A_r", A_o") aus dem Referenz- und Objektarm eingeführt.
      Dieser Querversatz delta_q dieser Hybrid-Retro-Referenz-Mikro-Endreflektoren ist maximal gleich oder kleiner dem zehnten Teil der Brennweite dieses Referenzobjektivs oder dieses Objektivs, welches sich außerhalb des Referenzarms befindet, und mindestens gleich oder größer als der objektivbezogene Airy-Scheibchen-Radius für die Schwerpunktwellenlänge im detektierten räumlichen Interferogramm gemacht.
(ii) Oder zum anderen ist weiterhin
   ein Hybrid-Retro-Referenz-Endreflektor im Referenzarm angeordnet,
      - welcher entweder als Zylinder-Optik-Hybrid-Retro-Referenz-Endreflektor mit einem Zylinder-Objektiv mit einer Zylinderachse und einem Planspiegel in der Brennebene des Zylinder-Objektivs ausgebildet und die Zylinderachse jeweils senkrecht zur Längsrichtung der Linien-Lichtquelle ist,
      - oder welcher als Dachkantreflektor ausgebildet ist und die Dachkante jeweils senkrecht zur Längsrichtung der Linien-Lichtquelle ist.
   Dabei ist der Kippwinkel maximal gleich oder kleiner 6 Altgrad (6°) und mindestens gleich oder größer als eine halbe Schwerpunktwellenlänge lambda_S im detektierten räumlichen Interferogramm, bezogen auf den ausgeleuchteten Pupillendurchmesser des Objektivs am Interferometerausgang (62, 91, 9) gemacht, jeweils um eine Kipp-Achse, die zumindest näherungsweise sowohl senkrecht zur Quer-Achsen-Richtung (q) als auch senkrecht zur Richtung der Lichtausbreitung ist, jeweils bezogen auf einen entfalteten Strahlengang des Zweistrahl-Interferometers.

Dem gerasterten Detektor sind vorzugsweise optische Mittel zur Einführung von Astigmatismus in den Detektionsstrahlengang vorgeordnet, welche dem Objektiv zur Abbildung des Objekts zugeordnet oder nachgeordnet sind.

**Ausführungsbeispiel 9.** Ausführungsbeispiel 9 betrifft eine Anordnung mit einem Endreflektor-Array im Referenzarm eines Zweistrahl-Interferometers.

Es handelt sich um eine Anordnung zur robusten One-shot-Interferometrie, insbesondere auch zur optischen Kohärenz-Tomografie nach dem Spatial-domain-Ansatz (SD-OCT), insbesondere zur Materialmessung und auch Tumorzellen-Erkennung. Dieses kann zur Abstandsmessung, insbesondere auch zur Messung in Verbindung mit einer 3D-Koordinaten-Messmaschine, zur zwei- oder dreidimensionalen Profilmessung, Rauheits-, Welligkeits- und Ebenheitsmessung an technischen und biologischen Objekten, insbesondere dort auch zur Tumorzellen-Erkennung mittels Erfassung des 3D-Profils von Zellen, und zur Schichtenmessung eingesetzt werden. Dazu wird ein Zweistrahl-Interferometer mit Referenz- und Objektarm, insbesondere auch in Form eines Interferenzmikroskops, eingesetzt. Im Referenzarm ist mindestens ein Endreflektor angeordnet. Das Zweistrahl-Interferometer ist insbesondere in Freistrahlausbildung und insbesondere auch als Michelson-, Twyman-Green-, Mirau-, Linnik-, Hybrid-Linnik- oder Mach-Zehnder-Interferometer ausgebildet. Das Zweistrahl-Interferometer weist folgende Komponenten auf:
- entweder mindestens eine, in mindestens einer Achsen-Richtung q schmal ausgebildete Linien-Lichtquelle
- oder ein Linien-Lichtquellen-Array, ausgebildet mit einer Vielzahl von in einer Achsen-Richtung q schmal gebildeten, zumindest näherungsweise parallelen Linienspots.

Die Achsenrichtung der schmalen Ausbildung wird hier jeweils als Quer-Achsen-Richtung (q) bezeichnet.

Weiterhin weist dieses Zweistrahl-Interferometer folgende Komponenten auf:
- ein der Linien-Lichtquelle oder dem Linien-Lichtquellen-Array nachfolgendes Lichtquellen-Objektiv zur zumindest näherungsweise beugungsbegrenzten Abbildung derselben,
- ein das Objekt abbildendes Objektiv,
- ein Detektor-Objektiv, wobei das Objektiv, welches das Objekt abbildet, und das Detektor-Objektiv auch als ein einziges Objektiv ausgebildet sein können,
- ein gerasterter Detektor mit Empfängerelementen für elektromagnetische Strahlung,
- und mindestens ein Digitalrechner mit einem Auswerteprogramm für detektierte Interferogramme zur schnellen Gewinnung von Informationen über das Objekt.

Ferner ist im Referenzarm
- entweder die Linien-Lichtquelle in der Quer-Achsen-Richtung (q) jeweils so schmal ausgebildet ist,
- oder die Linienspots des Linien-Lichtquellen-Arrays in der Quer-Achsen-Richtung (q) jeweils so schmal ausgebildet sind,
dass diese für das nachfolgende Lichtquellen-Objektiv in dieser Quer-Achsen-Richtung (q) zumindest näherungsweise gleich oder unterhalb dessen beugungsbegrenzter Lateral-Auflösung ist/sind, welche durch die numerische Apertur dieses Lichtquellen-Objektivs und der eingesetzten Lichtwellenlänge oder der eingesetzten Lichtwellenlängen der Linien-Lichtquelle oder des Linien-Lichtquellen-Arrays bestimmt ist.

Es besteht eine zumindest näherungsweise beugungsbegrenzte Abbildung der Linien-Lichtquelle oder der Linienspots des Linien-Lichtquellen-Arrays auf das Objekt mittels Objektiv. Die Airy-Breite in dieser Abbildungssituation übersteigt also zumindest näherungsweise die der schmal ausgebildeten Linien-Lichtquelle oder die Breite der schmalen Linienspots ist dieser Airy-Breite zumindest näherungsweise gleichgemacht. Die Airy-Breite in dieser Abbildungssituation ergibt sich aus der numerischen Apertur der nachfolgenden Abbildungsoptik und der eingesetzten Wellenlänge.

Weiterhin ist
- entweder ein Hybrid-Retro-Referenz-Endreflektor-System im Referenzarm R angeordnet, welches mit einem mit einem Referenzobjektiv ausgebildet ist,
- oder ein Hybrid-Retro-Referenz-Endreflektor-System angeordnet, das mit einem Objektiv ausgebildet ist, welches sich außerhalb des Referenzarms befindet.

Dieses Hybrid-Retro-Referenz-Endreflektor-System ist jeweils in Kombination mit
- entweder mit einem Hybrid-Retro-Referenz-Mikro-Endreflektor-Array mit Hybrid-Retro-Referenz-Mikro-Endreflektoren vom 90°-Dachkant-Spiegel-Typ oder 90°-Dachkant-Prismen-Typ
- oder einem Referenz-Mikro-Endreflektor-Array mit Hybrid-Retro-Referenz-Mikro-Endreflektoren vom Zylinder-Linsen-Cat' eye-Typ oder vom Zylinder-Spiegel-Cat'eye-Typ im Referenzarm ausgebildet.

Dieses Hybrid-Retro-Referenz-Endreflektor-System ist jeweils zumindest näherungsweise in der Brennebene des Referenzobjektivs oder des Objektivs, welches sich außerhalb des Referenzarms befindet, angeordnet.

Im Hybrid-Retro-Referenz-Endreflektor-System besteht jeweils ein vergleichsweise kleiner Querversatz delta_q dieser Hybrid-Retro- Referenz-Mikro-Endreflektoren vom Zylinder-Cat' eye-Typ oder vom 90°-Dachkant-Typ zumindest näherungsweise parallel zur Richtung der Quer-Achsen-Richtung (q)
- entweder in der Brennebene (BER) des dem Hybrid-Retro-Referenz-Endreflektor zugeordneten Referenzobjektivs (4, 42)
- oder in der Brennebene (BER) eines sowohl dem Referenz- als auch dem Objektarm zugeordneten Objektivs,
in der dem Detektorobjektiv vorgeordneten Brennebene (F_91_r, F_9').

So wird ein Querversatz 2 delta_q_strich von zueinander kohärenten Lichtspots (A_r", A_o") aus dem Referenz- und Objektarm eingeführt.

Der Querversatz delta_q der Hybrid-Retro-Referenz-Mikro-Endreflektoren ist maximal gleich oder kleiner dem zehnten Teil der Brennweite dieses Referenzobjektivs oder dieses Objektivs, welches sich außerhalb des Referenzarms befindet, und mindestens gleich oder größer als der objektivbezogene Airy-Scheibchen-Radius für die Schwerpunktwellenlänge im detektierten räumlichen Interferogramm gemacht.

Dem gerasterten Detektor sind vorzugsweise optische Mittel zur Einführung von Astigmatismus in den Detektionsstrahlengang vorgeordnet, welche dem Objektiv zur Abbildung des Objekts zugeordnet oder nachgeordnet sind.

**Ausführungsbeispiel 10.** Ausführungsbeispiel 10 betrifft eine Anordnung mit einem Endreflektor-Array im Referenzarm eines Zweistrahl-Interferometers.

Es handelt sich um eine Anordnung zur robusten One-shot-Interferometrie, insbesondere auch zur optischen Kohärenz-Tomografie nach dem Spatial-domain-Ansatz (SD-OCT), insbesondere zur Materialmessung und auch Tumorzellen-Erkennung. Dieses kann zur Abstandsmessung, insbesondere auch zur Messung in Verbindung mit einer 3D-Koordinaten-Messmaschine, zur zwei- oder dreidimensionalen Profilmessung, Rauheits-, Welligkeits- und Ebenheitsmessung an technischen und biologischen Objekten, insbesondere dort auch zur Tumorzellen-Erkennung mittels Erfassung des 3D-Profils von Zellen, und zur Schichtenmessung eingesetzt werden. Dazu wird ein Zweistrahl-Interferometer mit Referenz- und Objektarm, insbesondere auch in Form eines Interferenzmikroskops, eingesetzt. Im Referenzarm ist mindestens ein Endreflektor angeordnet. Das Zweistrahl-Interferometer ist insbesondere in Freistrahlausbildung und insbesondere auch als Michelson-, Twyman-Green-, Mirau-, Linnik-, Hybrid-Linnik- oder Mach-Zehnder-Interferometer ausgebildet. Das Zweistrahl-Interferometer weist folgende Komponenten auf:
- entweder mindestens eine fein ausgebildete Punkt-Lichtquelle
- oder eine fein ausgebildete Punkt-Lichtquellen-Matrix, ausgebildet mit einer Vielzahl von fein gebildeten Spots,
- ein der Lichtquelle nachfolgendes Lichtquellen-Objektiv zur zumindest näherungsweise beugungsbegrenzten Abbildung derselben oder der Spots derselben,
- ein das Objekt abbildendes Objektiv,
- ein Detektor-Objektiv, wobei das Objektiv, welches das Objekt abbildet, und das Detektor-Objektiv auch als ein einziges Objektiv ausgebildet sein können.
- ein gerasterter Detektor mit Empfängerelementen für elektromagnetische Strahlung,
- und mindestens ein Digitalrechner mit einem Auswerteprogramm für detektierte Interferogramme zur schnellen Gewinnung von Informationen über das Objekt.

Ferner
- ist im Referenzarm entweder die fein ausgebildete Punkt-Lichtquelle jeweils so fein ausgebildet,
- oder sind im Referenzarm die Spots der fein ausgebildeten Punkt-Lichtquellen-Matrix jeweils so fein ausgebildet,
dass diese für das nachfolgende Lichtquellen-Objektiv zumindest näherungsweise gleich oder unterhalb dessen beugungsbegrenzter Lateral-Auflösung ist/sind, welche durch numerische Apertur dieses Lichtquellen-Objektivs und der eingesetzten Lichtwellenlänge oder der eingesetzten Lichtwellenlängen der fein ausgebildeten Lichtquelle (198) oder der fein ausgebildeten Punkt- Lichtquellen-Matrix bestimmt ist.

Mittels eines Voll-Retro-Referenz-Mikro-Endreflektor-Systems mit einem Referenzobjektiv und mit Voll-Retro-Referenz-Mikro-Endreflektoren in jeweils
- entweder einem Mikro-Endreflektor-Array mit Voll-Retro-Referenz- Mikro-Endreflektoren vom rotationssymmetrischen Linsen-Cat' eye-Typ
- oder einem Mikro-Endreflektor-Array mit Voll-Retro-Referenz-Mikro-Endreflektoren vom rotationssymmetrischen Spiegel-Cat' eye-Typ
- oder einem Tripelprismenspiegel-Mikro-Endreflektor-Array mit Tripelprismenspiegel-Mikro-Endreflektoren
- oder einem Hohl-Tripelspiegel-Mikro-Endreflektor-Array mit Tripelspiegel-Mikro-Endreflektoren
angeordnet ist,
und bei den Mikro-Endreflektoren jeweils ein Querversatz delta_q in der Brennebene des zugeordneten Referenzobjektivs besteht, der maximal gleich oder kleiner dem zehnten Teil der Brennweite des Referenzobjektivs beträgt, und mindestens gleich oder größer als der objektivbezogene Airy-Scheibchen-Radius für die Schwerpunktwellenlänge im detektierten räumlichen Interferogramm gemacht ist.

In der dem Detektorobjektiv vorgeordneten Brennebene besteht ein Querversatz 2 delta_q oder 2 delta_q_strich von zueinander kohärenten Lichtspots (A_r", A_o") aus dem Referenz- und Objektarm.

Dem Detektor sind vorzugsweise optische Mittel zur Einführung von Astigmatismus in den Detektionsstrahlengang vorgeordnet, welche dem Objektiv zur Abbildung des Objekts zugeordnet oder nachgeordnet sind.

Weiterhin ist vorzugsweise bei einer Anordnung zur robusten One-Shot-Interferometrie die Lichtquelle als Multiwellenlängen-Lichtquelle ausgebildet. Dies ist eine Voraussetzung für die Gewinnung kurzkohärenter Interferogramme (KKI).

Weiterhin ist vorzugsweise bei einer Anordnung zur robusten One-Shot-Interferometrie die Lichtquelle als Kurzpuls-Lichtquelle ausgebildet. Mit Pulslängen im Nano-, Mikro- bis Millisekundenbereich können kurzkohärente Interferogramme auch an bewegten Objekten gewonnen werden. Es erfolgt vorzugsweise eine Synchronisierung mit dem gerasterten Detektor.

Weiterhin ist vorzugsweise bei einer Anordnung zur robusten One-Shot-Interferometrie die Lichtquelle als Frequenzkamm-Lichtquelle ausgebildet ist. Damit kann ein unbalancierten Zweistrahl-Interferometer zur Generierung von Kurz-Kohärenz-Interferenzen verwendet werden, indem der optische Gangunterschied des unbalancierten Zweistrahl-Interferometers durch die optische Verzögerungslänge Y oder ein ganzzahliges Vielfaches der optischen Verzögerungslänge Y der Frequenzkamm-Lichtquelle ausgeglichen wird.

Weiterhin ist vorzugsweise bei einer Anordnung zur robusten One-Shot-Interferometrie die Lichtquelle als durchstimmbare Frequenzkamm-Lichtquelle ausgebildet. Mittels feinem Durchstimmen der Frequenzkamm-Lichtquelle kann vorbestimmt eine Phasenschiebung in Zweistrahl-Interferogrammen erzeugt werden.

Weiterhin ist vorzugsweise bei einer Anordnung zur robusten One-Shot-Interferometrie die Rasterkonstante von gerasterter Lichtquelle und gerasterten Referenz-Endreflektoren, bezogen auf die Brennebene des Tubusobjektivs oder auf die Brennebene des objektabbildenden Objektivs, zumindest näherungsweise gleichgemacht.

Weiterhin ist vorzugsweise bei einer Anordnung zur robusten One-Shot-Interferometrie mindestens ein Mikro-Endreflektor-Array mit Voll-Retro-Mikro-Endreflektoren vom Spiegel- oder Linsen- Cat' eye-Typ angeordnet, wobei jeder Mikro-Endreflektoren um den Querversatz delta_ q - in Bezug auf den Objektarm im entfalteten Interferometer-Strahlengang - lateral versetzt ist.

Weiterhin ist vorzugsweise bei einer Anordnung zur robusten One-Shot-Interferometrie ein Mess-Abtastraster in Nagelbrett-Form, das durch eine gerasterte Punkt-Lichtquellen-Matrix vorgegeben ist, zu einem flächigen Detektor um einen spitzen Winkel gedreht angeordnet ist, wobei die Drehung der Punkt-Lichtquellen-Matrix zumindest näherungsweise um die optische Achse der optischen Anordnung im entfalteten Strahlengang ausgeführt ist.

Weiterhin ist vorzugsweise bei einer Anordnung zur robusten One-Shot-Interferometrie eine Dachkante (DK) mindestens eines 90°-Hohl-Dachkantreflektors oder der Dachkanten eines 90°-Hohl-Dachkantreflektor-Arrays zumindest näherungsweise in der Ebene BER und zumindest näherungsweise parallel zur Richtung (Io) der Längsausdehnung der Linienlichtquelle oder der feinen Linienspots des Linien-Lichtquellen-Arrays angeordnet. Diese Dachkante muss sich im wellenoptischen Schärfentiefebereich befinden, was eine Voraussetzung für die Funktion des Verfahrens darstellt.

Weiterhin ist vorzugsweise bei einer Anordnung zur robusten One-Shot-Interferometrie eine Dachkante (DK) mindestens eines 90°-Prismen-Dachkantreflektors oder eines 90°-Prismen-Dachkantreflektor-Arrays optisch - unter Berücksichtigung des Bildversatzes v - zumindest näherungsweise in der Ebene BER und zumindest näherungsweise parallel zur Richtung (Io) der Längsausdehnung der Linienlichtquelle oder der feinen Linienspots des Linien-Lichtquellen-Arrays abgebildet - bei Beobachtung vom Referenzobjektiv im Referenzraum aus.

Weiterhin ist vorzugsweise bei einer Anordnung zur robusten One-Shot-Interferometrie eine Dachkante (DK) eines 90°-Hohl-Dachkantreflektors in der Ebene FER zumindest näherungsweise senkrecht zur Richtung Io und parallel zur Richtung q angeordnet.

Weiterhin ist vorzugsweise bei einer Anordnung zur robusten One-Shot-Interferometrie eine Dachkante (DK) eines 90°-Prismen-Dachkantreflektors zumindest näherungsweise in der Ebene FER optisch - unter Berücksichtigung des Bildversatzes v - und zumindest näherungsweise senkrecht zur Richtung Io und parallel zur Richtung q abgebildet - bei Beobachtung vom Referenzobjektiv aus. Hier muss eine Frequenzkamm-Lichtquelle, vorzugsweise in Form eines Frequenzkamm-Lasers zur Kompensation des optischen Gangunterschieds zwingend eingesetzt werden.

Weiterhin sind vorzugsweise bei einer Anordnung zur robusten One-Shot-Interferometrie die Tripelpunkte (TP) der Tripel-Hohl-Reflektoren in einem Hohl-Tripel-Reflektor-Array angeordnet oder der Tripelpunkt eines einzelnen Tripel-Hohl-Reflektors ist zumindest näherungsweise in der Ebene BER angeordnet. Diese Tripelpunkte (TP) sollen sich vorzugsweise im wellenoptischen Schärfentiefebereich befinden.

Weiterhin sind vorzugsweise bei einer Anordnung zur robusten One-Shot-Interferometrie die Tripelpunkte (TP) der Tripel-Prismen-Reflektoren in einem Tripelprismen-Reflektor-Array oder der Tripelpunkt eines einzelnen Tripelprisma-Reflektors optisch - unter Berücksichtigung des Bildversatzes v -zumindest näherungsweise in der Ebene BER abgebildet - bei Beobachtung von einem Referenzobjektiv aus.

Weiterhin ist vorzugsweise bei einer Anordnung zur robusten One-Shot-Interferometrie der Brennpunkt einer rotationssymmetrischen Mikro-Linse in einem Voll-Retro-Mikro-Endreflektor als Bestandteil eines Voll-Mikro-Endreflektors jeweils zumindest näherungsweise in der Ebene BER angeordnet. Alle Brennpunkte aller rotationssymmetrischen Mikro-Linsen in jeweils einem Voll-Retro-Mikro-Endreflektor eines Voll-Mikro-Endreflektors sollen sich vorzugsweise in der Ebene BER befinden.

Weiterhin sind vorzugsweise Anordnung zur robusten One-Shot-Interferometrie die Brennpunkte der Zylinder-Mikro-Linsen in einem Hybrid-Retro-Mikro-Endreflektor als Bestandteil eines Voll-Mikro-Endreflektors jeweils zumindest näherungsweise in der Ebene BER angeordnet. Alle Brennpunkte aller Mikro-Linsen in jeweils einem Voll-Retro-Mikro-Endreflektor eines Voll-Mikro-Endreflektors sollen sich vorzugsweise in der Ebene BER befinden.

Weiterhin sind vorzugsweise brechkraftvariable Mittel zur chromatischen Tiefenaufspaltung im Objektarm angeordnet. Damit kann der wellenoptisch begründete Tiefenmessbereich wesentlich vergrößert werden.

Der erfinderische Ansatz wird in Folgendem zusammengefasst:
Es handelt sich um ein Verfahren und eine Anordnung zur robusten One-shot-Interferometrie, insbesondere auch zur optischen Kohärenz-Tomografie nach dem Spatial-domain-Ansatz (SD-OCT), an Material und lebendem Gewebe, zur Abstandsmessung, insbesondere auch zur Messung in einer Messmaschine, zur 2D- oder 3D- Profilmessung an technischen und biologischen Objekten, insbesondere auch zur Tumorzellen-Erkennung mittels Erfassung des geometrischen Zell-Profils an Gewebeoberflächen und Messung im oberflächennahen Bereich, mit einer feinstrukturierten, auf das Objekt beugungsbegrenzt abgebildeten Lichtquelle, mit einem Interferometer mit Objekt- und Referenzarm sowie einem Detektor für elektromagnetische Strahlung. Im Referenzarm ist entweder mindestens ein Hybrid-Retro-Referenz-Endreflektor oder es ist ein Array von Hybrid-Retro-Referenz-End-Reflektoren oder Array von Voll-Retro-Referenz-End-Reflektoren zur Erzeugung von verkippten Wellenfronten angeordnet. Nach dem Passieren einer Transfer-Optik durch die interferierenden Bündel entstehen bei der Detektion räumliche Interferogramme mit zueinander geneigter Referenz- und Objektwellenfront, welche zur Bestimmung von Abstand, Profil oder optischer Weglänge in einem Detektor-Frame detektiert und mittels Digitalrechner und Rechnerprogramm ausgewertet werden.

Ferner kann eine Defokussierung von Referenzlicht im Referenzarm des Zweistrahl-Interferometers erfolgen, wobei bei Letzterem der Lichtfeld-Ansatz zur Anwendung kommt. Bei der Detektion werden räumliche Interferogramme mit ringförmigen Interferenzstreifen durch eine unterschiedlich gekrümmte Referenz- und Objektwellenfront von jedem erfassten Objektpunkt gebildet und detektiert werden können, welche zur Bestimmung von Abstand, Profil oder optischer Weglänge in einem Detektor-Frame detektiert und mittels Digitalrechner und Rechnerprogramm ausgewertet werden.

Das Messsystem ist mit einer feinstrukturierten, mindestens in einer Dimension auf das Objekt beugungsbegrenzt abgebildeten Strahlungsquelle, die vorzugsweise als feine Linienquelle und die vorzugsweise als Kurzpuls-Kurzkohärenz-Lichtquelle ausgebildet, wobei definitionsgemäß deren Längsrichtung in Richtung der y-Achse ausgebildet ist, vorzugsweise mit einem Teil-Spektralbereich, der sich vom EUV- bis zum Terahertz-Bereich erstrecken kann und diese Strahlungsquelle vorzugsweise kurzkohärent ist.

Der gerasterte Detektor für elektromagnetische Strahlung ist vorzugsweise in einem spektralen Teilbereich sensitiv, der sich vom EUV- bis zum Terahertz-Bereich erstrecken kann.

Im Referenzarm ist entweder mindestens ein Hybrid-Retro-Referenz-Endreflektor, vorzugsweise in Form eines Dachkantspiegel-Reflektors, sowohl zur Erzeugung von einer oder mehreren verkippten Referenz-Wellenfronten als auch einer Wellenfron-Inversions-Kompensation entweder um seine kipp-sensitive Achse geneigt oder es besteht im Referenzarm in Verbindung mit einem Objektiv ein Querversatz des mindestens eines Dachkantspiegel-Reflektors, so dass Lateral-Shear erzeugt wird. Es können auch eindimensionale Retro-End-Reflektoren in Form von Arrays ausgebildet sein. In allen Fällen entsteht - nach anschließendem Passieren der interferierenden Bündel einer Transfer-Optik mit vorzugsweise konfokaler Diskriminierung von Objekt-Strahlung - bei der Detektion mindestens ein räumliches Zweistrahl-Interferogramm mit zueinander geneigter Referenz- und Objektwellenfront, welches zur Bestimmung von Abstand, Tiefe, Profil oder des optischen Wegs in einem einzigen Detektor-Frame detektiert und mittels Digitalrechentechnik ausgewertet wird.

Ein eindimensionaler Retro-End-Reflektor wird auch als hybrider Retro-Reflektor bezeichnet. Ein hybrider Retro-Reflektor weist entweder eine Dachkante - im Fall eines Dachkantreflektors oder eine Zylinderachse - im Fall eines Zylinder-Cat' eye-Reflektors - auf. Sowohl die Dachkante (DK) als auch eine zur Zylinder-Achse ZA parallele Linie, welche modellhaft die Ebene der Reflexion enthält, werden verallgemeinernd im Weiteren als DK-Linie (DKL) des hybriden Retro-Reflektors bezeichnet.

Die Lage des eindimensionalen Retro-End-Reflektors im Referenzarm, wenn im Objektarm ein fokussierendes Objektiv angeordnet ist, welches das Objekt abbildet, jedoch im Referenzarm kein fokussierendes Objektiv angeordnet ist, ist jeweils mit seiner DK-Linie (DKL) senkrecht zur Längsrichtung Io der Linien-Lichtquelle angeordnet.

Die Lage des eindimensionalen Retro-End-Reflektors im Referenzarm, wenn sowohl im Objektarm als auch im Referenzarm jeweils ein fokussierendes Objektiv angeordnet ist, ist jeweils mit seiner DK-Linie (DKL) parallel zur Längsrichtung lo der Linien-Lichtquelle angeordnet.

**Ausführungsbeispiel 13:** Ausführungsbeispiel 13 betrifft ein Messverfahren nach einem der obigen Ausführungsbeispiele mit einem Endreflektor im Referenzarm eines Zweistrahl-Interferometers.

Es handelt sich um ein Verfahren zur robusten One-shot-Interferometrie, insbesondere auch zur optischen Kohärenz-Tomografie nach dem Spatial-domain-Ansatz (SD-OCT) und insbesondere auch nach dem Lichtfeld-Ansatz, insbesondere zur Materialmessung und auch Tumorzellen-Erkennung. Dieses kann zur Abstandsmessung, insbesondere auch zur Messung in Verbindung mit einer 3D-Koordinaten-Messmaschine, zur zwei- oder dreidimensionalen Profilmessung, Rauheits-, Welligkeits- und Ebenheitsmessung an technischen und biologischen Objekten, insbesondere dort auch zur Tumorzellen-Erkennung mittels Erfassung des 3D-Profils von Zellen, und zur Schichtenmessung eingesetzt werden.

Dazu wird ein Zweistrahl-Interferometer mit Referenz- und Objektarm, insbesondere auch in Form eines Interferenzmikroskops, eingesetzt. Im Referenzarm ist mindestens ein Endreflektor angeordnet. Das Zweistrahl-Interferometer ist insbesondere in Freistrahlausbildung und insbesondere auch als Michelson-, modifiziertes Michelson-, Twyman-Green-, Mirau-, Linnik-, Hybrid-Linnik-, Mach-Zehnder-Interferometer oder auch als vielkanalige Interferometeranordnung ausgebildet. Das Zweistrahl-Interferometer weist folgende Komponenten auf:
- entweder mindestens eine fein ausgebildete Punkt-Lichtquelle
- oder eine fein ausgebildete Punkt-Lichtquellen-Matrix, ausgebildet mit einer Vielzahl von fein gebildeten Spots,
- mindestens ein der Lichtquelle nachfolgendes Lichtquellen-Objektiv zur zumindest näherungsweise beugungsbegrenzten Abbildung derselben oder der Spots derselben,
- mindestens ein das Objekt abbildendes Objektiv,
- mindestens ein Detektor-Objektiv, wobei das Objektiv, welches das Objekt abbildet, und das Detektor-Objektiv auch als ein einziges Objektiv ausgebildet sein können.
- mindestens ein gerasterter Detektor mit Empfängerelementen für elektromagnetische Strahlung,
- und mindestens ein Digitalrechner mit einem Auswerteprogramm für detektierte Interferogramme zur schnellen Gewinnung von Informationen über das Objekt.

Ferner
- ist entweder die fein ausgebildete Punkt-Lichtquelle jeweils so fein ausgebildet,
- oder sind die Spots der fein ausgebildeten Punkt-Lichtquellen-Matrix jeweils so fein ausgebildet,
dass diese für das nachfolgende Lichtquellen-Objektiv zumindest näherungsweise gleich oder unterhalb dessen beugungsbegrenzter Lateral-Auflösung ist/sind, welche durch numerische Apertur dieses Lichtquellen-Objektivs und der eingesetzten Lichtwellenlänge oder der eingesetzten Lichtwellenlängen der fein ausgebildeten Lichtquelle oder der fein ausgebildeten Punkt- Lichtquellen-Matrix bestimmt ist.

Dabei werden Objektpunkte des Objekts mit optischen Mitteln
- bei optisch rauen Objekten mit zumindest näherungsweise beugungsbegrenzt scharf fokussierten Bildpunkten der Punkt-Lichtquelle oder der fein ausgebildeten Punkt-Lichtquellen-Matrix beleuchtet
- oder bei optisch glatten Objekten werden auch mit schwach defokussierten Bildpunkten der Punkt-Lichtquelle oder der fein ausgebildeten Punkt-Lichtquellen-Matrix beleuchtet.

Nach der Strahlvereinigung wird mit optischen Mitteln eine geometrisch-optisch unscharfe Abbildung dieser beleuchteten Objektpunkte auf den Detektor durchgeführt und Licht von jedem Objektpunkt beleuchtet dabei einen Teilbereich mit einer Vielzahl von Detektorelementen des gerasterten Detektors. Mit optischen Mitteln wird im Referenzarm R eine geometrisch-optisch unscharfe Abbildung von Punkten der Punkt-Lichtquelle oder der fein ausgebildeten Punkt-Lichtquellen-Matrix durchgeführt und Licht von jedem leuchtenden Punkt der Punkt-Lichtquelle oder der fein ausgebildeten Punkt-Lichtquellen-Matrix beleuchtet nach der Strahlvereinigung einen Teilbereich mit einer Vielzahl von Elementen des gerasterten Detektors. Die Teilbereichsflächen auf dem gerasterten Detektor von zueinander kohärenten Strahlenbündeln aus 0 und R werden zumindest näherungsweise zueinander zur Überdeckung gebracht. Ferner wird mindestens ein räumliches Interferogramm von kohärenter Strahlung aus Referenzarm R und Objektarm 0 gebildet, wobei das Interferenzgebiet in der Lateralausdehnung mindestens 30% der maximalen Ausdehnung der beiden Teilbereichsflächen beträgt.

Am Ausgang des Zweistrahl-Interferometers werden im Raum
- nach dem Tubusobjektiv
- oder einem zumindest näherungsweise fokussierenden Objektiv
- oder einem zumindest näherungsweise fokussierenden Objektiv aus dem Beleuchtungsstrahlengang, welches auch zur Detektion mitbenutzt wird,
- sowohl Bildpunkte A_r" von der Punkt-Lichtquelle oder der fein ausgebildeten Punkt-Lichtquellen-Matrix, die über den Referenzarm R abgebildet werden,
- als auch Bildpunkte A_o" von der Punkt-Lichtquelle oder der fein ausgebildeten Punkt-Lichtquellen-Matrix, die über Objektarm 0 abgebildet werden,
   o entweder bei optisch rauen Objekten zumindest näherungsweise beugungsbegrenzt scharf abgebildet
   o oder bei optisch glatten Objekten auch schwach defokussiert abgebildet.
zumindest in der Tiefe voneinander separiert gebildet.

Die Tiefenseparierung def_r-o weist dabei im Minimum den folgenden Betrag auf:
Halbe Schwerpunktwellenlänge (lambda_S) im detektierten Signal vom räumlichen Interferogramm
dividiert

durch das Quadrat der effektiv genutzten numerischen Apertur
- entweder des Tubusobjektivs
- oder des zumindest näherungsweise fokussierenden Objektivs
- oder des zumindest näherungsweise fokussierenden Objektivs aus dem Beleuchtungsstrahlengang, welches auch zur Detektion mitbenutzt wird.

Im Maximum ist dieser Betrag def_r-o gleich der Brennweite
- entweder des Tubusobjektivs
- oder des zumindest näherungsweise fokussierenden Objektivs
- oder des zumindest näherungsweise fokussierenden Objektivs aus dem Beleuchtungsstrahlengang, welches auch zur Detektion mitbenutzt wird.

Bei optisch glatten, asphärischen Oberflächen, die etwas außerhalb des wellenoptischen Schärfentiefebereiches des Objektivs im Objektarm eines Linnik-Interferometers liegen, welches eine Lichtquelle in Form einer Punktmatrix aufweist, kann es etwas Astigmatismus in den Objektbündeln nach dem Tubusobjektiv geben, so dass dort keine scharfen Spots im Detektionsstrahlengang entstehen können. Dennoch können bei vergleichsweise geringer Ablage der Beleuchtungsspots von der Objektivbrennebene auf der Objektoberfläche im Objektarm eines Linnik-Interferometers im Detektionsstrahlengang nach einem Mikrolinsen-Array noch Lichtfeld-Interferogramme detektiert und ausgewertet werden.

**Ausführungsbeispiel 14:** Weiterhin ist vorzugsweise bei dem Verfahren zur robusten One-shot-Interferometrie gemäß dem obigen Ausführungsbeispiel 13 die Tiefenseparierung def_r-o_strich von Foki A_r", A_o" von Licht aus dem Referenzarm R und dem Objektarm O im Detektionsstrahlengang bei zumindest näherungsweise gleicher optischer Weglänge in den beiden Armen des Zweistrahl-Interferometers
- entweder durch Nutzung etwas unterschiedlicher Laufwege in unterschiedlich optisch dichten Medien im Referenz- und im Objektarm des Zweistrahl-Interferometers vorbestimmt gegeben
- oder die Separierung der Foki im Detektionsstrahlengang durch die Ausbildung schwach defokussierter Spots im Referenzarm auf dem Endreflektor vorbestimmt gegeben.

**Ausführungsbeispiel 15:** Weiterhin wird vorzugsweise bei dem Verfahren zur robusten One-shot-Interferometrie (wie z.B. bei dem Verfahren gemäß dem Ausführungsbeispiel 13 und/oder 14) vor der Detektion eine Strahlformung mit Elementen positiver oder negativer Brechkraft eines Arrays durchgeführt, dessen Brenn-Ebenen zumindest näherungsweise in einer gemeinsamen Ebene liegen und diese Brennebenen mit der Detektionsebene des gerasterten Detektors zusammenfallen oder zu dieser mittels einer optischen Transferstufe optisch konjugiert gemacht sind.

Die Elemente positiver oder negativer Brechkraft eines Arrays stellen einen optischen Fourier-Transformator dar. Es erfolgt das Durchführen einer objektpuntweisen/messpunktweisen Fourier-Transformation und es wird ein flächenhaftes räumliches Interferogramm gebildet, das auch als Lichtfeld-Interferogramm bezeichnet werden kann. Dieses wird mit einer Vielzahl von Pixeln vom gerasterten Detektor erfasst. Bei Anwendung von Schwarzschild-Objektiven stört die konstruktiv gegebene Mittenabschattung nicht, da genug aktive Pixel belichtet werden und Redundanz im räumlichen Interferogramm gegeben ist.

Es ist möglich zusätzlich zu einer Defokussierung auch noch eine Lateral-Shear in der beschriebenen Art einzuführen. Dies kann Eindeutigkeit über die Tiefen-Position eines Objektpunkts bei speziellen Anordnungen bringen, insbesondere wenn mit Licht großer Kohärenzlänge gearbeitet wird. Hierbei handelt es sich um einen Ansatz mit Trägerfrequenz, die optimal angepasst sein kann.

**Ausführungsbeispiel 16:** Weiterhin wird vorzugsweise bei dem Verfahren zur robusten One-shot-Interferometrie (wie z.B. bei dem Verfahren gemäß einem oder mehrerer der Ausführungsbeispiele 13 bis 15) aus dem sphärischen Anteil im räumlichen Interferogramm die Ringordnungszahl bestimmt. Dies ermöglicht auch den Einsatz langkohärenter Lichtquellen wie der eines HeNe-Lasers zur absoluten Formmessung.

Durch die Trennung der Foki im Detektionsstrahlengang in lateraler Weise oder in der Tiefe können bei den hier beschriebenen Ansätzen Referenzfoki im Detektionsstrahlengang auch vorbestimmt ausgeblendet werden. Damit ist es möglich, auch das Winkelspektrum des Objektlichts messpunktweise unbeeinflusst durch Referenzlicht zu erfassen, was bei wenig kooperativen Objekten zum Gewinnen weiterer Informationen über das Objekt beitragen kann. Die Erfassung des Objektlicht-Winkelspektrums erfolgt dabei vorzugsweise in einem separierten, vergleichsweise schmalen Spektral-Kanal oder in mehreren, vergleichsweise schmalen Spektral-Kanälen, um mittels verbleibender Spektral-Kanäle mit Zweistrahl-Interferenzen für die Messpunkte Kurzkohärenz-Zweistrahl-Interferogramme gewinnen zu können. Die Auskopplung des Objektlichts für das Winkelspektrum kann dabei mittels Mikro-Blenden oder Mikro-Reflektoren erfolgen. Wird beispielsweise der Rotanteil des Referenzlichts gesperrt, kann die Beobachtung im Rotkanal einer Farb- oder Hyperspektral-Kamera erfolgen. Auch die simultane Beobachtung der Objektlicht-Ausbreitung in der Tiefe, ohne das Referenzlicht merklich vorhanden ist, nach der Fokussierung von Objektlicht und simultaner Detektion in verschiedenen diskreten Tiefen ist bei diesen Ansätzen mit Separierung der Foki grundsätzlich gegeben. Dies kann genutzt werden, um auch Phase Retrieval-Verfahren zur Anwendung bringen zu können.

**Ausführungsbeispiel 17:** Ausführungsbeispiel 17, welcher mit einem der obigen Ausführungsbeispiele kombiniert wird, betrifft eine Anordnung zur robusten One-shot-Interferometrie, insbesondere auch zur optischen Kohärenz-Tomografie nach dem Spatial-domain-Ansatz (SD-OCT) und insbesondere auch nach dem Lichtfeld-Ansatz, insbesondere zur Materialmessung und auch Tumorzellen-Erkennung, zur Abstandsmessung, insbesondere auch zur Messung in Verbindung mit einer 3D-Koordinaten-Messmaschine, zur zwei- oder dreidimensionalen Profilmessung, Rauheits-, Welligkeits- und Ebenheitsmessung an technischen und biologischen Objekten, insbesondere dort auch zur Tumorzellen-Erkennung mittels Erfassung des 3D-Profils von Zellen, und zur Schichtenmessung und dazu ein Zweistrahl-Interferometer mit Referenz- und Objektarm, insbesondere auch in Form eines Interferenzmikroskops, wobei im Referenzarm mindestens ein Endreflektor angeordnet ist, insbesondere in Freistrahlausbildung und insbesondere auch als Michelson-, modifiziertes Michelson-, Twyman-Green-, Mirau-, Linnik-, Hybrid-Linnik-, Mach-Zehnder-Interferometer oder auch als vielkanalige Interferometeranordnung ausgebildet ist weist folgende Komponenten auf:
- entweder mindestens eine fein ausgebildete Punkt-Lichtquelle
- oder eine fein ausgebildete Punkt-Lichtquellen-Matrix, ausgebildet mit einer Vielzahl von fein gebildeten Spots,
- mindestens ein der Lichtquelle nachfolgendes Lichtquellen-Objektiv zur zumindest näherungsweise beugungsbegrenzten Abbildung derselben oder der Spots derselben,
- mindestens ein das Objekt abbildendes Objektiv,
- mindestens ein Detektor-Objektiv, wobei das Objektiv, welches das Objekt abbildet, und das Detektor-Objektiv auch als ein einziges Objektiv ausgebildet sein können,
- mindestens ein gerasterter Detektor mit Empfängerelementen für elektromagnetische Strahlung,
- und mindestens ein Digitalrechner mit einem Auswerteprogramm für detektierte Interferogramme zur schnellen Gewinnung von Informationen über das Objekt.

Ferner ist
- entweder die fein ausgebildete Punkt-Lichtquelle jeweils so fein ausgebildet,
- oder die Spots der fein ausgebildeten Punkt-Lichtquellen-Matrix sind jeweils so fein ausgebildet,
dass diese für das nachfolgende Lichtquellen-Objektiv zumindest näherungsweise gleich oder unterhalb dessen beugungsbegrenzter Lateral-Auflösung ist/sind, welche durch numerische Apertur dieses Lichtquellen-Objektivs und der eingesetzten Lichtwellenlänge oder der eingesetzten Lichtwellenlängen der fein ausgebildeten Lichtquelle oder der fein ausgebildeten Punkt- Lichtquellen-Matrix bestimmt ist.

Es besteht im Zweistrahl-Interferometer eine schwache Asymmetrie, hervorgerufen
- entweder durch unterschiedliche Laufwege des Lichts in unterschiedlichen Materialien unterschiedlicher Brechkraft
- und/oder durch in den beiden Interferometerarmen R und 0 ungleich verteilte Brechkräfte optischer Komponenten
   und/oder durch in den beiden Interferometerarmen ungleich in der Tiefe positionierte Komponenten mit Brechkraft.

Ferner bestehen entweder
- im Raum nach dem Tubusobjektiv
- oder einem zumindest näherungsweise fokussierenden Objektiv
- oder dem zumindest näherungsweise fokussierenden Objektiv aus dem Beleuchtungsstrahlengang, welches auch zur Detektion mitbenutzt wird,
zwei separierte Fokusebenen in der Tiefe, die zumindest näherungsweise um einen Betrag Def_strich voneinander vorbestimmt separiert sind.

Der Betrag Def_strich stellt eine durch Optik-Design, Konstruktion, Montage und einschließlich der Gerätejustierung fest eingestellte Gerätekonstante dar. Dieser Betrag Def_strich ist mindestens oder größer als die halbe Schwerpunktwellenlänge lambda_S im detektierten Signal vom räumlichen Interferogramm
dividiert durch das Quadrat der effektiv genutzten numerischen Apertur
- des Tubusobjektivs
- oder des zumindest näherungsweise fokussierenden Objektivs
- oder des zumindest näherungsweise fokussierenden Objektivs aus dem Beleuchtungsstrahlengang, welches auch zur Detektion mitbenutzt wird,
gemacht.

Dieser Betrag Def_strich ist außerdem kleiner als die Brennweite des
- Tubusobjektivs
- oder des zumindest näherungsweise fokussierenden Objektivs
- oder des zumindest näherungsweise fokussierenden Objektivs aus dem Beleuchtungsstrahlengang, welches auch zur Detektion mitbenutzt wird,
gemacht.

Es ist aber auch möglich ein stark unbalanciertes Interferometer - also mit einem signifikant von null verschiedenen optischen Gangunterschied - mit einer Frequenzkamm-Strahlungsquelle zu betreiben, um Kurzkohärenz-Interferogramme generieren zu können. Dabei können die Komponenten in den beiden Interferometerarmen R und O als refraktive, als spiegelnde oder auch als Spiegellinsen-Komponenten ausgebildet sein. Vorzugsweise sind baugleiche Komponenten im Referenzarm R und im Objektarm O eingesetzt. Vorzugsweise sind unterschiedliche Komponenten im Referenzarm R und im Objektarm O eingesetzt.

**Ausführungsbeispiel 18:** Weiterhin ist bei der Anordnung zur robusten One-shot-Interferometrie (wie z.B. bei der Anordnung gemäß Ausführungsbeispiel 17) in der Bildebene des Zweistrahl-Interferometers in der Art einer Lichtfeld-Kamera oder Plenoptic-Kamera ein optisches Array mit Elementen positiver oder negativer Brechkraft angeordnet und jedem Paar, bestehend aus einem beugungsbegrenzten Bildspot aus dem Referenzarm R und dem Objektarm 0, die jeweils kohärente Spots darstellen, ist mindestens eines dieser Elemente positiver oder negativer Brechkraft zugeordnet.

Es ist vorzugsweise ein Mikrolinsen-Array, oder ein Array von Mikro-Spiegeln mit positiver oder negativer Brechkraft angeordnet.

**Ausführungsbeispiel 19:** Weiterhin ist bei der Anordnung zur robusten One-shot-Interferometrie (wie z.B. bei der Anordnung gemäß Ausführungsbeispiel 17 und/oder Ausführungsbeispiel 18) das optische Array mit Elementen positiver oder negativer Brechkraft zumindest näherungsweise schwach gekrümmt ausgebildet. Dabei bedeutet hier "schwach gekrümmt", dass die Elemente positiver oder negativer Brechkraft (mit ihren Pupillenzentren) zumindest näherungsweise auf einer Kugelschale liegen, deren Kugelschalen-Radius größer/gleich (mindestens gleich) der Brennweite des zugehörigen Tubusobjektivs ist.

Dies dient zur Anpassung an die Rest-Bildfeldkrümmung eines Tubusobjektivs. Dabei ist es vorzugsweise auch möglich, dass die jeweilige Brennweite der Elemente positiver oder negativer Brechkraft an diese Rest-Bildfeldkrümmung adaptiert ist. Dies ermöglicht die Generierung zumindest näherungsweise ebener Referenzwellen auf einem Detektor, wodurch einerseits ein unerwünschtes Übersprechen durch Teil-Überdeckung von benachbarten Interferenzfeldern auf dem Detektor minimiert und andererseits eine gute Ausnutzung der Detektorfläche erreichbar wird.

Weiterhin entspricht bei der Anordnung zur robusten One-shot-Interferometrie die Brennweite der Elemente positiver oder negativer Brechkraft im Array zumindest näherungsweise der vorbestimmten Tiefenseparierung Def_strich.

**Ausführungsbeispiel 20:** Weiterhin befinden sich bei der Anordnung zur robusten One-shot-Interferometrie (wie z.B. die Anordnung gemäß einem oder mehrerer der Ausführungsbeispiele 17 bis 19) die fokussierten Objektbildspots A_o" zumindest näherungsweise in der Pupille der Elemente positiver oder negativer Brechkraft im Array.

**Ausführungsbeispiel 21:** Weiterhin besteht bei der Anordnung zur robusten One-shot-Interferometrie (wie z.B. die Anordnung gemäß einem oder mehrerer der Ausführungsbeispiele 17 bis 20) im Zweistrahl-Interferometer zumindest näherungsweise der optische Gangunterschied null. Somit kann eine Lichtquelle mit geringer Kohärenzlänge eingesetzt werden.

**Ausführungsbeispiel 22:** Weiterhin ist bei der Anordnung zur robusten One-shot-Interferometrie (wie z.B. die Anordnung gemäß einem oder mehrerer der Ausführungsbeispiele 17 bis 20) das Mikrolinsen-Array im Detektionsstrahlengang mit diffraktiven optischen Elementen, DOEs, auf der Basis eines schaltbaren räumlichen Lichtmodulators ausgebildet.

Damit ist es möglich, bei Einsatz eines schaltbaren räumlichen Lichtmodulators als erstes Array für die Punktlichtquelle, welches Bestandteil einer gepulsten Lichtquelle ist, eine strenge zeitliche und räumliche Synchronisation zu diesem zweiten Array im Detektionsstrahlengang durchzuführen, um das Objekt schnell lateral mit einer Folge von gepulsten Beleuchtungen, die jeweils lateral etwas versetzt sind, abtasten zu können. Dabei bleiben die diffraktiven optischen Elementen, die vorzugsweise Fresnel-Linsen mit positiver oder negativer Brechkraft darstellen, wobei jeweils nur eine Beugungsordnung genutzt wird, vorzugsweise zumindest näherungsweise zentriert zu den fokussierten Lichtspot-Paaren aus dem Zweistrahl-Interferometer. Dabei handelt es sich jeweils um räumliche Interferogramme, hier auch als Lichtfeld-Interferogramme bezeichnet, die völlig separiert voneinander ausgewertet werden können. Die mehrfach aufgenommenen Detektor-Frames mit räumlichen Interferogrammen erhöhen die Dichte der Messpunkte auf dem Objekt und können mittels geeigneter Algorithmik in einen Datensatz zusammengebracht werden, so dass eine möglichst vollständige 3D-Information über das Objekt - im Sinne der Messaufgabe - erhalten werden kann.

Es kann des Arrays, vorzugsweise ein Mikrolinsen-Array, im Detektionsstrahlengang auch als Array mit refraktiven Linsen ausgebildet sein, die mit einem Lateral-Scanner der lateralen Bewegung von Punktlichtquellen zwischen den einzelnen Belichtungen nachgeführt werden.

Bei optisch rauen Oberflächen muss sich das Messobjekt oder die erfassenden Messpunkte zumindest näherungsweise im wellenoptischen Schärfentiefebereich, auch als Depth of View (DOV) bekannt, befinden, damit ein für die Interferogramm-Auswertung hinreichend hoher Interferenzkontrast gegeben ist.

Dagegen können sich bei diesem Ansatz zur Lichtfeld-Interferometrie Objekte mit optisch glatten und stetigen Oberflächen, die sich auch außerhalb des wellenoptischen Schärfentiefebereiches der optischen Antastung befinden, erfasst werden, vorausgesetzt die Kohärenzlänge des verwendeten Lichts ist ausreichend groß gemacht. Unter dieser Voraussetzung können in diesem Fall räumliche Interferogramme beobachtet und detektiert werden. Damit ist auch ein Ansatz zur Vermessung von Asphären auf der Basis eines Michelson-Interferometers in Twyman-Green-Anordnung oder bei Mikro-Asphären oder in Teilbereichen von Asphären mit eher größeren Gradienten auch auf der Basis eines modifizierten Mirau- oder eines modifizierten Linnik-Interferometers nach dem Lichtfeld-Interferometer-Ansatz gegeben. Für diese Applikation kann es sinnvoll sein, die Anzahl der Lichtspots zu limitieren, beispielsweise auf 30 x 30 in einer Matrix-Punktlichtquelle mit angepasster zeitlicher Kohärenz der genutzten Strahlung. Die Lichtfeld-Interferogramme liefern in der Regel neben der Tiefeninformation auch noch eine Information über den Gradienten des Objekts. Dies kann für die Auswertung, speziell bei der Messung von Asphären, sehr von Vorteil sein.

In ausgewählten Fällen kann auch die Nutzung eines Fizeau-Interferometers mit dem Lichtfeld-Ansatz von Vorteil sein. Hierzu kann, wie bereits von J. Schwider in "Multiple beam Fizeau interferometer with filtered frequency comb illumination," Opt. Comm. 282, 3308-3324 (2009) beschrieben, eine Frequenzkamm-Lichtquelle eingesetzt werden, um den im Fizeau-Interferometer stets gegebenen optischen Gangunterschied zu überbrücken.

Weiterhin ist es möglich, zur Gewinnung des Winkelspektrums von Messpunkten am Objekt in Echtzeit bei Interferometern mit Asymmetrie wegen der hierbei auftretenden Fokuslagen-Differenzen zwischen Strahlenbündeln aus dem Referenzarm und dem Objektarm im Detektionsstrahlengang eine spektrale Bandsperr-Filter-Matrix von Mikrospot-Blenden im Raster der Referenz-Foki auf einem dünnen Substrat, das alle Bündel aus R und O gemeinsam passieren, permanent anzuordnen. Diese Matrix von Mikrospot-Blenden sperrt die Foki in einem Spektralbereich. Dies erfolgt vorzugsweise im blauen Spektralbereich. Eine RGB-Farbkamera oder ein Farbkamera-System detektiert dann nur im grünen und roten Bereich die räumlichen Interferenzen, jedoch im blauen Spektralbereich nur Objektlicht, da hier das zugehörige Referenzlicht geblockt wurde. Für jeden Messpunkt wird ein Bereich des Winkelspektrums vorzugsweise durch Nutzung einer Transferoptik auf dem gerasterten Detektor im blauen Spektralbereich reserviert, so dass die Winkelspektren parallel mit den räumlichen Interferenzen zur Detektion kommen. Das Winkelspektrum von separiertem Objektlicht wird vorzugsweise in der Fourier-Ebene eines Objektivs oder eines Mikrolinsen-Arrays detektiert.

Weiterhin kann Objektlicht gleichzeitig objektpunktweise in verschiedenen Tiefen beobachtet werden, um das Phase Retrieval-Verfahren anwenden zu können.

Vorzugsweise wird weiterhin durch eine vergleichsweise kleine Asymmetrie im Strahlengang des Zweistrahl-Interferometers erreicht, dass für einen Winkel im mittleren Bereich des Winkelspektrums in der Detektionsebene der optische Gangunterschied null besteht. Dabei wird in einem Mirau-Interferometer mit einem Plattenstrahlteiler, bestehend aus Teiler- und Kompensationsplatte aus demselben Werkstoff, die geometrische Weglänge im Bereich von 5 µm bis 50 µm unterschiedlich gestaltet. So können für diesen Ansatz typischerweise 10 µm oder 20 µm Differenz genügen. Es ist aber möglich, gezielt ein gechirptes Kurzkohärenz-Interferogramm (chirped white light) zu erzeugen mit geometrischen Weglängendifferenzen im Bereich bis maximal 100 µm, was je nach Spektralbereich und Werkstoff-dispersion ganz unterschiedlich sein kann. Das Ergebnis ist ein gechirptes Kurzkohärenz-Interferogramm, dessen Asymmetrie bewusst gewollt ist. Diese besteht darin, dass die beiden Streifen erster Ordnung in ihren Intensitätsmaxima stark unterschiedlich sind. Es besteht ein großer Hub auf der einen Seite des Kurzkohärenz-Wavelets - also zwischen Streifen nullter Ordnung und einer erster Ordnung) und auf der anderen Seite dieses Wavelets ein kleiner Hub. Es kann mit Vorteil der Ort des halben Hubs bestimmt werden für die z-Position eines Objektpunkts. Dies ist zwar nicht der exakte Ort des optischen Gangunterschiedes gleich null, stellt aber eine sehr gute Referenz dar. Hierbei handelt es sich um einen in der Regel akzeptablen Offset, der bei üblichen Messobjekten einen zumindest näherungsweise gleichen Wert weit im Submikrometerbereich haben sollte.

In einem Linnik-Interferometer wird vorzugsweise eine kleine Defokussierung im Referenzarm eingebracht. Der optische Gangunterschied lässt sich hierbei durch feines Verschieben des gesamten Endreflektors im Referenzarm auf einen Wert einstellen, so dass für eine Strahlneigung zumindest näherungsweise in der Mitte des Winkelbereichs des Winkelspektrums sich in der Detektionsebene der optische Gangunterschied ergibt. Für ein Linnik-Interferometer ist dies vergleichsweise einfach im Referenzarm einzustellen als eine Kombination von Defokussierung und Verschiebung des Endreflektorsystems mit Objektiv. Dies erfordert keine sehr engen Komponenten-Toleranzen wie z.B. bei einem Mirau-Interferometer bezüglich der Plattendicken. Dort besteht nur die Möglichkeit, die Einstellung des optischen Gangunterschieds null an geeigneter Stelle über die Differenz der Plattendicke von Teiler- und Kompensationsplatte und die Tiefenlage (z) des planen Referenzspiegels oder auch noch über das Einführen einer schwachen Brechkraft durch Einführen einer Flächenkrümmung am Strahlteiler-System zu bewerkstelligen. Die Verschiebung des Endreflektorsystems mit Objektiv erzeugt in der Regel einen kleinen sphärischen Phasenanteil durch Interferenzen gleicher Neigung, was aber numerisch vergleichsweise einfach zu korrigieren ist.

Bei einem weiteren Ansatz zur eindimensionalen Profilmessung, Rauheits-, Welligkeits- und Ebenheitsmessung oder zur Optischen Kohärenz-Tomografie (OCT) an technischen und biologischen Objekten mit einem Zweistrahl-Interferometer auf der Basis eines Michelson-Interferometers, welches ein objektabbildendes Messobjektiv im Objektarm aufweist, ist der Endspiegel vorzugsweise als schwach gekrümmter Spiegel ausgebildet. Dieser Ansatz arbeitet mit einer Punktlichtquelle, die sich zumindest näherungsweise auf der optischen Achse des objektabbildenden Messobjektivs befindet. Der Einsatz einer feinen Punktlichtquelle ist notwendig, da bei der Interferenz eine punktsymmetrische Wellenfrontinversion besteht. Vorzugsweise ist dieser Endspiegel im Referenzarm R des Zweistrahl-Interferometers in einer Entfernung vom Strahlteiler angeordnet, die auch zu einem optischen Gangunterschied null auf dem gerasterten Detektor führt. So können bei Einsatz einer spektral breitbandigen Lichtquelle Kurzkohärenz-Interferogramme detektiert werden. Bei optischen Komponenten zwischen Lichtquelle und Detektor mit hinreichend kleinen Aberrationen können in der Detektionsebene ringförmige Interferenzen beobachtet werden.

Vorzugsweise ist im Referenzarm R des Zweistrahl-Interferometers der Endspiegel als schwach gekrümmter Konkav-Spiegel ausgebildet. Vorzugsweise ist dessen Brennweite so ausgelegt, dass nach dem Tubusojektiv der sich ergebende Fokusspot A_r" in der vorderen Brennebene einer Mikrolinse besteht. So entsteht nach dieser eine ebene Referenzwelle zur Interferenz mit kohärentem Licht mit einer gekrümmten Wellenfront von einem im wellenoptischen Schärfevolumen erfassten Objektpunkt A_o in der Fourier-Ebene der Mikrolinse. Vorzugsweise ist das objektabbildende Messobjektiv als Schwarzschild-Spiegelobjektiv ausgebildet. Da hierbei ringförmige Interferenzen beobachtet werden, ist die bei einem Schwarzschild-Spiegelobjektiv gegebene Mittenabschattung in diesem Fall ohne Nachteil, da das Zweistrahl-Interferometer so abgeglichen werden kann, dass die Interferenzringe mit dem Interferenzring nullter Ordnung im verbleibenden Kreisring detektiert werden.

Um den optischen Gangunterschied im Zweistrahl-Interferometer auszugleichen, der besteht, wenn der gekrümmte Spiegel in einer Position angeordnet ist, die nicht zu einem optischen Gangunterschied null führt, wird vorzugsweise ein Laser mit hinreichend großer Kohärenzlänge eingesetzt. Kurzkohärenz-Interferogramme treten in diesem Fall nicht auf. Um auch Kurzkohärenz-Interferenzen detektieren zu können, wird vorzugsweise eine Frequenzkamm-Lichtquelle verwendet, die diesen optischen Gangunterschied im Zweistrahl-Interferometer in bekannter Art und Weise ausgleicht, so dass Kurzkohärenz-Interferogramme detektiert werden können.

Die in einem Zweistrahl-Interferometer mit spektral breitbandiger Lichtquelle auftretenden und nahezu unvermeidlichen chromatischen Restaberration, insbesondere die chromatischen Längsaberrationen, können vorzugsweise genutzt werden, um ein Kurzkohärenz-Interferogramm mit einem moderaten vorbestimmten, eng vergleichsweise tolerierten Chirping zu erzeugen, das zu einem asymmetrischen Intensitäts-Wavelet führt. Dabei ist das Chirping im Signal vorzugsweise so eingestellt, dass zwischen dem absoluten Maximum Kurzkohärenz-Interferogramm und einem ersten Nebenmaximum einseitig ein möglichst großer Intensitäts-Hub besteht, der beispielsweise bei hinreichender spektraler Bandbreite des detektierten Lichts und passendem Chirping durchaus 50% des Intensitätsmaximums im Kurzkohärenz-Interferogramm betragen kann. Dabei besteht vorzugsweise eine Differenz der Laufwege in brechenden Materialien oberhalb des Brechungsindexes 1,4 für Objekt-Licht und Referenz-Licht von bis zu maximal 100 µm im Zweistrahl-Interferometer im sichtbaren und nahinfraroten Bereich. Im Werkstoff BK7 beispielsweise genügt im sichtbaren Spektralbereich jedoch beispielsweise bereits eine Laufwegsdifferenz in den beiden Interferometer-Strahlengängen im unteren zweistelligen Mikrometerbereich, um eine merkliche Asymmetrie im Kurzkohärenz-Interferogramm-Wavelet zu erreichen, die dann jedoch eine spezielle Signalauswertung erfordert. Dazu kann vorzugsweise auf dem halben Wert des Intensitäts-Hubs numerisch eine gleitende Triggerschwelle gesetzt werden. Auch die Nutzung von refraktiven Mikrolinsen in einem Array für die Lichtfeld-Interferometrie kann Chirping bewirken, da Objekt-Licht und Referenz-Licht in der Regel getrennte Wege durch eine Mikrolinse gehen oder unterschiedliche Strahleinfalls-Winkel auf die Mikrolinsenfläche aufweisen.

Bei gechirpten räumlichen Interferogrammen kann bei einem hinreichend breiten Spektralbereich auf der langen Flanke zwischen dem nullten Streifen und dem kleineren Intensitätsmaximum eines Streifens erster Ordnung vorzugsweise ein hochgenauer Triggerpunkt numerisch gesetzt werden.

Weiteren Aufgaben, Merkmale und Vorteile der vorliegenden Erfindung werden offensichtlich aus der detaillierten Beschreibung einer bevorzugten Ausführungsform der vorliegenden Erfindung mit Bezug auf die Zeichnungen, welche zeigen:
- Fig. 1: einen linienhaft messenden OCT-Sensor auf der Basis eines One-shot-Interferometers;
- Fig. 2: einen weiteren linienhaft messenden OCT-Sensor auf der Basis eines One-shot-Interferometers;
- Fig. 3: ein Lichtquellen-bezogenes Koordinaten-System;
- Fig. 4: einen feiner Lichtspalt, der lateral versetzt angeordnet ist;
- Fig. 5: eine feine Punktlichtquelle in Kettenform;
- Fig. 6: eine fein ausgebildete Punktlichtquellen-Matrix;
- Fig. 7: eine feine Linien-Lichtquelle;
- Fig. 8: einen entfalteten Referenzarm R;
- Fig. 9: einen gekippten Dachkantreflektor;
- Fig. 10: einen Dachkantreflektor 5 mit einer Dachkante DK im Referenzarm;
- Fig. 11: den Referenzstrahlengang im Referenzarm R in einem modifizierten Linnik-Interferometer;
- Fig. 12: ein Linnik-Interferometer mit einer fein ausgebildeten Punkt-Lichtquellen-Matrix;
- Fig. 13: eine Anordnung zur robusten One-shot-Interferometrie mit einem Linnik-Interferometer;
- Fig. 14: eine Anordnung zur robusten One-shot-Interferometrie mit einem Mirau-Interferometer;
- Fig. 15: eine Interferometeranordnung mit einer schmalen Linienquelle;
- Fig. 16: eine Anordnung zur robusten One-shot-Interferometrie mit einem Hybrid-Interferometer;
- Fig. 17: ein Detail der in Fig. 16 gezeigten Anordnung;
- Fig. 18: ein Hybrid-Interferometer nach dem Linnik-Ansatz;
- Fig. 19: das Bild eines feines Lichtspalts;
- Fig. 20: einen Sensor für die Messung von jeweils einer sehr kurzen Profil-Linie;
- Fig. 21: einen weiteren Sensor für die Messung von jeweils einer sehr kurzen Profil-Linie;
- Fig. 22: ein Linnik-Interferometer;
- Fig. 23: kohärente Airy-Spots A_o_i' und A_r_i' sowie A_o_i+1' und A_r_i+1', die jeweils von einer feinen Lichtquelle mit feinen Lichtspots gebildet sind;
- Fig. 24: räumliche Interferogramme;
- Fig. 25: kohärente Airy-Spots A_o_i' und A_r_i' sowie A_o_i+1 ' und A_r_i+1 ';
- Fig. 27: einen Linien-Sensor in Mirau-Interferometer-Anordnung;
- Fig. 28: einen Linien-Sensor für das Erfassen der Oberfläche von menschlicher Haut;
- Fig. 29: ein Mirau-Interferometer für die Weißlicht-Interferometrie mit einem Schwarzschild-Objektiv;
- Fig. 30: den Verlauf des Weges des Mirau-Interferometers über der Zeit;
- Figur 31: den Wegverlauf in x-Richtung über der Zeit t in dem Mirau-Interfermeter;
- Fig. 32: den Scanweg an einem lebenden Organ;
- Fig. 33: eine Mirau-Interferometer-Anordnung mit einem Schwarzschild-Messobjektiv;
- Fig. 34: eine Mirau-Interferometer-Anordnung mit einem refraktiven Messobjektiv;
- Fig. 35: ein Mirau-Interferometer-System für die Messung von zwei Linienprofilen dar, die senkrecht zueinander angeordnet sind;
- Fig. 36: die Scanrichtung des Messkopfes, der sich auf einer Dreikoordinatenmessmaschine befindet;
- Fig. 37: ein Sensor-Array zur mikroskopischen 3D-Untersuchung eines menschlichen Organs;
- Fig. 38: ein Multi-Linien-Sensor mit einem 90°-Hohl-Dachkantspiegel-Mikro-Endreflektor-Array in Kombination mit einem rotationssymmetrischen Referenzobjektiv im Referenzarm in einer Mirau-Konfiguration;
- Fig. 39, 40: unterschiedliche Ansichten des 90°-Hohl-Dachkantspiegel-Mikro-Endreflektor-Arrays;
- Fig. 41: ein Sensor-Array zur mikroskopischen 3D-Untersuchung von metallischen Werkstücken;
- Fig. 42: einen Linien-Sensorkopf;
- Fig. 43: einen Teil ines Detektionsstrahlenganges;
- Fig. 44: ein Hybrid-Interferometer dar;
- Fig. 45: ein Michelson-Interferometer;
- Fig. 46: ein beispielhaftes Michelson-Interferometer mit einem das Objekt abbildendem Objektiv;
- Fig. 47: ein Michelson-Interferometer mit einem g-Ausgang dar;
- Fig. 48: ein Linnik-Interferometer mit einem "üblichem" Ausgang;
- Fig. 49: ein hybrides Linnik-Interferometer;
- Fig. 50: ein Hybrid-Linnik-Interferometer mit hier eher unerwünschten Interferenzen gleicher Neigung im Feld;
- Fig. 51: ein Hybrid-Linnik-Interferometer mit Zylinder-Spiegel-Objektiv und planem Endspiegel im Referenzarm;
- Fig. 52: ein Michelson-Interferometer mit einem Objekt-abbildendem Objektiv und einem Tubus-Objektiv;
- Fig. 53: ein Michelson-Interferometer mit einem Objekt-abbildendem Objektiv;
- Fig. 54: ein Michelson-Interferometer mit einem g-Ausgang;
- Fig. 55: ein Linnik-Interferometer zur Formmessung einer kleinen schwachen Asphäre;
- Fig. 56: ein Linnik-Interferometer mit Punktlichtquellen-Matrix-Array und Tripel-Array;
- Fig. 57: ein Voll-Retro-Referenz-Endreflektor-Array mit Tripel-Reflektor;
- Fig. 58: ein Mikro-Endreflektor-Array-System 498 mit einem Mikro-Endreflektor-Array mit Voll-Retro-Mikro-Endreflektoren vom rotationssymmetrischen Linsen-Cat'eye-Typ;
- Fig. 59: ein Mikro-Endreflektor-Array-System 499 mit einem Mikro-Endreflektor-Array mit Hybrid-Retro-Mikro-Endreflektoren vom Zylinder-Linsen-Cat 'eye-Typ;
- Fig. 60: ein Mirau-Interferometer zur Mikroformmessung gemäß einem von den Ansprüchen nicht gedeckten Beispiel;
- Fig. 61: eine Tiefenseparierung def_r-o von Bildspots im Detektionsstrahlengang des in Fig. 60 gezeigten Interferometers;
- Fig. 62: Lichtfeld-Interferenzen mit unterschiedlichen Phasenlagen;
- Fig. 63: das Mirau-Interferometer aus Figur 60, mit einem Winkel alpha_0 aus dem Winkelspektrum der Beleuchtung, für den der optische Gangunterschied bei der Detektion null wird;
- Fig. 64: Ensemble von Lichtfeld-Interferogrammen - jeweils mit einem Interferenzring nullter Ordnung - dargestellt, das von einem gerasterten Detektor 13 aufgenommen wird;
- Fig. 65, 66: Kurzkohärenz-Wavelets mit vorbestimmt eingeführtem Chirping;
- Fig. 67: einen Interferometeraufbau, der mit gepulster Beleuchtung betrieben wird;
- Fig. 68: räumliche Interferograme;
- Fig. 69: die Ausblendung des Referenzlichts bei dem in Fig. 67 gezeigten Interferometer;
- Fig. 70: ein Linnik-Interferometer mit einer fein ausgebildeten Punkt-Lichtquellen-Matrix;
- Fig. 71: ein Twyman-Green-Interferometer dargestellt, bei dem die gewünschte Asymmetrie durch ungleiche optische Wege in unterschiedlichen optischen Materialien erzeugt ist;
- Fig. 72: ein Mikrolinsen-Array;
- Fig. 73: ein Zweistrahlinterferometer mit einem objektabbildenden Schwarzschild-Objektiv im Objektarm;
- Fig. 74, 75: eine miniaturisierte Variante eines Single-Shot-Ansatzes in Form eines vielkanaligen Linnik-Interferometers.
- Fig. 76: einen vielkanaligen Mirau-Interferometer-Ansatz.

**Bezugszeichenliste mit Erläuterungen**

| **Bezugszeichen** | **Bezeichnung** |
|---|---|
| 1 | feine Linienquelle im NIR mit Längsrichtung in y-Richtung, vorzugsweise spektral breitbandig, in Querrichtung q mit Breite unterhalb der beugungsbegrenzten Lateral-Auflösung der jeweils nachfolgenden Optik |
| 101 | gepulste Linienquelle im NIR, vorzugsweise spektral breitbandig, in Querrichtung q mit Breite unterhalb der beugungsbegrenzten Lateral-Auflösung der jeweils nachfolgenden Optik |
| 102 | feine punktförmige Lichtquelle, in Querrichtung q mit Breite unterhalb der beugungsbegrenzten Lateral-Auflösung der jeweils nachfolgenden Optik |
| 103 | feine Linienquelle ausgebildet mittels Frequenzkamm-Lichtquelle, vorzugsweise ein Frequenzkamm-Laser (FCL), spektral breitbandig feine Linienquelle, vorzugsweise spektral breitbandig gepulste feine Linienquelle, vorzugsweise spektral breitbandig |
| 104 | räumlicher Lichtmodulator, DMD, Digital micro mirror device, erzeugt Lichtmuster an oder unterhalb der lateralen Auflösung der nachfolgenden Optik zur Abbildung derselben in den Objektraum |
| 105 | räumlicher Lichtmodulator, transmissives oder reflexives LCD, erzeugt feine Lichtmuster an oder unterhalb der lateralen Auflösung der nachfolgenden Optik zur Abbildung derselben in den Objektraum |
| 187 | Lichtquelle mit zwei Pinhole-Ketten um 90° zueinander gedreht, gespeist von einer gepulsten, spektral breitbandigen Lichtquelle |
| 188 | feine Matrix-Lichtquelle mit feinen Lichtspots jeweils ein Spot am Ende einer Single-Mode-Faser. Die Faserenden sind in einer Matrix-Struktur angeordnet. Das Licht wird in die Fasern von spektral vergleichsweise breitbandigen fasergekoppelter Superlumineszenz-Dioden eingekoppelt. Die sich ergebende effektive Lichtspotgröße ist unterhalb der beugungsbegrenzten Auflösung der nachfolgenden Optik. |
| 189 | feine Linien-Lichtquelle mit feinen Lichtspots, erzeugt mittels spektral breitbandiger LEDs und Pinholes. Die Spotgröße, hier die Pinhole-Größe, ist unterhalb der beugungsbegrenzten Auflösung der nachfolgenden Optik. |
| 190 | feine Matrix-Lichtquelle mit feinen Lichtspots, erzeugt mittels spektral breitbandiger LED-Matrix und nachgeordneten Pinholes. Die Spotgröße, hier die Pinhole-Größe, ist unterhalb der beugungsbegrenzten Auflösung der nachfolgenden Optik. |
| 191 | feine Linien-Lichtquelle mit räumlichem rechnergesteuerten Lichtmodulator ausgebildet. Ermöglicht die rechnergesteuerte Lateralverschiebung von einem oder mehreren feinen Lichtspalten, die unterhalb der beugungsbegrenzten Auflösung der nachfolgenden Optik sind. |
| 192 | feine Linien-Lichtquelle, vorzugsweise spektral breitbandig |
| 193 | gepulste feine Linien-Lichtquelle (Pulslängen im Millisekundenbereich, bzw. im einstelligen Mikrosekundenbereich) vergleichsweise spektral breitbandig und mit einem Frequenzkamm-Laser (FCL) mit der optischen Verzögerungslänge Y dargestellt, |
| 194 | feiner Spalt mit Single-Mode Laser-Strahlung beleuchtet, in Querrichtung q mit Breite unterhalb der beugungsbegrenzten Lateral-Auflösung der jeweils nachfolgenden Optik |
| 195 | feine Punktlichtquellen-Kette, vorzugsweise spektral breitbandig, in Querrichtung q mit Breite unterhalb der beugungsbegrenzten Lateral-Auflösung der jeweils nachfolgenden Optik |
| 196 | feiner Lichtspalt in y-Richtung, Licht vorzugsweise spektral breitbandig, in Querrichtung q mit Breite unterhalb der beugungsbegrenzten Lateral-Auflösung der jeweils nachfolgenden Optik |
| 197 | Linien-Lichtquellen-Array (gerastert) mit mehreren feinen, zumindest näherungsweise parallelen Linien-Spots, vorzugsweise spektral breitbandig, in Querrichtung q mit Breite der Linien-Spots unterhalb der beugungsbegrenzten Lateral-Auflösung der jeweils nachfolgenden Optik. Das Linien-Lichtquellen-Array kann auch mittels räumlichen Lichtmodulators elektronisch steuerbar ausgebildet sein. |
| 198 | fein ausgebildete Punkt-Lichtquelle, vorzugsweise spektral breitbandig, mit Ausdehnung der Lichtspots unterhalb der beugungsbegrenzten Lateral-Auflösung der jeweils nachfolgenden Optik |
| 199 | fein ausgebildete (gerasterte) Punkt-Lichtquellen-Matrix, vorzugsweise spektral breitbandig, mit Ausdehnung der Lichtspots unterhalb der beugungsbegrenzten Lateral-Auflösung der jeweils nachfolgenden Optik |
| LS | feiner Linienspalt, mit Breite unterhalb der beugungsbegrenzten Lateral-Auflösung der jeweils nachfolgenden Optik |
| LSO' | Bild des feines Linienspalts im Objektraum |
| LSR' | Bild des feines Linienspalts im Referenzraum |
| LSR" | Bild des feines Linienspalts im Referenzraum nach erster Reflexion am Hohl-Dachkantreflektor |
| 2 | Kollimator-Objektiv, Lichtquellenobjektiv |
| 21 | fokussierendes Lichtquellen-Objektiv |
| 23 | linienhaftes Array von Mikro-Kollimator-Objektiven |
| 24 | flächenhaftes Array von Mikro-Kollimator-Objektiven |
| 3 | Strahlteiler, allgemein |
| 31 | Strahlteilerschicht in einem Mirau-Interferometer, auf einer Platte |
| 32 | Strahlteilerschicht in einem Mirau-Interferometer mit Kompensations-Platte |
| 33 | Platte in einem Strahlteiler, die Strahlteilerschicht tragend |
| 34 | Kompensationsplatte in einem Strahlteiler |
| 35 | Auskoppel-Strahlteiler |
| 36 | Auskoppel-Strahlteiler |
| 361 | Strahlteilerschicht |
| 37 | Strahlteilerwürfel |
| 38 | Strahlteilerschicht für Michelson-lnterferometer für interferometrisches Referenzsystem für Lageregelung des hochdynamischen Linear-Antriebs für Referenzobjektiv |
| 39 | Strahlteiler-System mit mehreren Einzel-Strahlteilern |
| 391 | Strahlteilerpatte mehrere Strahlteiler-Schichten tragend |
| 4 | rotationssymmetrisches Referenz-Schwarzschildobjektiv im Hybrid Retro-Referenz-Endreflektor im Referenzarm |
| 401 | Mikrolinsen-Array in Linienform im Referenzarm eines vielkanaligen Linnik-Interferometers |
| 402 | Mikrolinsen-Array in Matrix-Form im Referenzarm eines vielkanaligen Linnik-Interferometers |
| 407 | rotationssymmetrische Mikrolinse in einem Mikrolinsen-Array in Linienform 401 im Referenzarm R eines vielkanaligen Linnik-Interferometers |
| 408 | rotationssymmetrische Mikrolinse in einem Mikrolinsen-Array in Matrix-Form 402 im Referenzarm R eines vielkanaligen Linnik-Interferometers |
| 41 | Zylinder-Spiegel-Objektiv im ZSHRRER (44), Zylinder-Objektiv-Achse (CA) ist senkrecht zur Längsrichtung lo der Linienquelle 1 |
| 42 | rotationssymmetrisches Referenzobjektiv im Referenz-Endreflektor, auch in 45, 46, 47, 471 angeordnet |
| 421 | ebener Interferometer-Endspiegel im Referenzarm R eines Linnik-Interferometers |
| 422 | ebener Interferometer-Endspiegel im Referenzarm R eines Twyman-Green-Interferometers |
| 423 | Konkaver, rotationssymmetrischer Endspiegel im Referenzarm eines Hybrid-Linnik-Interferometers |
| 424 | ebener Interferometer-Endspiegel im Referenzarm eines vielkanaligen Linnik-Interferometers |
| 43 | Planspiegel im Zylinder-Objektiv (41) im Zylinderspiegel-Hybrid-Retro-Referenz-Endreflektor (ZSHRRER) |
| 44 | Zylinder-Spiegel-Hybrid-Retro-Referenz-Endreflektor (ZSHRRER) |
| | Ein ZSHRRER stellt auch einen (speziellen) hybriden Retro-Reflektor dar, der im Referenzarm als Endreflektor eingesetzt wird und eine spiegelsymmetrische Wellenfront-Inversion erzeugt. |
| | Der ZSHRRER ist schwach verkippt um eine Achse, die parallel zur Längsrichtung (optischer Strahlengang des Interferometers entfaltet) der Linienquelle und senkrecht zur Zylinder-Objektiv-Achse ist. |
| 441 | Zylinder-Linsen-Hybrid-Retro-Referenz-Endreflektor (ZLHRRER) |
| 45 | Hybrid-Retro-Referenz-Endreflektor (HRRER), Oberbegriff ohne Spezifikation |
| 46 | Hybrid-Retro-Referenz-Endreflektor-System mit einem Hohl-90°-Dachkantreflektor-Array (564) und einem rotationssymmetrischen Referenzobjektiv 42, 4 im Referenzarm (R) |
| 460 | Hybrid-Retro-Referenz-Endreflektor-System mit einem 90°-Mikro-Dachkantprismen-Spiegel-Endreflektor-Array (565)und einem rotationssymmetrischen Referenzobjektiv 42, 4 im Referenzarm (R) |
| 461 | Hybrid-Retro-Referenz-Endreflektor-System mit einem 90°-Hohl-Dachkantreflektor-Array (564) und einem rotationssymmetrischen Objektiv 61, 62 oder 91 außerhalb des Referenzarms R |
| 462 | Hybrid-Retro-Referenz-Endreflektor-System mit einem 90°-Mikro-Dachkantprismen-Spiegel-Endreflektor-Array (565) und einem rotationssymmetrischen Objektiv 61, 62 oder 91 außerhalb des Referenzarms R |
| 47 | Voll-Retro-Referenz-Endreflektor (VRRER) mit einem Hohl-Tripel-Reflektor (567) und einem rotationssymmetrischen Referenzobjektiv 42 |
| 471 | Voll-Retro-Referenz-Endreflektor-System (VRRERS) mit einem Hohl-Tripel-Reflektor-Array (566) und einem rotationssymmetrischen Referenzobjektiv 42 |
| 480 | Mikro-Planspiegel in einem Voll-Retro-Referenz-Mikro-Endreflektor |
| 481 | Voll-Retro-Referenz-Mikro-Endreflektor vom rotationssymmetrischen Linsen-Cat's eye-Typ |
| 482 | Voll-Retro-Referenz-Mikro-Endreflektor vom rotationssymmetrischen Spiegel-Cat's eye-Typ |
| 483 | Hybrid-Retro-Referenz-Mikro-Endreflektor vom Zylinder-Linsen-Cat's eye-Typ |
| 484 | Hybrid-Retro-Referenz-Mikro-Endreflektor vom Zylinder-Spiegel-Cat's eye-Typ |
| 485 | rotationssymmetrische Mikro-Linse in einem Voll-Retro-Referenz-Mikro- Endreflektor 481 |
| 486 | Zylinder-Mikro-Linse in einem Hybrid-Retro- Referenz-Mikro-Endreflektor 483 |
| 491 | Mikro-Endreflektor-Array mit Voll-Retro-Referenz-Mikro- |
| 492 | Referenz-Mikro-Endreflektor-Array mit Voll-Retro-Mikro-Endreflektoren vom rotationssymmetrischen Spiegel- Cat's eye-Typ |
| 493 | Referenz-Mikro-Endreflektor-Array mit Hybrid-Retro-Referenz-Mikro-Endreflektoren vom Zylinder-Linsen- Cat's eye-Typ |
| 494 | Referenz-Mikro-Endreflektor-Array mit Hybrid-Retro-Referenz-Mikro-Endreflektoren vom Zylinder-Spiegel- Cat's eye-Typ |
| 498 | Voll-Retro-Referenz-Mikro-Endreflektor-System |
| 499 | Hybrid-Retro-Referenz-Mikro-Endreflektor-Systern |
| | Der Referenz-Endreflektor 45 und die Referenz-Endreflektor-Arrays 481 bis 484 und 491 bis 494 sind vorzugsweise mit einem feinen rechnersteuerbaren Lateral-Translator ausgebildet, der vorzugsweise mit dem räumlichen Lichtmodulator 104, 105 zum Anfahren definierter Messpositionen auf dem Objekt (71, 72, 73, 74) synchronisiert ist. Dies dient zur schnellen lateralen Abtastung des Messfeldes durch mehrere Messungen in den "Zwischenräumen", um einen quasi-Vollflächen 3D-Datensatz vom Objekt (71, 72, 73, 74) gewinnen zu können. |
| 5 | 90°-Hohl-Dachkantreflektor, alleinstehend im Referenzarm, sehr enge 90°-Toleranz, Dachkante ist parallel zur Längsrichtung lo der Linien-Lichtquelle und senkrecht zur Querrichtung q |
| 51 | Umlenkspiegel |
| 52 | Umlenkspiegel |
| 53 | Umlenkspiegel |
| 54 | Umlenkspiegel |
| 55 | Umlenkspiegel |
| 56 | 90°-Hohl-Dachkant-Endreflektor in Verbindung mit einem rotationssymmetrischen Objektiv (62, 91) außerhalb des Interferometers 90°, einen Hybrid-Retro-Referenz-Endreflektor bildend |
| 561 | 1. Hohl-90°-Dachkant-Endreflektor im Referenzarm in Kombination mit einem rotationssymmetrischen Referenzobjektiv 42 im HRRER, eher geringe 90°-Toleranz, Dachkante in y-Richtung stehend, Dachkante ist parallel zur Längsrichtung lo der Linien-Lichtquelle und senkrecht zur Querrichtung q |
| 562 | 2. Hohl-90°-Dachkant-Endreflektor im Referenzarm in Kombination mit einem rotationssymmetrischen Referenzobjektiv 42 im HRRER, Dachkante in x-Richtung angeordnet (hier liegend), 90°-Winkel-Toleranz eher gering |
| 564 | 90°-Hohl-Dachkantspiegel-Mikro-Endreflektor-Array in Kombination mit einem rotationssymmetrischen Referenzobjektiv 42 im Referenzarm |
| 565 | 90°-Dachkantprismenspiegel-Mikro-Endreflektor-Array in Kombination mit einem rotationssymmetrischen Referenzobjektiv 42 im Referenzarm |
| 566 | Hohl-Tripelspiegel-Mikro-Endreflektor-Array (Jeder Tripel-Mikro-Endreflektor ist jeweils als Raumecke ausgebildet.) |
| 567 | Hohl-Tripelspiegel-Mikro-Endreflektor (Raumecke) |
| 568 | Tripeiprismenspiegel-Mikro-Endreflektor-Array |
| | Alle Mikro-Endreflektor-Arrays 564 bis 566 und 568 sind vorzugsweise mit einem feinen rechnersteuerbaren Lateral-Translator ausgebildet, der vorzugsweise mit dem räumlichen Lichtmodulator 104, 105 synchronisiert ist. Dies dient zur schnellen lateralen Abtastung des Messfeldes durch mehrere Messungen, um einen Vollflächen- oder quasi-vollflächen 3D-Datensatz vom Objekt 71 bis 74 gewinnen zu können. |
| 569 | Kompensationsplatte im Objektarm (Platte zur zumindest näherungsweisen Kompensation der Dicke d des optischen Mediums für ein 90°-Dachkantprismen-Reflektor-Array, 90°-Dachkantprisma (58) oder Tripel-Prisma oder für Cat'eye-Linsen, auch für ein Cat'eye-Linsen-Array) |
| 57 | 90°-Hohl-Leichtbau-Dachkant-Endreflektor in Kombination mit einem rotationssymmetrischen Objektiv 42 im RHRER, eher geringe 90°-Winkel-Toleranz |
| 58 | 90°-Dachkantprisma im Objektiv 42, 64 jeweils feststehend, 90°-Winkel-Toleranz des 90°-Dachkantprismas eher groß |
| 581 | Um 90° gedrehtes 90°-Dachkantprisma im Objektiv 42, 64 feststehend |
| 59 | 90°-Dachkantprisma bewegt in z-Richtung im Referenzarm |
| 6 | rotationssymmetrisches Schwarzschildobjektiv im Objektarm O, dem Messstrahlengang |
| 61 | rotationssymmetrisches Objektiv dem Michelson- Interferometer zugeordnet in zweifachem Strahldurchgang genutzt |
| 62 | rotationssymmetrisches Objektiv am Interferometer-Ausgang in einfachem Strahldurchgang genutzt, Detektorobjektiv, Objektiv 62 bildet Objekt nach Unendlich ab |
| 63 | Schwarzschild-Messobjektiv als Bestandteil eines Mirau-Interferometers, rotationssysmmetrisch, |
| 64 | Messobjektiv als Bestandteil eines Mirau-Interferometers, rotationssysmmetrisch, |
| 651 | Mikrolinsen-Array in Linienform im Objektarm eines vielkanaligen Linnik-Interferometers |
| 652 | Mikrolinsen-Array in Matrix-Form im Objektarm eines vielkanaligen Linnik-Interferometers |
| 653 | Mikrolinsen-Array in Matrix-Form in einem vielkanaligen Mirau-Interferometer |
| 654 | rotationssysmmetrische Mikrolinse in einem Mikrolinsen-Array in Matrix-Form in einem vielkanaligen Mirau-Interferometer |
| 657 | rotationssysmmetrische Mikrolinse in einem Mikrolinsen-Array in Linien-Form im Objektarm eines vielkanaligen Linnik-Interferometers |
| 658 | rotationssysmmetrische Mikrolinse in einem Mikrolinsen-Array in Matrix-Form im Objektarm eines vielkanaligen Linnik-Interferometers |
| 66 | Messobjektiv, rotationssysmmetrisch, ansonsten unspezifiziert, hier i.d.R. in einem Linnik-Interferometer |
| 67 | Mirau-Objektiv-Interferometer mit Messobjektiv 64, rotationssymmetrisch |
| 68 | Mirau-Objektiv-Interferometer mit rotationssysmmetrischem Schwarzschild-Objektiv 63 |
| 681 | ebener Referenzspiegel in einem Mirau-Objektiv-Interferometer |
| 69 | geführtes Sensor-Array, bestehend aus einer Vielzahl von Mirau-Systemen - mindestens jedoch zwei - ausgebildet mit 90°-Hohl-Dachkantreflektor 56 |
| 71 | menschliche Haut |
| 72 | lebendes Organ |
| 73 | raue Metalloberfläche |
| 74 | optisch glatte Metalloberfläche |
| 75 | polierte Asphärenoberfläche, optisch glatt |
| 8 | astigmatisches Abbildungssystem |
| 81 | astigmatisches Abbildungssystem mit zwei rotationssysmmetrischen Objektive in 4f-Anordnung, eines davon das Tubusobjektiv 9, mit nachgeordnetem Zylinder-Objektiv 12, dessen Brennebene mit der letzten Brennebene der 4f-Anordnung zusammenfällt, wo sich der gerasterte Detektors 13 befindet. |
| 82 | astigmatisches Detektor-Objektiv, bestehend aus einem rotationssymmetrischen Objektiv 11 und einem Zylinder-Objektiv 12 |
| 821 | astigmatisches Detektor-Objektiv für zweiten Kanal, bestehend aus einem rotationssymmetrischen Objektiv 11 und einem Zylinder-Objektiv 12 |
| 83 | Schwarzschild-Zylinder-Objektiv für Detektor 13 |
| 84 | Mikrolinsen-Array arbeitet als optischer Fourier-Prozessor |
| 841 | Mikrolinse in einem Mikrolinsen-Array |
| 842 | Mikrolinse |
| 85 | kleines Spiegelobjektiv als Fourier-Transformations-Komponente |
| 86 | Spiegelobjektiv-Array als Fourier-Transformations-Array |
| 87 | 4f-Transferstufe |
| 88 | Rot-Durchlass-Filter |
| 89 | Mikro-Rotsperrfilter-Array |
| 891 | Mikro-Rot-Sperrfilter |
| 9 | rotationssymmetrisches Tubusobjektiv |
| 901 | Tubus-Mikrolinsen-Array in Linienform im Detektionsstrahlengang eines vielkanaligen Linnik-Interferometers |
| 902 | Tubus-Mikrolinsen-Array in Matrix-Form im Detektionsstrahlengang eines vielkanaligen Linnik-Interferometers |
| 903 | Tubus-Mikrolinsen-Array in Matrix-Form im Detektionsstrahlengang eines vielkanaligen Mirau-Interferometers |
| 907 | rotationssymmetrische Tubus-Mikrolinse in einem Tubus-Mikrolinsen-Array in Linienform |
| 908 | rotationssymmetrische Tubus-Mikrolinse in einem Tubus-Mikrolinsen-Array in Matrix-Form |
| 95 | feine Spaltblende für Raumfilterung von Licht aus dem Referenzarm kommend |
| 96 | feine Spaltblende für Raumfilterung von Licht aus dem Objektarm kommend |
| 961 | feine Spaltblende dient auch der Unterdrückung des Hintergrundlichts aus der Tiefe von angemessenen Haut |
| 97 | feines Spaltblenden-Array für Raumfilterung von Licht vom Objekt |
| 98 | planer Umlenkspiegel |
| 99 | planer Umlenkspiegel mit feiner Spaltblende |
| 11 | rotationssymmetrisches Objektiv |
| 111 | rotationssymmetrische Mikro-Linse in einem Mikro-Linsen-Array |
| 115 | rotationssymmetrisches Objektiv |
| 116 | hochdynamischer Linear-Antrieb für Mirau-Objektiv |
| 117 | hochdynamischer Linear-Antrieb für 90°-Dachkant-Prisma, vorzugsweise ein Piezo-Steller |
| 118 | hochdynamischer Linear-Antrieb für Referenzobjektiv |
| 119 | hochdynamischer Linear-Antrieb für Messobjektiv |
| 12 | Zylinder-Objektiv |
| 121 | Zylindrische Mikro-Linse in einem Zylinder-Mikro-Linsen-Array |
| 122 | Single-mode-Laser. für interferometrisches Referenzmesssystem als Sensor für Lageregelung des Antriebs für das Referenzobjektiv 104 |
| 128 | astigmatisches Abbildungssystem für Referenzmesssystem mit Vierfach-Fotodioden-Block |
| 129 | Spiegel-Zylinder-Objektiv |
| 13 | gerasterter Detektor für elektromagnetische Strahlung |
| 132 | Ein zweiter gerasterter Detektor für elektromagnetische Strahlung, Chip einer Digital-Kamera, |
| 133 | Beobachtungs-Kamera |
| | |
| A | leuchtender Punkt auf der Lichtquelle oder im leuchtenden Lichtspalt |
| A_r' | Bild eines leuchtenden Punkts im Referenzarm R |
| A_o' | Bild eines leuchtenden Punkts im Objektarm 0 |
| A_r" | Bild eines leuchtenden Punkts im Detektionsstrahlengang, aus dem Referenzarm R kommend |
| A_o" | Bild eines leuchtenden Punkts im Detektionsstrahlengang, aus dem Objektarm 0 kommend |
| | Die Bilder A_o" und A_r" eines leuchtenden Punkts sind optisch konjugiert zueinander. |
| alpha_y_source | Aperturwinkel, der zumindest näherungsweise vom beugungsbegrenzten Lichtquellenabbildungssystem noch erfasst wird. |
| alpha_0 | Aperturwinkel im Winkelspektrum der Beleuchtung, bei dem der optische Gangunterschied auf dem gerasterten Detektor 13 null wird. |
| ASL | Airy-Scheibchen-Symmetrie-Linie |
| aTS | adaptive Triggerschwelle, entspricht einem Referenzpunkt bei einem Kurzkohärenz-Interferogramm mit Chirping in der Umgebung des Streifens nullter Ordnung, der noch klar identifizierbar ist |
| BER | Äußere Brennebene (dem Strahlteiler abgewandte Brennebene) des Referenzobjektivs im Referenzraum R |
| b_spot | Breite der Linienlichtquelle |
| b_spot_g' | geometrisch-optisch abgebildete Breite der Linienlichtquelle im Objektraum |
| b_spot | Linien-Spot-Breite des leuchtenden Linien-Spots |
| b_spot_g' | geometrisch-optisch abgebildete Breite des leuchtenden Linien-Spots b_spot der Lichtquelle im Objektraum |
| 2 b1_Airy | volle Breite bis zu den beiden ersten Nullstellen im Airy-Muster im Objektraum, hier auch als volle Airy-Breite bezeichnet |
| CA | Zylinderachse bei einem zylindrischen Objektiv |
| d_r | geometrische Dicke der Strahlteilerplatte im Referenzarm R in einem Mirau-Interferometer |
| d_o | geometrische Dicke der Strahlteilerplatte im Objektarm O in einem Mirau-Interferometer |
| DK | Dachkante eines Dachkantreflektors |
| DKL | Dachkant-Linie des Hybrid-Retro-Referenz-Endreflektors (im Referenzarm) mit spiegelsymmetrischer Wellenfront-Inversion (spiegelsymmetrisch) |
| HRRER | Hybrid-Retro-Referenz-Endreflektor (im Referenzarm) mit spiegelsymmetrischer Wellenfront-Inversion |
| 2 delta_beta_tilt | Verkippungswinkel der interferierenden Wellenfronten im Referenzarm nach Passieren des Hybrid-Retro-Referenz-Endreflektors mit spiegelsymmetrischer Wellenfront-Inversion, Kippachse ist die y-Achse. |
| 2 delta_beta'_tilt | Verkippungswinkel der interferierenden Wellenfronten bei der Detektion, vorzugsweise auch als Zylinderwellen ausgebildet und dabei der Verkippungswinkel zwischen den Fokuslinien der Zylinderwellen, Kippachse ist die y-Achse. |
| delta_beta_HRRER | Verkippung des Hybrid-Retro-Referenz-Endreflektors (im Referenzarm) ist gleich beta_tilt, Kippachse ist die y-Achse. |
| delta_beta_DK | Verkippung der Dachkante um y-Achse |
| delta_d | geometrische Dickendifferenz der beiden Strahlteilerplatten in einem Mirau-Interferometer |
| delta_q | Querversatz des Hybrid-Retro-Referenz-Endreflektors |
| 2 delta_q = s | Lateral-Shear zwischen einer Objekt- und einer Referenz- Wellenfront im Referenzarm nach Passieren des Hybrid-Retro-Referenz-Endreflektors |
| 2 delta_q_strich = s' | Lateral-Shear unter Berücksichtigung des Abbildungsmaßstabes der Optik am Ausgang des Zweistrahl-Interferometers zwischen einem Objekt- (A_o") und einem optisch konjugierten Referenzpunkt (A_r") als die notwendige Voraussetzung für eine Verkippung jeweils von Objekt- und Referenz-Wellenfront zueinander in der Ebene des gerasterten Detektors 13 |
| d_H | Durchmesser des Strahlenbündels auf dem Hybrid-Retro-Referenz Endreflektor in y-Richtung |
| DK_hollow | Dachkante im hohlen 90°-Dachkant-Spiegelreflektor |
| Dm_spot | Durchmesser eines leuchtenden Punkt-Spots der Lichtquelle |
| Dm_spot_g' | Durchmesser des geometrisch-optischen Bildes eines leuchtenden im Objektraum, der geometrisch-optisch abgebildet ist |
| DOV | wellenoptischer Schärfentiefereich des Sensors (depth of field) |
| def_r-o | aktuelle, Objektpunkt-abhängige (variable) Tiefenseparierung (z-Richtung) von einem Referenzbildpunkt und einem Objektbildpunkt im Detektionsstrahlengang nach dem Objektiv (62, 91, 9). Dies stellt eine sowohl durch Konstruktion und Justierung als auch durch die aktuelle Objektpunktlage gegebene Größe dar. def_r-o1 beschreibt dann die Tiefenseparierung der Bildpunkte im Detektionsstrahlengang, die aus dem leuchtenden Punkt A1 der Punktlichtquelle 199 gebildet sind. |
| def_r-o_strich | aktuelle, Objektpunkt-abhängige (variable) Tiefenseparierung (z-Richtung) von einem Referenzbildpunkt und einem Objektbildpunkt im Detektionsstrahlengang in einer weiteren Abbildungssituation nach dem Objektiv (9, 62, 91, ggf. auch 2 im Rückwärtsstrahlengang). def_r-o_strich stellt eine sowohl durch Konstruktion und Justierung als auch durch die jeweils aktuelle Objektpunktlage gegebene variable Größe dar. |
| Def | feste Tiefenseparierung (z-Richtung) der Foki im Interferometer als Bilder der Punktlichtquelle, bezogen auf einen entfalteten Interferometerstrahlengang. Def stellt eine durch Konstruktion und Justierung gegebene feste Größe im optischen System dar. |
| Def_strich | feste Tiefenseparierung (z-Richtung) der Foki im Detektionsstrahlengang, Def_strich kann hier der Brennweite der Mikrolinse 841 entsprechen. |
| Def_strich_2 | feste Tiefenseparierung (z-Richtung) der Foki im Detektionsstrahlengang nach einer weiteren Abbildung, üblicherweise nach dem Tubusobjektiv 9 |
| E_R | Ebene, wo die Reflexion der Referenzwellen lokalisiert ist. |
| EFL | Ebene E_xy im gewählten Koordinatensystem, welches fest der Linien-Lichtquelle zugeordnet ist |
| E_xz | Ebene E_xz im gewählten Koordinatensystem, welches fest der Linien-Lichtquelle zugeordnet ist |
| F, F', F" | Brennpunkte |
| F, auch F_o und F_r | Brennpunkt im Objektraum 0, beziehungsweise im Referenzraum R Brennpunkte im Objektraum sind stets ohne Strich, Dagegen bekommt die Fourier-Ebene einen Strich beim "F" also "F‴.) |
| F' auch F_o' oder F_r' | Brennpunkt in Fourier-Ebene des Objektraums O, beziehungsweise des Referenzraums R |
| F_11' | Bild-Brennpunkt des Zylinderobjektivs 11 |
| F_MLA | objektseitige Brennebene des Mikrolinsen-Arrays |
| F_MLA' | bildseitige Brennebene des Mikrolinsen-Arrays |
| FCL | Frequenzkamm-Laser |
| FEO | Fourier-Ebene im Objektraum 0 |
| FER | Fourier-Ebene im Referenzraum R |
| HRRER (45, 46) | Hybrid-Retro-Referenz-Endreflektor Ein Hybrid-Retro-Referenz-Endreflektor erzeugt hier per Definition immer eine zur Objekt-Wellenfront gekippte Referenz-Wellenfront. Dieser Querversatz wird mittels Dachkant-Reflektor in der Brennebene des Objektivs (4, 42) erzeugt. |
| h_spot' | Höhe/Länge des Bildes des leuchtenden Linien-Spots im Objektraum |
| KKI | Kurzkohärenz-Interferogramm |
| Lambda | Wellenlänge der detektierten elektromagnetischen Strahlung |
| Lambda_S | Schwerpunkt-Wellenlänge der detektierten elektromagnetischen Strahlung |
| Ib | lateraler Bereich im Schärfentiefebereich T des Sensors bei ideal ebener Oberfläche |
| LKI | Lang-Kohärenz-Interferogramm, entsteht, wenn Lichtquelle eine große Kohärenzlänge besitzt |
| I_lat | lateraler Bereich, in dem sich eine ebene Objektoberfläche eines gekippten Sensors im wellenoptischen Schärfentiefebereich T befindet |
| L_opt_P | optische Wegdifferenz im Prisma bei einfachem Lichtdurchgang |
| L_opt_T | optische Wegdifferenz in Teilerplatte bei einfachem Lichtdurchgang |
| LS | feiner Lichtspalt |
| LS_r' | Bild des feinen Lichtspalts im Referenzarm |
| LS_o' | Bild des feinen Lichtspalts im Objektarm |
| MEO | Messebene im Objektraum |
| n | ganze Zahl |
| n* | Brechzahl eines optischen Mediums |
| 2 q1_Airy | Voller Durchmesser bis zu den beiden ersten Nullstellen im Airy-Pattern, hier auch als voller Airy-Durchmesser bezeichnet, im Objektraum |
| OPD | Optical path difference |
| OPD-FCL_c | Optical path difference compensated by FCL, region of high spatial coherence |
| RL | Linie des Hybrid-Retro- Referenz-Endreflektors. Diese definiert die Ebene E_R, wo modellhaft die Reflexion mindestens einer Referenzwelle lokalisiert ist RL ist eine parallele Linie zu einer Zylinderachse CA bei einem Cat'eye-Reflektor, wo die Fokussierung von Zylinderwellen, generiert aus ebener Eingangswelle, lokalisiert ist. RL fällt bei einem 90°-Dachkantspiegel-Endreflektor mit der Dachkantlinie DKL zusammen. |
| RS_hin | hinlaufender Referenzstrahl |
| OWF | Objekt-Wellenfront |
| RWF | Referenz-Wellenfront |
| RS_rück | rücklaufender Referenzstrahl |
| SH | Signalhub zwischen Maximalwert in der nullten Streifenordnung und dem kleineren der beiden Maxima im Streifen erster Ordnung, die adaptive Triggerschwelle aTS |
| v | optischer Bildversatz durch Dicke d des optischen Mediums (Glas, Kunststoff, Flüssigkeit) und Brechungsindex n desselben in Bezug auf Luftweg v = (n-1)/n* |
| VRRERA | Voll-Retro-Referenz-Endreflektor-Array Ein Voll-Retro-Referenz-Endreflektor-Array mit Tripel-Reflektoren erzeugt immer eine gekippte Referenz-Wellenfront, d.h., es muss immer mindestens ein Objektiv (42) dazugehören. |
| Y | Optische Verzögerungslänge im FCL |
| y | Richtung der Längsausdehnung der (ersten) Linienquelle |
| y_tilt | Kippachse des Hybrid-Retro-Referenz-Endreflektors mit spiegelsymmetrischer Wellenfront-Inversion für die Verkippung delta_beta_y desselben |
| ZBE | Zwischenbildebene |
| z_Tsep | Tiefenseparierung der kohärenten Foki von Objektarm O und Referenzarm R |

### Beschreibung der Figuren

Die Erfindung wird beispielhaft anhand der Figuren 1 bis 76 beschrieben.

Hier wird der Begriff Licht stets als Synonym für elektromagnetische Strahlung vom Terahertz-, über das Infrarot- bis zum EUV-Spektrum verwendet.

Die Figur 1 stellt einen linienhaft messenden OCT-Sensor auf der Basis eines One-shot-Interferometers auf der Basis eines modifizierten Interferometers vom Linnik-Typs mit einem fokussierenden Objektiv 6 im Objektarm dar, der auch zur Messung von Haut am Menschen eingesetzt werden soll. Figur 1 stellt einen Ansatz dar, den gesamten Messkopf zu bewegen.

Die gepulste Lichtquelle 1 wird von einer Superlumineszenz-Dioden-Batterie mit nachgeordnetem und mit schnell rotierendem Schlitz-Chopper-Rad mit einer hochgenauen Drehzahl-Regelung und Drehwinkel-Regelung durch einen Digitalrechner gebildet. Die Schlitzbreite am Rad beträgt relativ näherungsweise 40% der mittleren Schlitzabstände. So ist eine gepulste Linien-Lichtquelle gegeben, die einen nachgeordneten feststehenden Spalt beleuchtet, dessen Längsachse hier in y-Richtung weist. Die Breite des Spalts beträgt 5 µm und die ausgeleuchtete Länge beträgt 5 mm.

Die wichtigsten Parameter der gepulste Linienquelle 1 sind:
Linienquelle im NIR mit 800 nm bis 900 nm Mittenwellenlänge, mit einer Pulsrate von 400 Hz und einer mittleren Pulszeit von 100 µs (40% relative Einschaltzeit, Trapez-Form, Licht kommt von Chopper), Kohärenzlänge 6 µm, Dauerstrichleistung einer Superlumineszenz-Dioden-Batterie: 20 mW.

Der Sensorkopf bewegt sich mit 1 mm/s in Tiefenrichtung über einen Tiefenbereich von 500 µm in Lichtrichtung (z-Richtung).

Das von der gepulsten kurzkohärenten-Lichtquelle 1 ausgehende Licht wird von einem beugungsbegrenzt arbeitenden Kollimator-Objektiv 2 mit dem halben Aperturwinkel alpha_y_source, der zu einer numerischen Apertur von 0,1 führt, erfasst und kollimiert. Das kollimierte Licht gelangt auf einen Strahlteiler 3 im Interferometer. Die Breite b_spot liegt unterhalb der beugungsbgrenzten Lateral-auflösung des Kollimator-Objektivs 2. Das Interferometer arbeitet zumindest näherungsweise am optischen Gangunterschied null. Das reflektierte Licht gelangt im Referenzarm R auf ein beugungsbegrenztes Schwarzschildobjektiv 4 mit einem Dachkantreflektor 561 mit dessen Dachkante in der Fokusebene. Der Dachkantreflektor 561 weist einen Querversatz delta_q auf. Das eingestrahlte Licht wird querversetzt und erfährt eine spiegelsymmetrische Wellenfrontinversion und trifft nach Passieren des Schwarzschildobjektivs 4 geneigt wieder auf den Strahlteiler 3, wo es transmittiert und in den Detektionsstrahlengang gelangt. Das hindurchgelassene Licht trifft im Objektarm 0 auf ein beugungsbegrenztes Schwarzschildobjektiv 6 mit einer numerischen Apertur von 0,25.

Das vom Strahlteiler 3 kommende Licht wird mittels Schwarzschildobjektiv 6 auf die menschliche Haut 71 in Linienform beugungsbegrenzt fokussiert und gestreut reflektiert. Der Tiefenbereich des mechanisch bewegten Sensorkopfs beträgt hier 500 µm, so dass auch fokussiertes Licht in die menschliche Haut 71 etwas eindringen kann, wo es gestreut wird und sich auch in Richtung des Schwarzschildobjektivs 6 ausbreitet. Dieses Schwarzschildobjektiv 6 erfasst das von der menschlichen Haut 71 zurückkommende Licht und lenkt es in Richtung Strahlteiler 3, wo es in den Detektionsstrahlengang reflektiert wird. Im Detektionsstrahlengang befindet sich ein astigmatisches Abbildungssystem 8 mit einer Spaltblende 9 zur konfokalen Diskriminierung des Objektlichts, mit zwei rotationssymmetrischen Objektiven 10 und 11 sowie mit einer Zylinderoptik 12 mit liegender Zylinderachse zur Bildung von Zylinderwellen auf dem Kamera-Chip 13 der Digital-Kamera. Diese Digital-Kamera wird mit 400 Hz betrieben. Deren zugeordneter Digital-Rechner gibt den zeitlichen Takt vor und erzwingt den streng synchronisierten Lauf des schnell rotierenden Schlitz-Chopper-Rad mit hochgenauer Drehzahl-Regelung und Drehwinkel- Regelung.

Auf der Digital-Kamera kommt von jedem fokussierten Messpunkt in oder auf der menschlichen Haut 71 eine fokussierte Zylinderwelle auf die Digital-Kamera, die mit einer fokussierten und zur Objekt-Zylinderwelle geneigten Referenz-Zylinderwelle aus dem Referenzarm zur Interferenz kommt. So entsteht ein räumliches, linienhaftes Interferogramm KKI entlang einer Zeile des Kamera-Chip 13 für jeden einzelnen Messpunkt, Das linienhafte Interferogramm KKI wird mit 256 Pixeln pro erfassten Messpunkt detektiert. Der Kamera-Chip 13 besitzt eine Frame-Rate von 400 Hz. In 1024 Spalten werden also 1024 Messpunkte in Linie im Messraum, wo sich die menschliche Haut 71 befindet, erfasst. Zu jedem Messpunkte gehört ein Interferogramm KKI, so dass maximal 1024 Interferogramme KKI in einem einzigen Kamera-Frame als One-shot-Interferogramme detektiert werden können.

Pro 5 µm Tiefenwert der Fokussierung wird im Mittel ein Kamerabild aufgenommen, wobei die Verschmierung durch die Pulsbeleuchtung mit 100 µs Dauer auf weniger als 0,1 µm in der Tiefe kommt. Der optische Gangunterschied zwischen den interferierenden Wellen ändert sich durch den Kurzpusls mit 100 µs mittlerer Pulszeit in Trapez-Dreieck-Form um etwa 0,2 µm, was bei der den Wellenlängen im NIR zu einer effektiven Phasenänderung im Interferogramm von weniger als Pi/2 führt. Das ist bekanntermaßen bezüglich des Kontrasts im zeitgemittelten Interferogramm noch gut zu akzeptieren.

Mit diesem System lässt sich ein Tiefen-Scan (A-Scan) mit 460 µm erfasster Messtiefe und 1024 lateral erfassten Messpunkten pro 100 ms durchführen. Dabei wird von einer lateralen Ortsauflösung von 2 µm und in der Tiefe einer Auflösung von 5 µm ausgegangen.

Die Auswertung der räumlichen Interferogramme, deren Ortsfrequenz zumindest näherungsweise bekannt ist, erfolgt mittels in der Weißlicht-Interferometrie bekannter Verfahren, welche den 3D-OCT-Datensatz der detektierten Haut liefern.

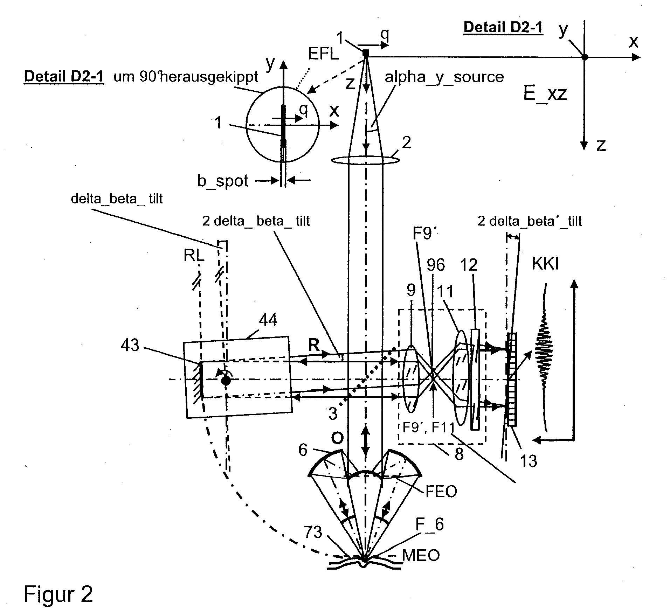

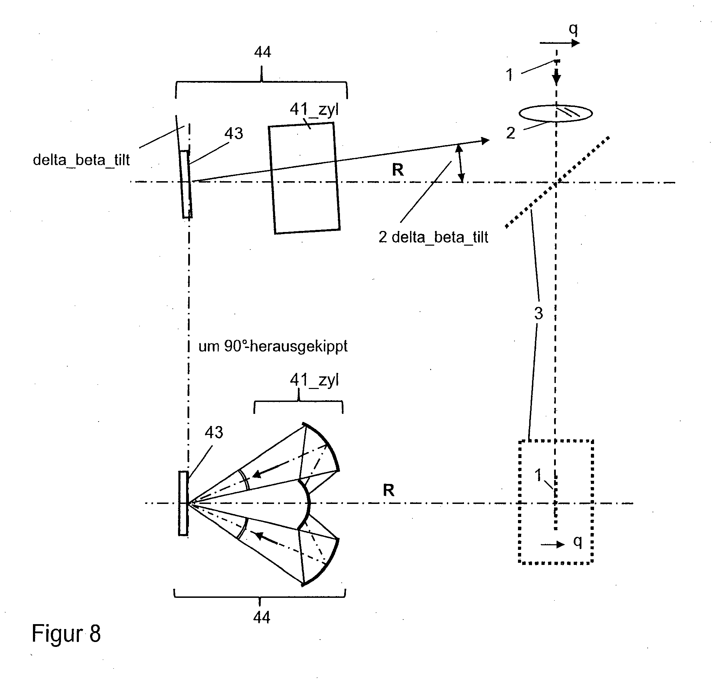

Fig. 2 stellt einen linienhaft messenden OCT-Sensor auf der Basis eines One-shot-Interferometers auf der Basis eines modifizierten Interferometers vom Linnik-Typs mit einem fokussierenden Schwarz-Schild-Objektivs 6 im Objektarm dar, der auch zur Messung von Haut am Menschen eingesetzt werden soll. Im Referenzarm ist ein Zylinder-Spiegel-Hybrid-Retro-Referenz-Endreflektor 44 eingesetzt, der wie in Figur 8 dargestellt mit einem Zylinder-Spiegel-Objektiv 41. Dabei ist dessen Zylinder-Objektiv-Achse (CA) senkrecht zur Längsrichtung lo der Linienquelle 1. Die Kippachse des Zylinder-Spiegel-Hybrid-Retro-Referenz-Endreflektors 44 ist zumindest näherungsweise parallel zur y-Achse. Das rotationssymmetrische Tubusobjektiv 9 bildet zumindest näherungsweise die Fourier-Ebene FEO des Schwarz-Schild-Objektivs 6 in die Ebene des gerasterten Detektors 13 ab. Mittels astigmatischer Abbildungsstufe 8 mit dem Zylinder-Objektiv 12 entstehen längliche und voneinander lateral separierte Kurzkohärenz-Interferogramme KKIs in der Ebene des gerasterten Detektors 13. Das Detail A stellt die um 90° herausgekippte Lichtquellenebene mit der Breite b der feinen Linienlichtquelle 1 dar.

Zu Figur 3: Hier wird ein Lichtquellen-bezogenes Koordinaten-System dargestellt, in dem die Längsrichtung der Lichtquelle, hier ein feiner Lichtspalt 196, die y-Achse darstellt und die Richtung der Minimalausdehnung der Lichtquelle die x-Achse darstellt. Die azimutale Lage des feinen Lichtspalts 196 kann beliebig sein. Die Endreflektor-Komponenten im Referenzarm sind darauf auszurichten. In x- beziehungsweise Querrichtung q liegt die Ausdehnung des feinen Lichtspalts 196 unterhalb der lateralen Auflösung, die durch die Beugungsbegrenzung der numerischen Apertur NA des nachfolgenden Kollimator-Objektivs 2 und des verwendeten Lichtspektrums gegeben ist.

Die Figur 4 stellt einen feinen Lichtspalt 196 dar, der lateral versetzt angeordnet ist. Endreflektor-Komponenten im Referenzarm sind darauf auszurichten. Zumindest in Querrichtung q liegt die Ausdehnung des feinen Lichtspalts 196 an oder unterhalb der lateralen Auflösung, die durch die Beugungsbegrenzung der numerischen Apertur NA des nachfolgenden Kollimator-Objektivs 2 und des verwendeten Lichtspektrums gegeben ist.

Die Figur 5 stellt eine feine Punktlichtquelle in Kettenform 195 dar. Zumindest in Querrichtung q liegt die Ausdehnung der feinen Punkte der Punktlichtquelle in Kettenform 195 an oder unterhalb der lateralen Auflösung, die durch die Beugungsbegrenzung aufgrund der numerischen Apertur NA des nachfolgenden Kollimator-Objektivs 2 und des verwendeten Lichtspektrums gegeben ist.

Die Figur 6 stellt eine fein ausgebildete Punktlichtquellen-Matrix dar. Auch ist zumindest in Querrichtung q die laterale Ausdehnung der einzelnen Lichtspots jeweils an oder unterhalb der lateralen Auflösung, die durch die Beugungsbegrenzung aufgrund der numerischen Apertur NA des nachfolgenden Kollimator-Objektivs 2 und des verwendeten Lichtspektrums gegeben ist.

Die Figur 7 stellt eine feine Linien-Lichtquelle dar, die mittels rechnergesteuerten räumlichen Lichtmodulators in Transmission auf Flüssigkristall-Basis und vorgeordneter kurzkohärenter Lichtquelle, die hier nicht dargestellt ist, gebildet ist. Die adressierten linienhaften Transmissionsbereiche sind rechnergesteuert verschiebbar, um bei der Messung eine Messfeld nahezu vollflächig erfassen zu können. In Querrichtung q ist die laterale Ausdehnung der einzelnen Lichtspots jeweils an oder unterhalb der lateralen Auflösung, die durch die Beugungsbegrenzung aufgrund der numerischen Apertur NA des nachfolgenden Kollimator-Objektivs 2 und des verwendeten Lichtspektrums gegeben ist.

Die Figur 8 stellt einen entfalteten Referenzarm R dar, in welchem sich ein Zylinder-Spiegel-Hybrid-Retro-Referenz-Endreflektor (ZSHRRER) 44 mit Zylinder-Spiegel-Objektiv 41 befindet. Die Zylinder-Objektiv-Achse (CA) des Zylinder-Spiegel-Objektivs 41 ist senkrecht zur Längsrichtung lo der Linienquelle 1 ausgerichtet. Der gesamte Zylinder-Spiegel-Hybrid-Retro-Referenz-Endreflektor 44 ist schwach verkippt um den Winkel delta-beta_tilt, so dass für Referenzbündel der Kippwinkel 2 delta-beta_tilt besteht.

Die Figuren 9 bis 12 beschreiben mögliche Hybrid-Retro-Referenz-Endreflektor-Anordnungen. Figur 9 stellt einen gekippten Dachkantreflektor 5 dar. Figur 10 stellt einen Dachkantreflektor 5 mit der Dachkante DK im Referenzarm dar.

Fig. 11 stellt den Referenzstrahlengang im Referenzarm R in einem modifizierten Linnik-Interferometer mit einem rotationssymmetrischen Referenzobjektiv 42 und einem Hohl-90°-Dachkant-Endreflektor 561 dar. Die feine Linienlichtquelle 1 weist eine Breite b auf. Der Dachkantspiegel-Reflektor 561 weist nicht zwingend eine enge Toleranz des 90°-Dachkantwinkels auf. Das Interferometer weist zur Beleuchtung einen feinen Lichtspalt auf, der hier nicht dargestellt ist. Es entsteht für das zurücklaufende Licht RS_r aus dem Lateralversatz ein Winkel 2 delta_beta_strich.

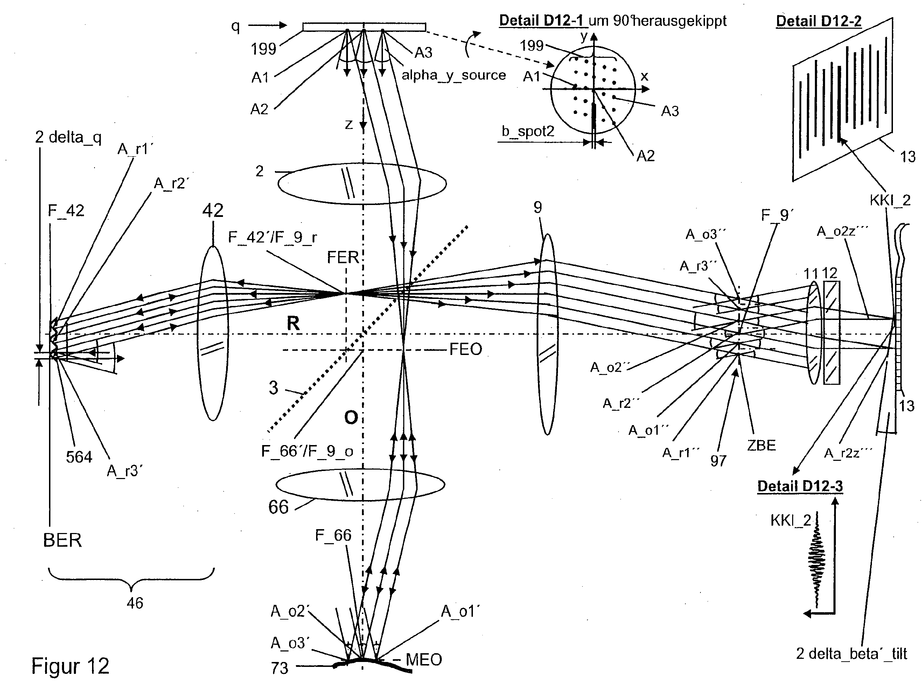

Die Figur 12 stellt vom Grundtyp ein Linnik-Interferometer mit einer fein ausgebildeten Punkt-Lichtquellen-Matrix 199 dar, die spektral breitbandig ausgebildet ist und eine Ausdehnung der Lichtspots unterhalb der beugungsbegrenzten Lateral-Auflösung des nachfolgenden Kollimator-Objektivs 2 aufweist. In der Messebene MEO des Interferometers befindet sich eine raue Metalloberfläche 73 als Objekt. Im Referenzarm ist ein Hybrid-Retro-Referenz-Endreflektor-System 46 mit einem rotationssymmetrischen Referenzobjektiv 42 angeordnet, dem ein 90°-Hohl-Dachkantspiegel-Mikro-Endreflektor-Array 564 zugeordnet ist. Die 90°-Dachkantspiegel-Reflektoren haben hierbei keine zwingend enge 90°-Winkel-Toleranz. Das 90°-Hohl-Dachkantspiegel-Mikro-Endreflektor-Array 564 ist lateral etwas versetzt, so dass auch die kleinen 90°-Hohl-Dachkantspiegel etwas versetzt sind und die Bildpunkte A_r1', A_r2' und A_r3' als leuchtende Spots A1, A2 und A3 von der Punkt-Lichtquellen-Matrix 199 nicht in die Dachkanten abgebildet werden, wohl zumindest näherungsweise jedoch scharf in die Dachkantebene. So entsteht vielfach ein Querversatz 2 delta_q.

Eine astigmatische Abbildung von Punkten der rauen Metalloberfläche 73, die von Lichtspots der fein ausgebildeten Punkt-Lichtquellen-Matrix 199 beleuchtet werden, auf den gerasterten Detektor 13 wird durch ein Zylinder-Objektiv 12 in Kombination mit einem rotationssymmetrischen Objektiv 11 erreicht.

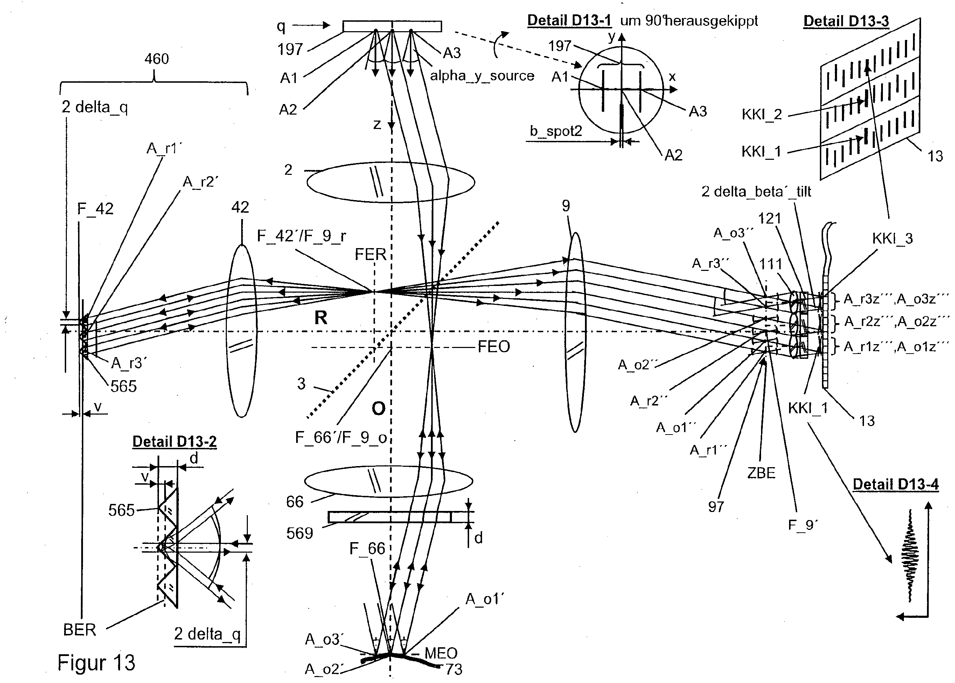

Die Figur 13 stellt eine Anordnung im Grundtyp nach Figur 12 dar. Hier ist im Referenzarm ein Hybrid-Retro-Referenz-Endreflektor-System 460 mit einem rotationssymmetrischen Referenzobjektiv 42 angeordnet, dem im Gegensatz zu Figur 12 ein 90°-Dachkantprismenspiegel-Mikro-Endreflektor-Array 565 zugeordnet ist. Im Objektarm befindet sich zur Kompensation der optischen Dicke der 90°-Prismen im 90°-Dachkantprismenspiegel-Mikro-Endreflektor-Array 565 zwecks Vermeidung von starker unkompensierter Dispersion im Interferometer eine Kompensationsplatte 569 gleicher optischer Dicke wie die 90°-Prismen im Mikro-Endreflektor-Array 565. So wird das unerwünschte und in diesem Fall auch viel zu starke Chirping im Kurzkohärenz-Interferogramm KKI verhindert. Eine astigmatische Abbildung von Punkten der rauen Metalloberfläche 73, die von Lichtspots der fein ausgebildeten Punkt-Lichtquellen-Matrix 199 be leuchtet werden, auf den gerasterten Detektor 13 wird durch zylindrische Mikro-Linsen 121 in einem Zylinder-Mikro-Linsen-Array erreicht, die mit rotationssymmetrischen Mikro-Linsen 111 in einem Mikro-Linsen-Array kombiniert sind. Das Detail D13-1 zeigt die Beleuchtung mit drei Lichtspalten. Das Detail D13-2 stellt das 90°-Dachkantprismenspiegel-Mikro-Endreflektor-Arrays 565 dar. Das Detail D13-3 zeigt die Anordnung von Kurzkohärenz-Interferogramm KKI auf dem gerasterten Detektor 13. Das Detail D13-4 zeigt den Signalverlauf in einem detektierten Kurzkohärenz-Interferogramm KKI.

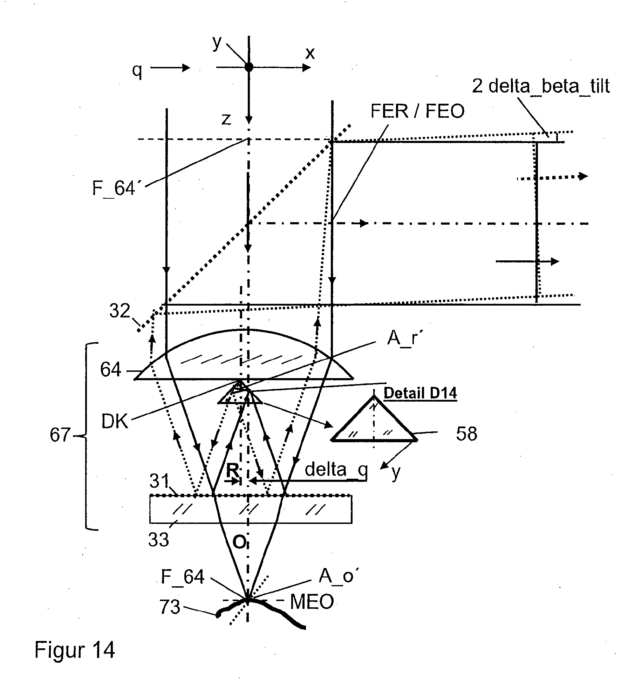

Die Figur 14 stellt eine Anordnung mit einem Mirau-Interferometer 67 dar. Die Beleuchtung erfolgt über einen, hier nicht dargestellten feinen Lichtspalt. Im Referenzarm R des Mirau-Interferometers ist ein feststehendes 90°-Dachkantprisma 58 angeordnet, dessen Dachkante DK sich optisch in der Fokusebene des Objektivs 64 befindet. Die Dachkante DK des 90°-Dachkantprismas 58 ist parallel zur y-Achse ausgerichtet und parallel zur Längsausdehnung des feinen Lichtspalts zur Interferometerbeleuchtung, die hier nicht dargestellt ist. Die 90°-Winkel-Toleranz des 90°-Dachkantprismas ist hier eher groß. Bei dieser Mirau-Interferometer-Anordnung wird eine Lateral-Shear 2 delta_q zwischen den Wellenfronten eingeführt, die nach dem Passieren des Objektivs 64 zu einer Verkippung der Wellenfronten von Objekt- und Referenzarm von 2 delta_beta_tilt führt. Die optische Weglänge des 90°-Dachkantprismas 58 wird durch die optische Weglänge des Strahlteilers 33 ausgeglichen. Das Detail D14 stellt das Dachkantprisma 58 dar, dessen optische Weglänge der Platte 33 im Strahlteiler 31 entspricht.

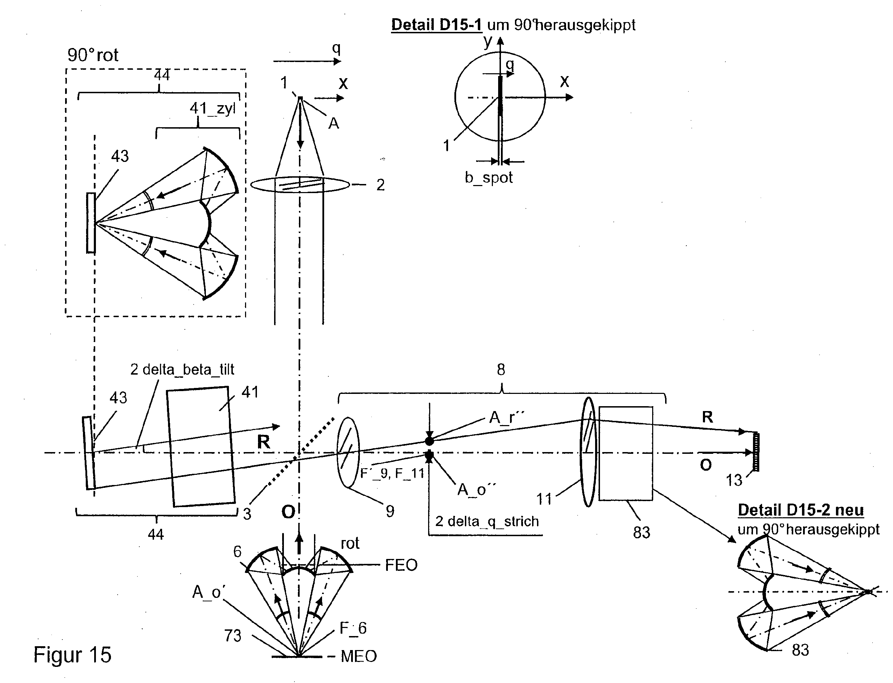

Die Figur 15 stellt eine Interferometeranordnung mit einer schmalen Linienquelle 1, dargestellt im Detail D15 und mit einem Schwarzschild-Zylinder-Spiegel-Objektiv 41 dar. Die Breite b_spot der schmalen Linienquelle 1 liegt unter der beugungsbegrenzten Auflösung des Lichtquellenobjektivs 2, s. Detail D15-1. Das Detail D15-2 zeigt ein Schwarzschild-Zylinder-Spiegel-Objektiv 83 im Detektionsstrahlengang, um Zylinderwellen auf dem gerasterten Detektor 13 zu erzeugen.

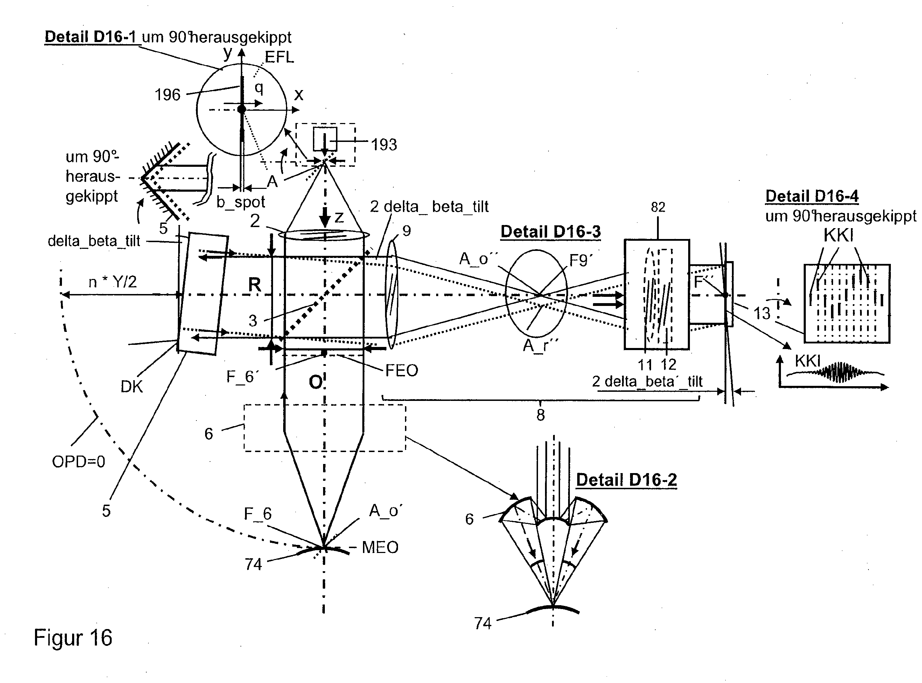

Die Figur 16 stellt eine Anordnung mit einem Hybrid-Interferometer nach dem Linnik-Ansatz dar oder kann auch als Zweistrahl-Anordnung auf der Basis eines Michelson-Interferometers mit einem objektabbildenden Objektiv im Objektarm verstanden werden. Dieses Zweistrahl-Interferometer wird mit einem Frequenzkamm-Laser betrieben. Es erfolgt eine linienhafte Beleuchtung mittels feinem Lichtspalt 196, dargestellt im Detail D16-1, wobei die Linienbreite b_spot unterhalb des lateralen Auflösungsvermögens des Kollimatorobjektivs 2 liegt. Die Anordnung wird zur Messung der Restrauheit einer optisch glatten Metalloberfläche 74 eingesetzt. Das Bild des Spalts (A_o') befindet sich auf der optischen Achse des Schwarzschild-Spiegel-Objektivs 6. Detail D-16-2 zeigt das Schwarzschild-Spiegel-Objektiv 6, das im Objektarm 0 des Interferometers angeordnet ist. Durch die Spiegeloptik wird Dispersion im Interferometer vermieden. Die Lateral-Shear der Dachkante DK des Dachkantreflektors 5 in y-Richtung ist zu null gemacht. Die Dachkante DK liegt optisch zumindest näherungsweise in der Fourier-Ebene des Fokussier-Objektivs 6, wodurch Interferenzen gleicher Neigung in der Detektionsebene weitgehend vermieden werden. Das Detail D16-4 zeigt Kurzkohärenz-Interferogramme KKI auf dem gerasterten Detektor 13, jeweils eines für jeden Messpunkt auf der optisch glatten Metalloberfläche 74.

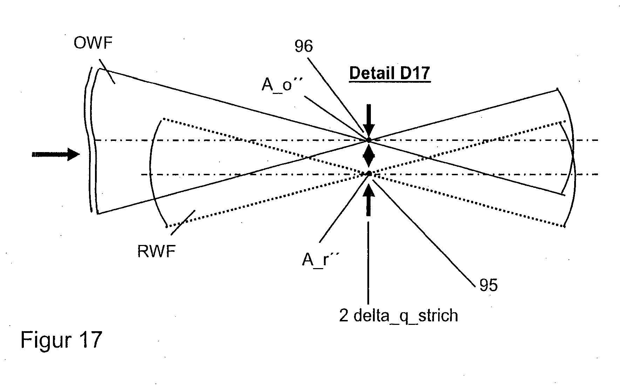

Das Detail D16-3 ist in Figur 17 vergrößert dargestellt. Hier ist eine feine Spaltblende 96 für Objektstrahlung angeordnet, die zur Tiefpassfilterung am Airy-Limit und darunter genutzt wird, damit eine Objekt-Wellenfront OWF auch von einem weniger kooperativen Messpunkt zumindest näherungsweise voll ausgebildet werden kann. Es ist jedoch möglich, dass es einen "unbestimmbaren" Phasensprung im detektierten räumlichen Interferogramm auf dem gerasterten Detektor 13 gibt, so dass die Phasen-Information eines Objektpunkts verloren gehen kann und nicht für die Tiefenbestimmung zur Verfügung steht.

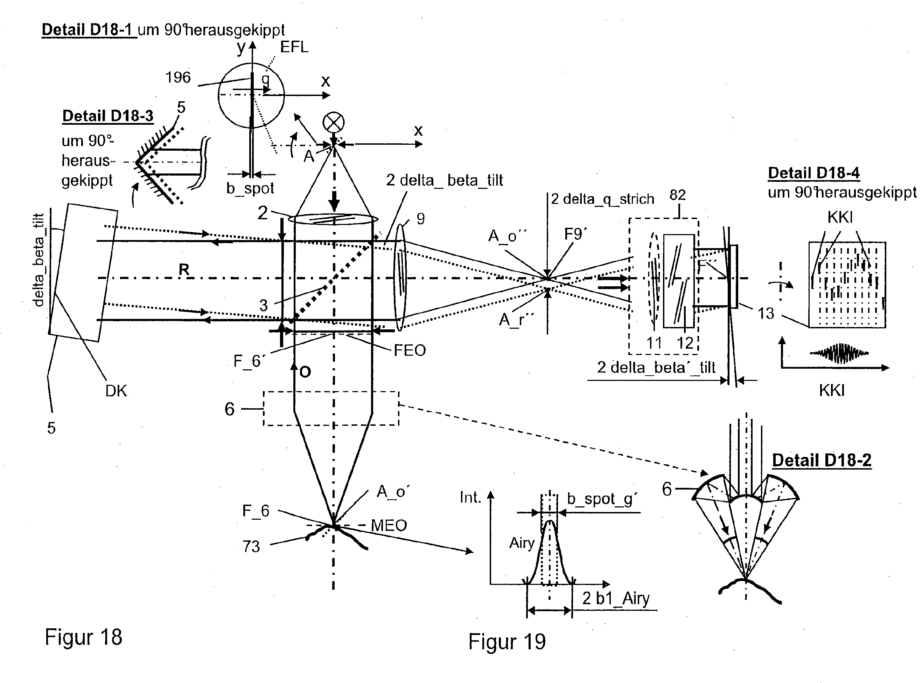

Die Figur 18 stellt ein Hybrid-Interferometer nach dem Linnik-Ansatz dar, in dessen Referenzarm R sich ein um den Kippwinkel delta_beta_tilt geneigter 90°-Hohl-Dachkantreflektor 5 mit der Dachkante DK befindet. Gemäß Detail D18-1 wird über einen feinen Lichtspalt 196 beleuchtet. Detail D18-2 stellt das im Objektarm O eingesetzte Schwarzschildobjektiv 6 im Objektarm O dar. Detail D18-3 zeigt den um 90° herausgekippten 90°-Hohl-Dachkantreflektor 5 im Referenzarm R. Nach der Strahlvereinigung am Strahlteiler des rückkommenden Lichts aus Referenzarm R und des Lichts von einer rauen Metalloberfläche 73 aus dem Objektarm O erfolgt die Fokussierung der Bündel mittels Tubusobjektiv 9. Aufgrund der Kippung des 90°-Hohl-Dachkantreflektors 5 sind die Fokusspots A_r" und A_o" in der Brennebene F_9' lateral separiert. A_r" und A_o" stellen Bilder eines leuchtenden Punktes A dar. Mittels astigmatischem Detektorobjektiv in der Kombination rotationssysmmetrisches Objektiv 11 und Zylinderobjektiv 12 entsteht auf dem gerasterten Detektor 13 von jedem im Schärfentiefebereich des Schwarzschildobjektivs 6 erfassten Objektpunkt eine Objekt-Zylinderwelle, die jeweils um den Winkelbetrag 2 delta_beta'_tilt zur Referenz-Zylinderwelle geneigt ist, so dass dort ein Paar interferierender Zylinderwellen besteht. Detail D18-4 zeigt die entstehenden Kurzkohärenz-Interferogramme KKI.

Das Rechteck in Figur 19 zeigt die Breite b_spot_g' des errechneten geometrisch-optisches Bildes vom feinen Lichtspalt 196, dessen Bild hier etwa der halben Airy-Scheibchenbreite entspricht.

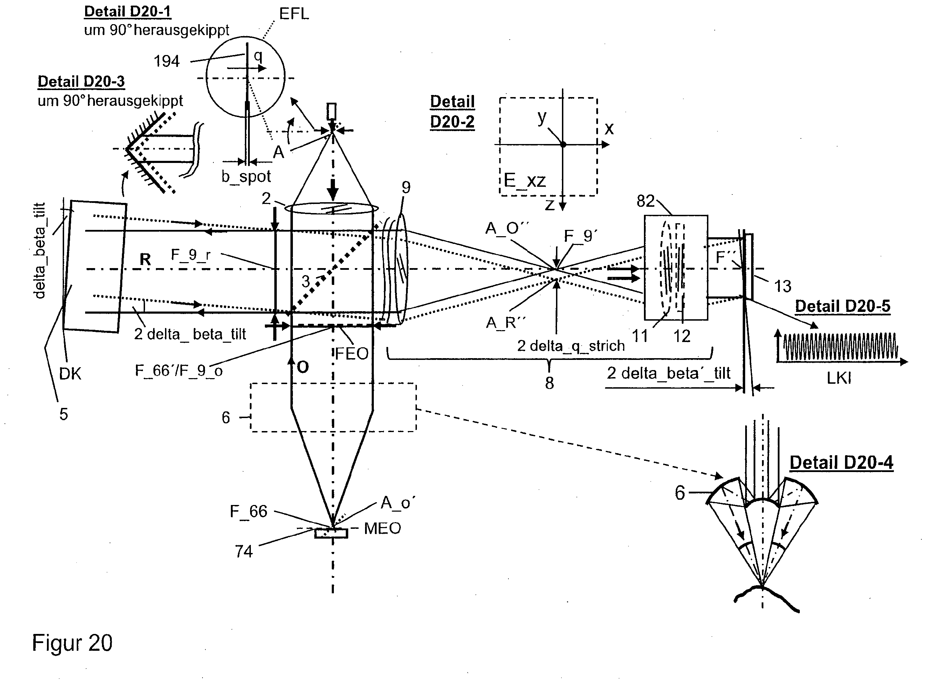

Die Figur 20 basiert aus der optischen Schaltung nach Figur 18 und stellt einen Sensor für die Messung von jeweils einer sehr kurzen Profil-Linie dar, da sich im räumlichen Interferogramm ein quadratischer Phasenterm befindet. Detail D20-2 zeigt das Koordinatensystem.

Als Lichtquelle wird ein vergleichsweise starker Single-Mode-Laser als Lichtquelle mit einem zum gerasterten Detektor 13 synchronisierten, rotierenden Chopper-Rad eingesetzt. Gemessen wird hier die Ebenheitsabweichung einer ebenen und optisch glatten Metalloberfläche 74. Somit entstehen auf dem gerasterten Detektor 13 Wavelets ohne einen Streifen nullter Ordnung. Die Höhenunterschiede benachbarter Messpunkte in einem sehr kurzen Bereich liegen deutlich unter einem Viertel der Laserwellenlänge. Demzufolge müssen zur Messung der optisch glatten Metalloberfläche 74 nur Phasendifferenzen deutlich unter 180 Altgrad (180°) ausgewertet werden, um das Profil der optisch glatten Metalloberfläche 74 darstellen zu können.

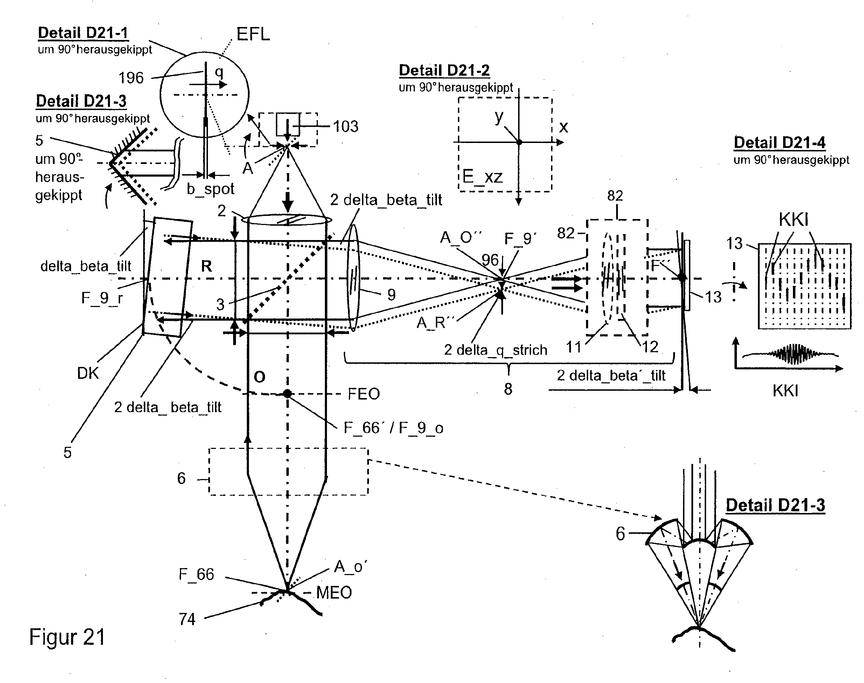

Die Figur 21 stellt eine zu Figur 20 bis auf die Referenz-Endreflektor-Position vergleichbare Anordnung dar. Jedoch wird hierbei eine feine Lichtquelle 103 mit einem Frequenzkamm-Laser eingesetzt, der mit einem rotierenden Chopper-Rad im einstelligen kHz-Bereich moduliert wird. Der Frequenzkamm-Laser kompensiert in bekannter Art und Weise den vergleichsweise großen optischen Gangunterschied des Zweistrahl-Interferometers, so dass Kurzkohärenz-Interferogramme detektiert werden können. Der Dachkantreflektor 5 steht hier zumindest näherungsweise in der zur Ebene FER konjugierten Ebene, so dass im Wavelet kein quadratischer Phasenterm auftritt und so auch längere Profilmessungen im Vergleich zur Anordnung in Figur 20 durchgeführt werden können.

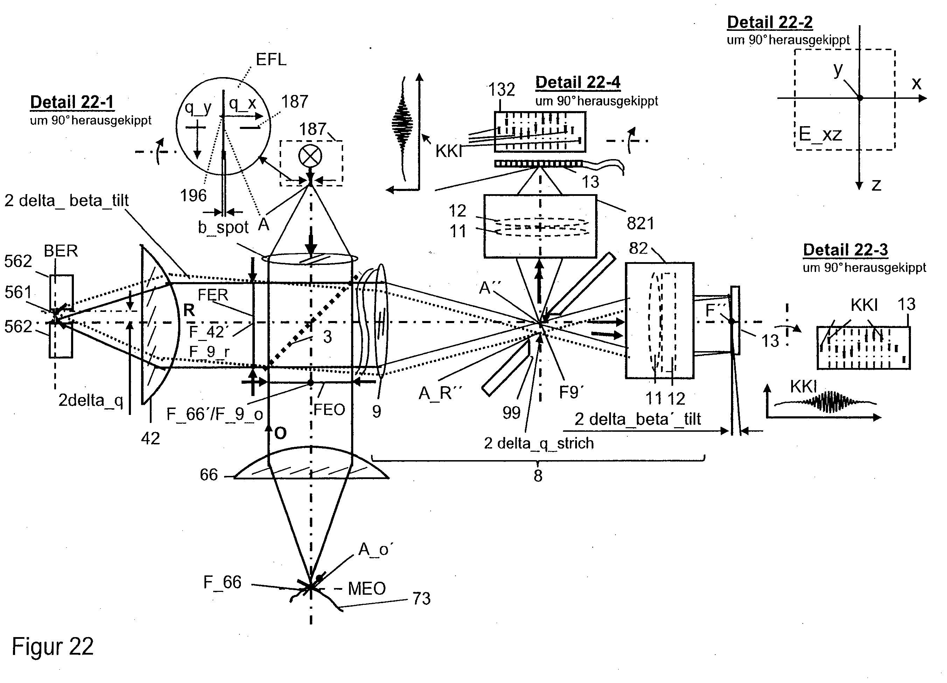

Die Figur 22 präsentiert ein Linnik-Interferometer, bei dem in zwei rechtwinklig zueinander stehenden Richtungen gleichzeitig linienhaft gemessen werden kann. Dazu sind, dargestellt im Detail D22-1, zwei feine Lichtquellen 197 als Punktlichtkette in 90°-Anordnung ausgebildet, die im niedrigen kHz-Bereich gepulstes Licht ausstrahlen. Die laterale Ausdehnung in q_x- und q_y-Richtung liegt jeweils unter der lateralen Auflösung bei der Abbildung auf die Objektoberfläche, die hier eine raue Metalloberfläche 73 darstellt. Im Referenzarm R sind entsprechend zwei um 90° zueinander angeordnete 90°-Hohl-Dachkantreflektoren 561 und 562 positioniert. So besteht eine Zweikanal-x-y-Messanordnung. Im Detektionsstrahlengang werden die beiden Kanäle mittels planem Umlenkspiegel 99 mit feiner Spaltblende strahlenoptisch getrennt und nach Passieren je eines astigmatischen Detektorobjektivs 82 und 821 jeweils einem gerasterten Detektor 13 und 132 zugeführt, wo von angemessenen Objektpunkten Kurzkohärenz-Interferogramme KKI detektiert werden.

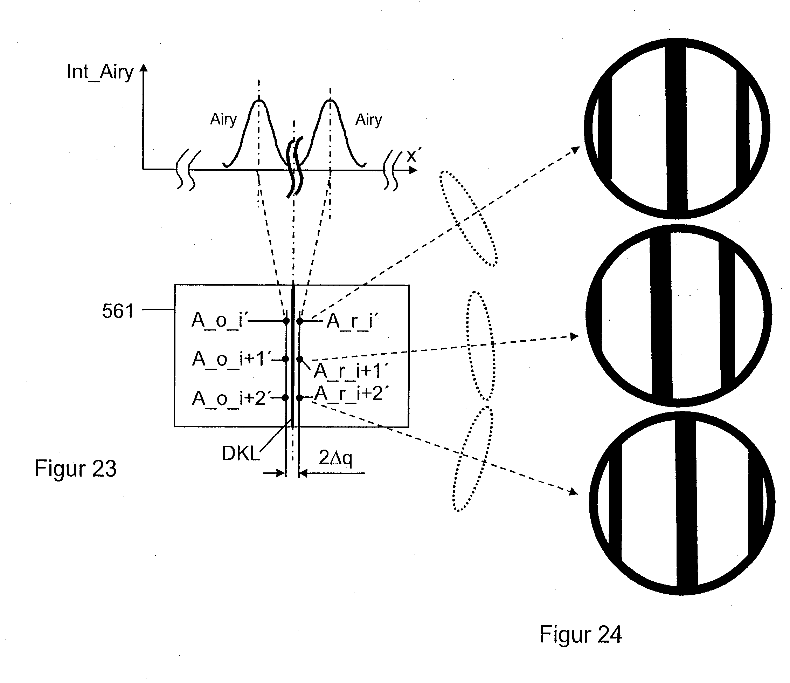

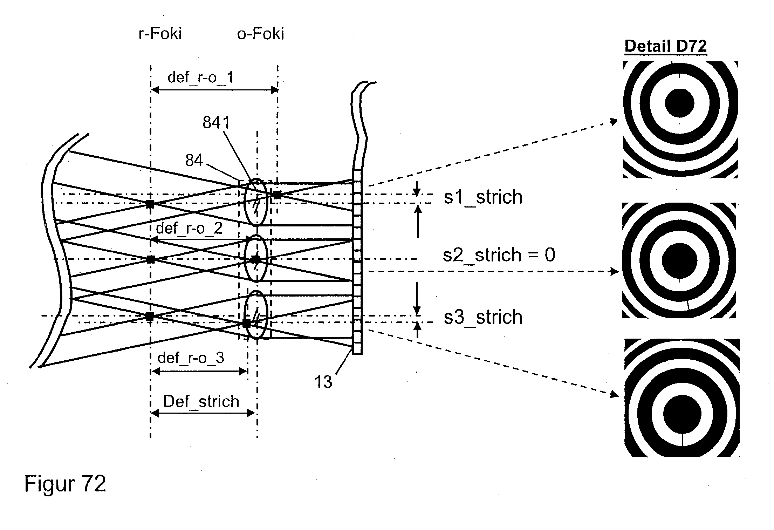

In Figur 23 sind die kohärenten Airy-Spots A_o_i ' und A_r_i' sowie A_o_i+1' und A_r_i+1', die jeweils von einer feinen Lichtquelle 190 mit feinen Lichtspots über Strahlteilung und Passieren von Referenzarm R und Objektarm O gebildet sind, vergleichsweise dicht beieinander positioniert. Die Darstellung ist auf den Hohl-90°-Dachkant-Endreflektor 561 bezogen, auf den die Objektspots rückprojiziert wurden. Der Abstand der Airy-Spots ist in der Größenordnung der Airy-Musterbreite. Figur 24 zeigt die sich hierbei ergebende räumlichen Interferogramme für drei Messpunkte, denen jeweils über hier nicht dargestellte Mikrolinsen ein eigener Strahlengang bis zur Detektion zugeordnet ist, wobei hier auf eine astigmatische Abbildung der interferierenden Bündel mit Bildung von Zylinderwellen verzichtet wurde. Einen Hinweis auf einen möglichen Strahlengang mit Mikrolinsen gibt hier die Anordnung in Figur 72, wo die Foki jedoch in der Tiefe separiert sind.

Die in Figur 24 dargestellten räumlichen Interferogramme können auch als Lichtfeld-Interferogramme bezeichnet werden, je eines für jeden einzelnen Objektpunkt. Aufgrund des geringen lateralen Abstands der kohärenten Spots A_o_i' und A_r_i' sowie A_o_i+1' und A_r_i+1 'entstehen nur sehr wenige Streifen bei der Detektion. Der mittlere Streifen stellt hier aufgrund der exakten Position des zugehörigen Objektpunkts den Streifen nullter Ordnung dar. Der Streifenabstand ist zumindest näherungsweise konstant.

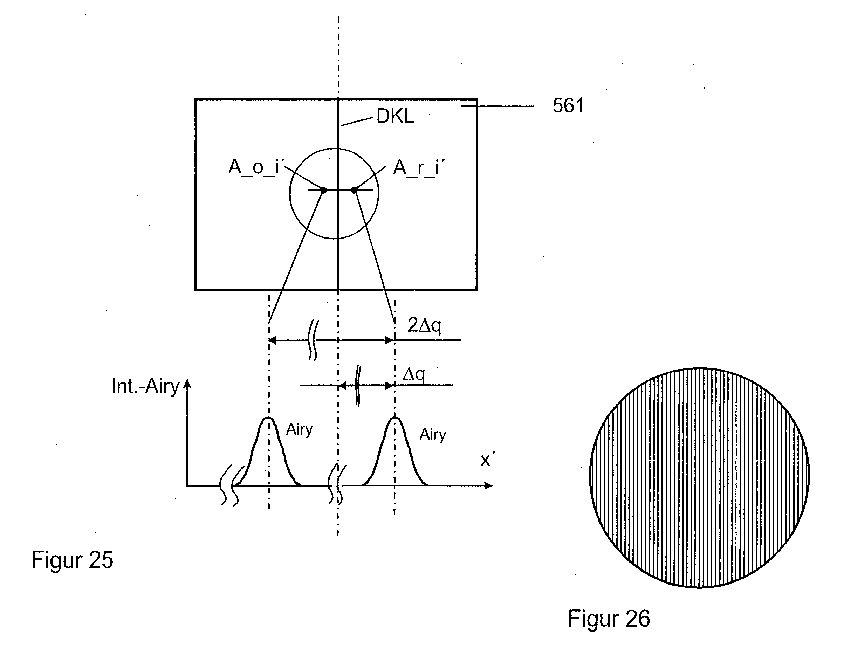

In Figur 25 sind Airy-Spots etwas weiter voneinander auf dem Hohl-90°-Dachkant-Endreflektor 561 im Referenzarm R des Interferometers beieinander positioniert mit einem Abstand in der deutlich mehr als zehnfachen Größenordnung der Airy-Musterbreite. Auch hierbei wurden die Objektspots zur Darstellung auf den Hohl-90°-Dachkant-Endreflektor 561 rückprojiziert. Figur 26 zeigt das sich hierbei ergebende räumliche Interferogramm als Überlagerung von kohärenten Referenz-Spots und Objektspots. Es entsteht aufgrund des hier im Vergleich zu Figur 23 deutlich größeren Querversatzes 2 delta_q eine Vielzahl von Interferenzstreifen bei der Detektion. Auch hier ist der Streifenabstand zumindest näherungsweise konstant und trägt eher keine Information über das Messobjekt.

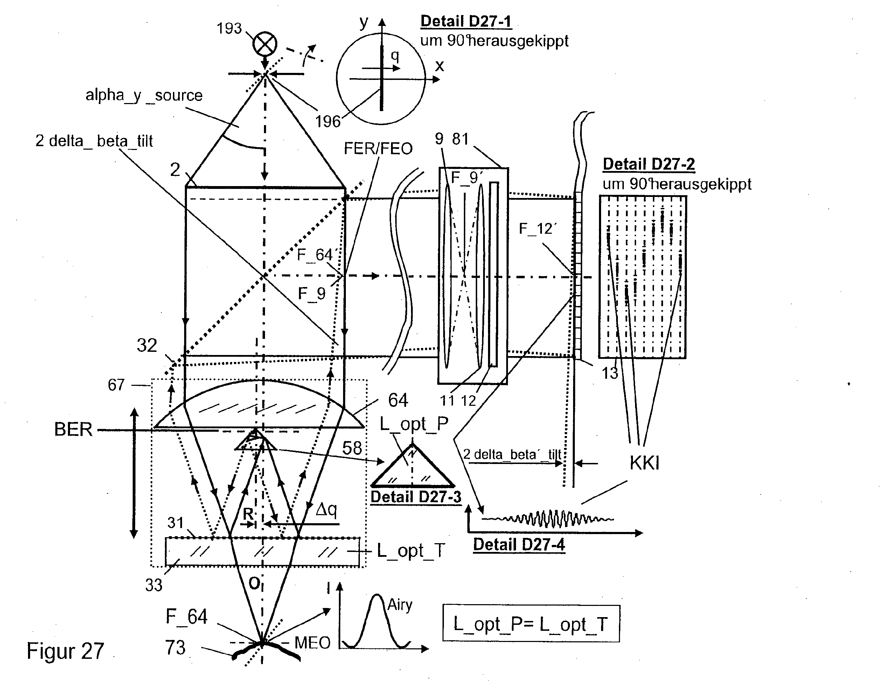

Die Figur 27 stellt einen Linien-Sensor in Mirau-Interferometer-Anordnung dar. Das Referenz-Dackantspiegelprisma 58 weist für Hauptstrahlen im gleichen Glastyp den gleichen geometrischen Weg wie die Teilerplatte 33 auf. Somit besteht eine Kompensation der optischen Glasweglängen und es tritt in den Kurzkohärenz-Interferogrammen kein Chirping auf. Zur schnellen Erfassung der Miniform einer rauen und etwas gekrümmten Metalloberfläche 73 bewegt sich das Mirau-Objektiv-Interferometer 67 mittels hier nicht dargestelltem Hubtisch in Tiefenrichtung zur Erfassung der Objektpunkte jeweils im Schärfentiefebereich, so dass dann vom feinen Lichtspalt 196 ein Bild mit Airy-Muster auf der Objektoberfläche entstehen kann. Im Detektionsstrahlengang werden schließlich durch astigmatische Abbildung Kurzkohärenz-Interferogramme auf dem gerasterten Detektor 13 gebildet.

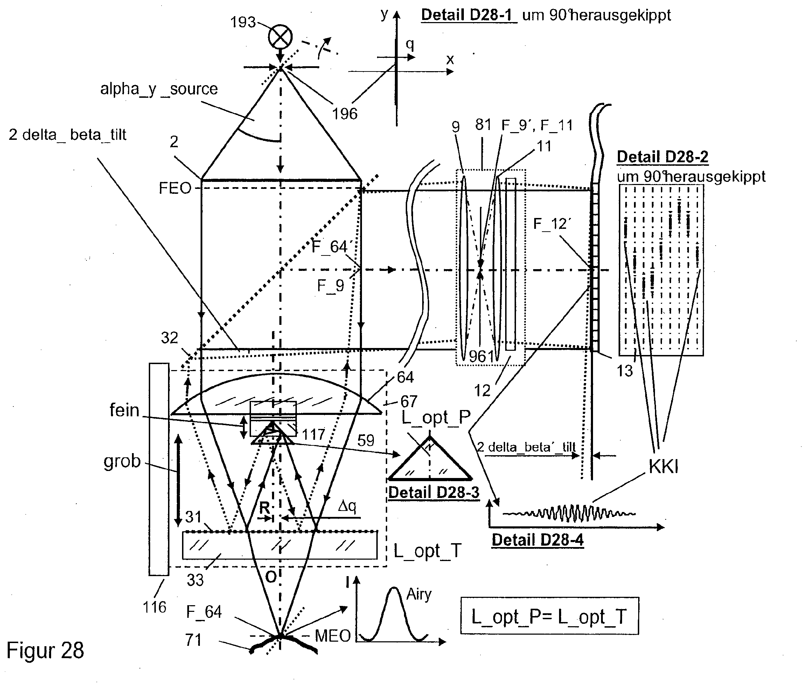

Die Figur 28 stellt einen Linien-Sensor mit schnellstmöglichem Scan des Mirau-Objektiv-Interferometers 67 für das Erfassen der Oberfläche von menschlicher Haut 71 am Patienten dar. Der optische Aufbau entspricht zunächst dem von Figur 27, jedoch ist hierbei neben dem hochdynamischen Linear-Antrieb 116 für das Mirau-Objektiv-Interferometer 67 auch noch ein hochdynamischer Linear-Antrieb für das 90°-Dachkant-Prisma 59, ein Piezo-Steller 117, angeordnet. Diese Kombination ermöglicht aufgrund der großen Beschleunigung des Piezo-Stellers 117 einen Weg-Zeit-Verlauf der optischen Abtastung mit einem vergleichsweise gut angenäherten Dreieckprofil und somit praktisch keinen Zeitverlust in den Umkehrpunkten der Abtastung. Somit ergibt sich für den Sensor ein Tiefenmessbereich für die Profilerfassung, der ein Mehrfaches des wellenoptischen Schärfetiefenbereiches beträgt. Mittels Lateral-Scan kann dadurch ein vergleichsweise großes Messvolumen schnell durchmustert werden. Auch bei dieser Anordnung weist das Referenz-Dackantspiegelprisma 59 in gleichem Glastyp den gleichen geometrischen Weg für Hauptstrahlen wie die Teilerplatte 33 auf. Die Spaltblende 961 dient auch der Unterdrückung des Hintergrundlichts aus der Tiefe der angemessenen Haut 71, um den Signalkontrast zu verbessern.

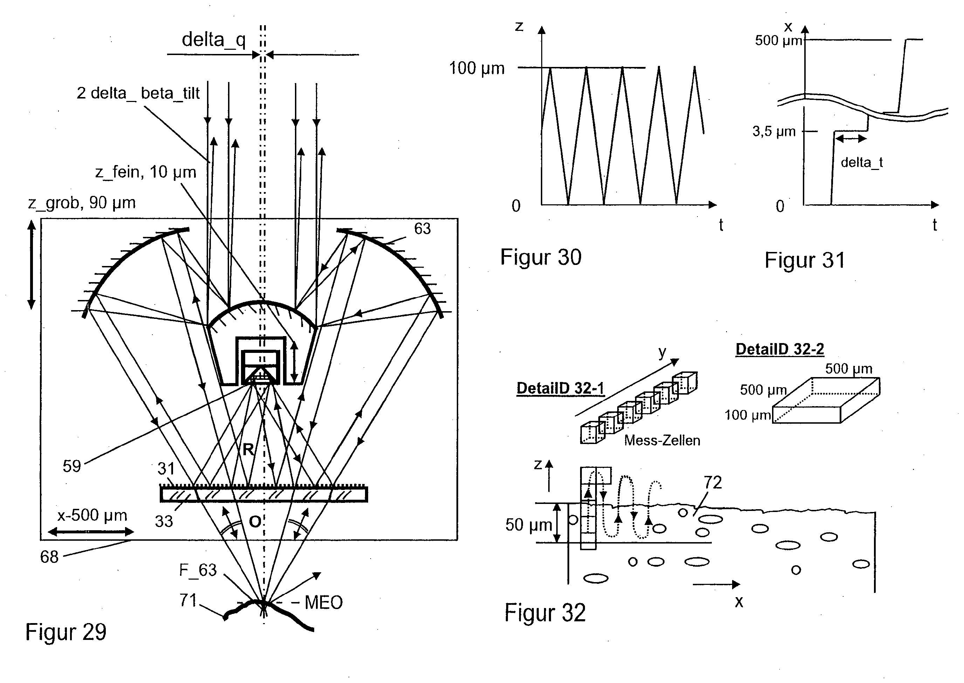

Die Figur 29 stellt ein Mirau-Interferometer 68 für die Weißlicht-Interferometrie mit einem Schwarzschild-Objektiv 63 dar. Es sind mehrere Positionier-Systeme zur schnellen Bewegung des Mirau-Interferometers 68 angeordnet. Auch das 90°-Dachkantprisma 59 bewegt sich im Referenzarm in z-Richtung hochdynamisch. So kann gemäß Figur 30 ein Dreiecks-Profil im Verlauf des Weges des Mirau-Interferometers 68 über der Zeit erreicht werden. Figur 31 zeigt näherungsweise den Wegverlauf in x-Richtung über der Zeit t. Figur 32 präsentiert den Scanweg an einem lebenden Organ. Das Detail D32-1 stellt einzelne Messzellen dar und Detail D32-2 das in einem Messvorgang erfassbare Messvolumen.

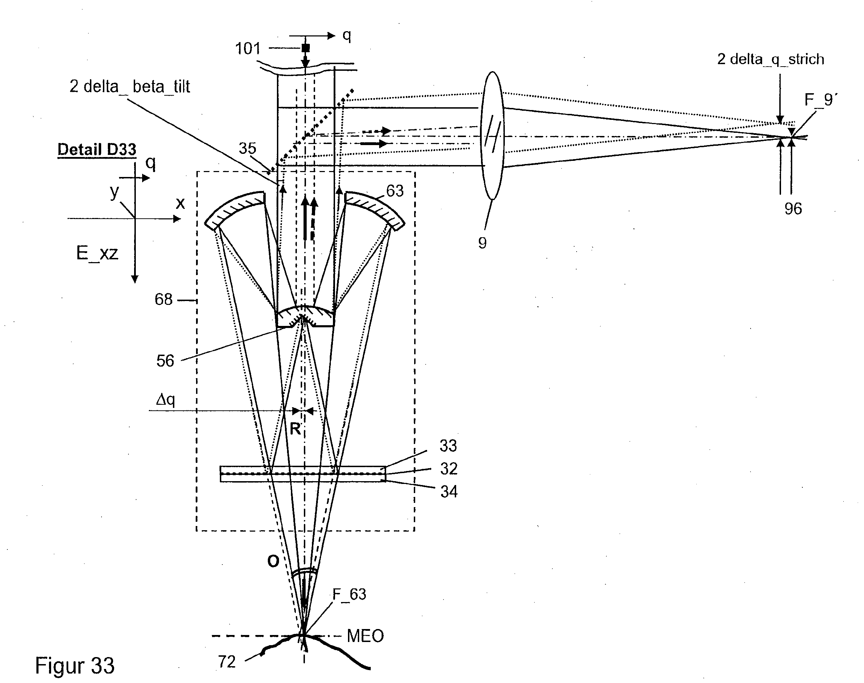

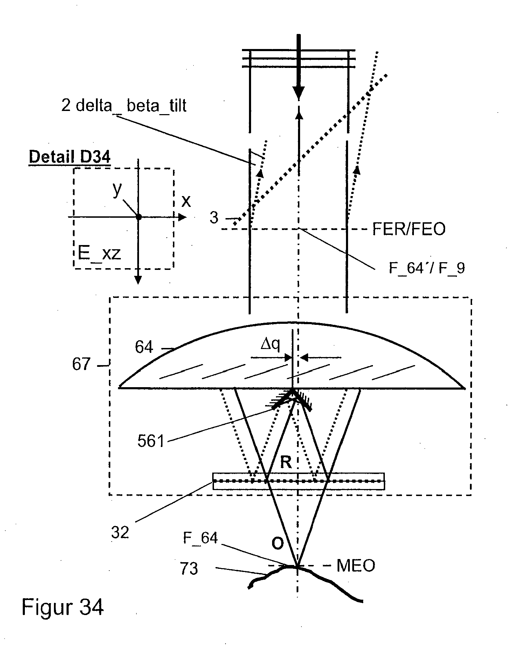

In Figur 33 ist eine weitere Mirau-Interferometer-Anordnung mit einem Schwarzschild-Messobjektiv 63 dargestellt. Das Detail D33 zeigt das Koordinatensystem. Figur 34 präsentiert eine Mirau-Interferometer-Anordnung mit einem refraktiven Messobjektiv 64.

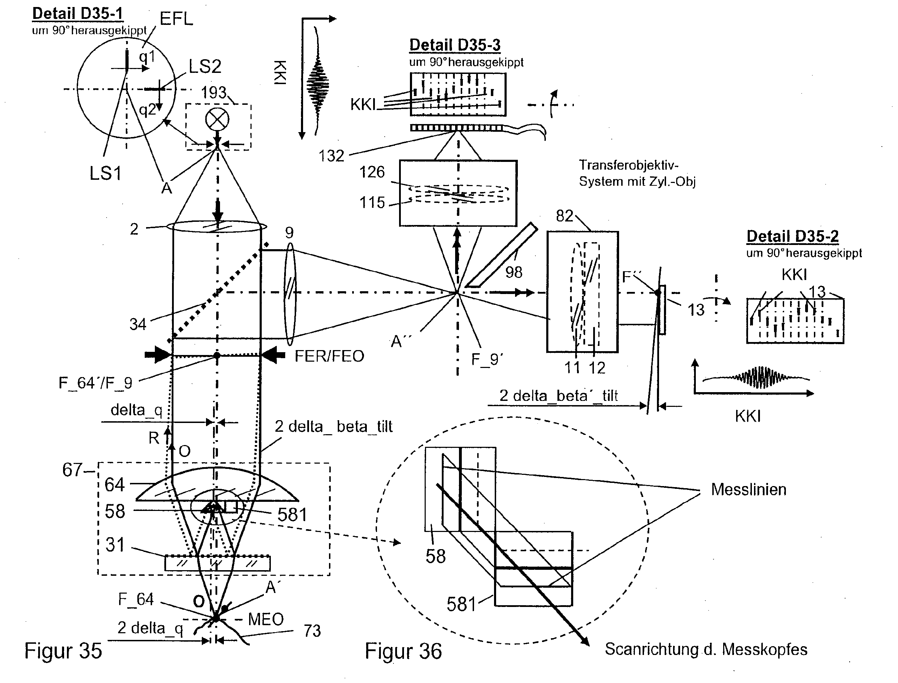

Die Figur 35 stellt ein Mirau-Interferometer-System für die Messung von zwei Linienprofilen dar, die senkrecht zueinander angeordnet sind.

Die Figur 36 zeigt die Scanrichtung des Messkopfes, der sich auf einer Dreikoordinatenmessmaschine befindet, und die rechtwinklig zueinander angeordneten feinen Messlinien, die aus der feinen, gepulsten Linien-Lichtquelle 193 stammen.

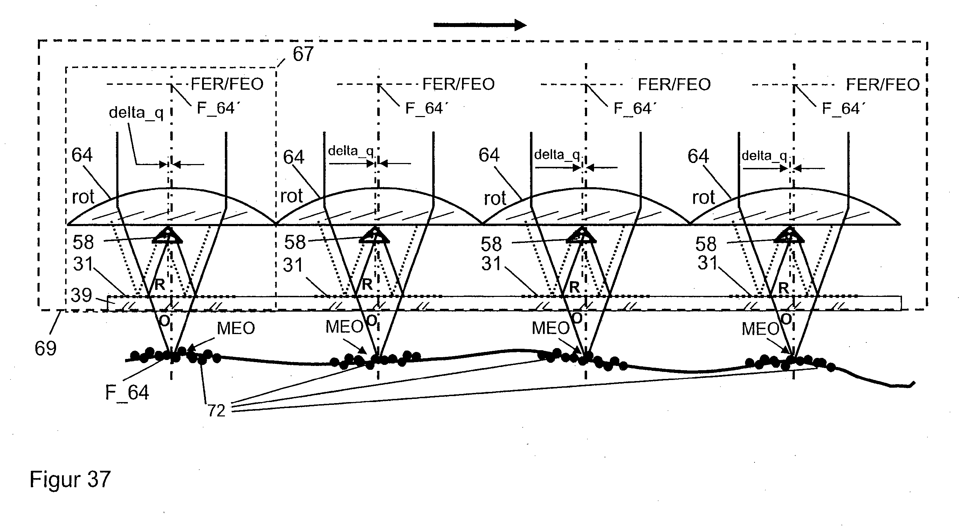

Die Figur 37 zeigt ein Sensor-Array zur mikroskopischen 3D-Untersuchung eines menschlichen Organs zum Auffinden von Krebszellen durch schnellen Linear-Scan auf der Oberfläche des lebenden Organs 72 bei einer chirurgischen Operation.

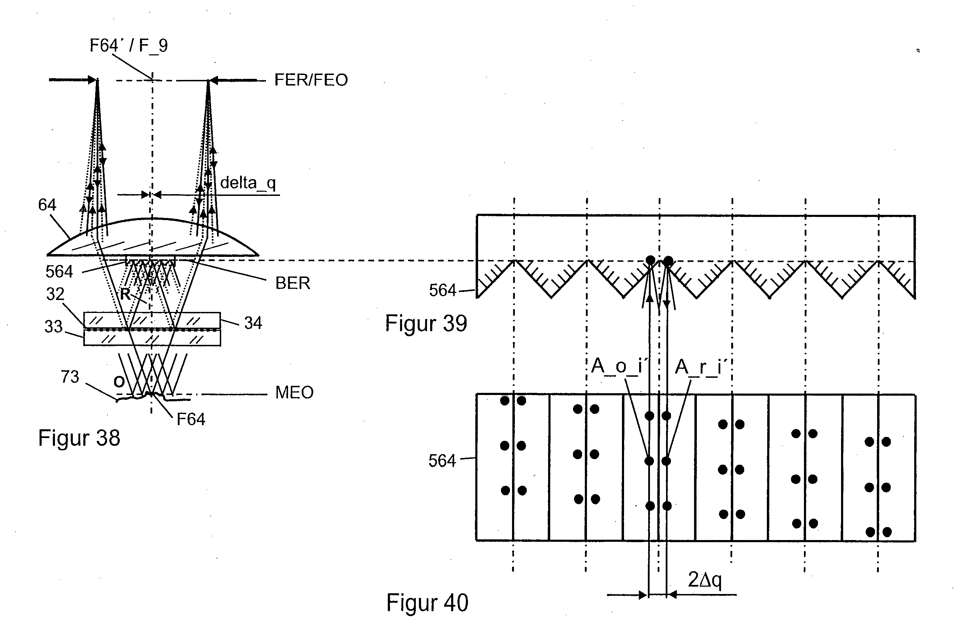

In der Figur 38 wird ein Multi-Linien-Sensor mit einem 90°-Hohl-Dachkantspiegel-Mikro-Endreflektor-Array 564 in Kombination mit einem rotationssymmetrischen Referenzobjektiv 42 im Referenzarm in einer Mirau-Konfiguration dargestellt. Somit können auf einer rauen Metalloberfläche 73 zur Profilerfassung mehrere Messlinien gleichzeitig adressiert werden. Die Figuren 39 und 40 zeigen das 90°-Hohl-Dachkantspiegel-Mikro-Endreflektor-Array 564 in je einer Ansicht.

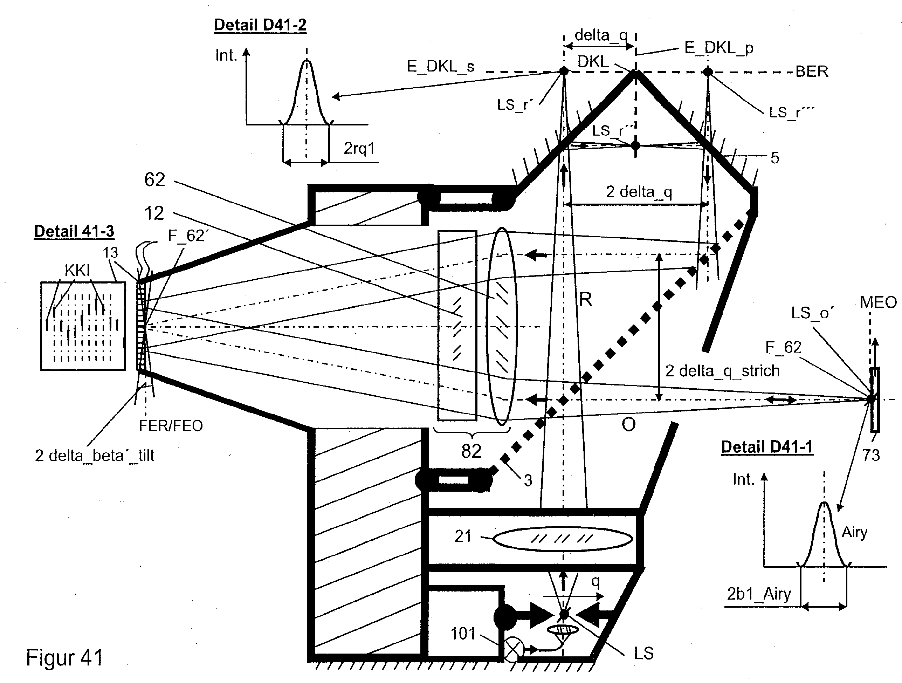

Die Figur 41 stellt ein Sensor-Array zur mikroskopischen 3D-Untersuchung von metallischen Werkstücken dar.

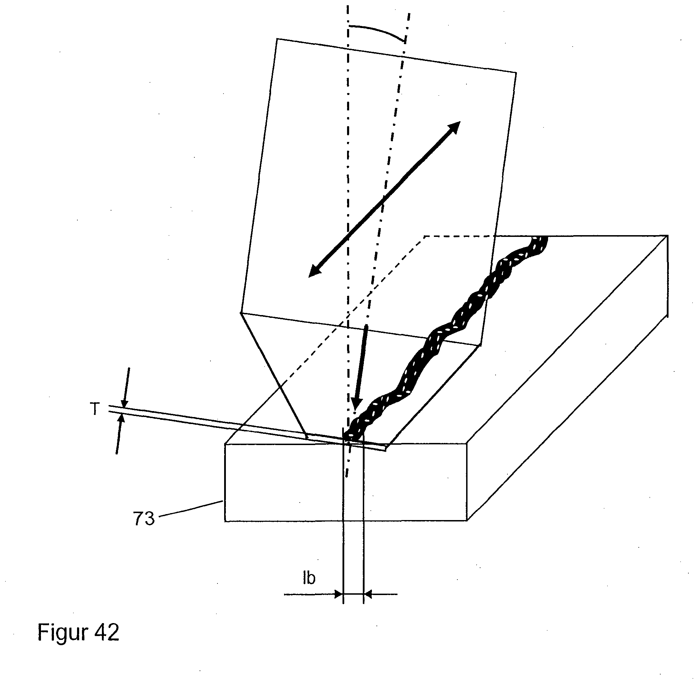

Die Figur 42 stellt einen Linien-Sensorkopf dar, der über ein großes Messobjekt 73 etwas geneigt ist und schnell über das Messobjekt 73 zur Formerfassung bewegt wird. Diese Bewegung erfolgt mittels einer 3-D-Koordinatenmessmaschine. Es wird beim Messen mit "Datenüberschuss" gearbeitet. Viele Messpunkte an optisch rauen Objekten, hier weiß dargestellt, sind nicht-kooperativ, d. h. nicht auswertbar. Dies stellt durch die parallelisierte Aufnahme und des "Datenüberschusses" i. d. R. jedoch kein Problem dar. Aus vielen gültigen Messwerten - hier stellt der schwarze Bereich die gültigen Messpunkte dar - wird das 3D-Profil in Teilbereichen ermittelt, auch, wenn z.T. in Teilbereichen des Messfeldes der Füllgrad mit gültigen Messpunkten z.B. nur 30% beträgt. Jeder Messpunkt erfährt einen "Qualitäts-Check", den er bestehen muss, damit dieser für die Weiterverarbeitung zu einem 3D-Datensatz "zugelassen" wird. Die sich ergebende Profillinie ist demzufolge ggf. nicht ganz geradlinig. Für die numerische Auswertung und weitere Nutzung der Daten muss dies aber in der Regel kein Problem darstellen.

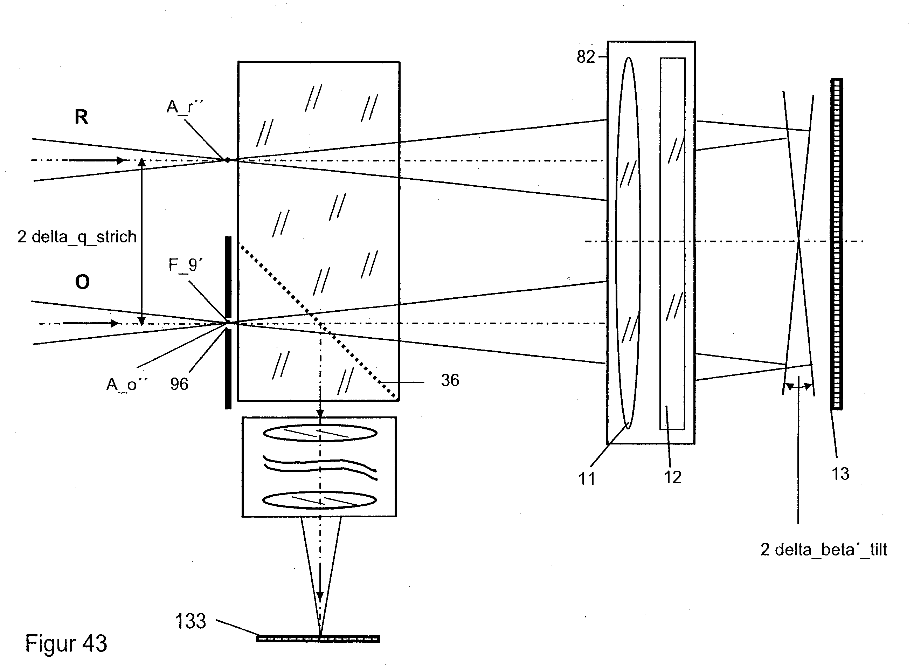

Die Figur 43 stellt einen Teil des Detektionsstrahlenganges mit der Möglichkeit zur Auskopplung von Objektlicht mittels Strahlteiler 36 zur spektroskopischen Analyse dar.

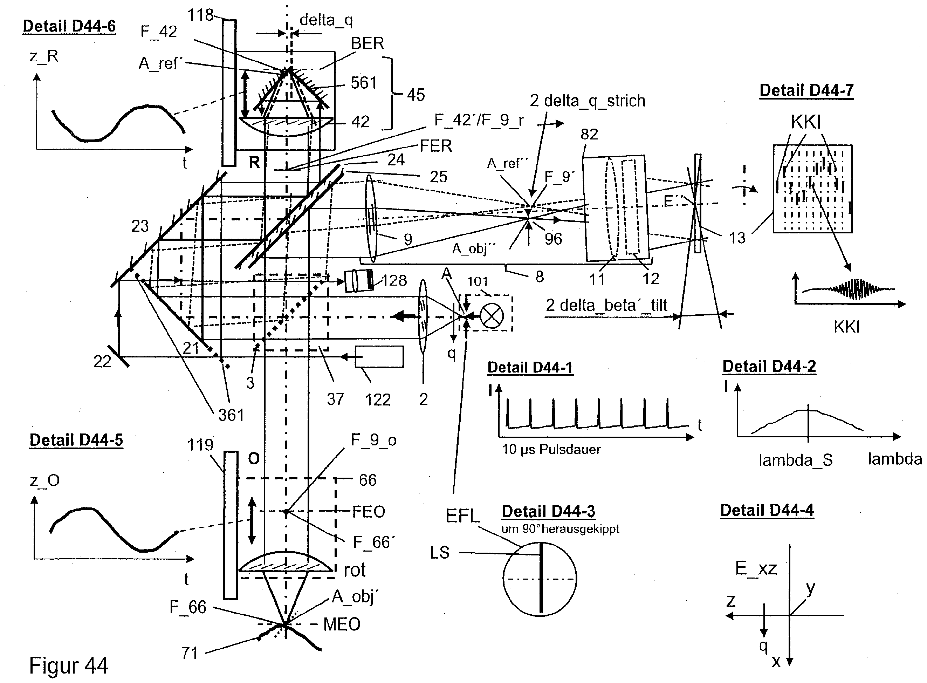

Die Figur 44 stellt ein Hybrid-Interferometer dar. Es erfolgt ein synchronisiertes Gegenschwingen der beiden Objektive 66 und 42 zur weitgehenden Kompensation von Rückstellkräften beim sehr schnellen Beschleunigen der Objektiv-Massen. Der Einsatz der Anordnung erfolgt als hochmobiler Haut-Scanner zur Krebszellen-Detektion für jede Körperregion an einem Menschen.

Das von einer gepulsten kurzkohärenten-Lichtquelle 1 ausgehende Licht - nach einem von dieser Lichtquelle 1 beleuchteten Spalt mit 10 µm Spaltbreite und 5 mm ausgeleuchteter Spaltbreite - wird von einem beugungsbegrenzt arbeitenden Kollimator 2 mit dem halben Aperturwinkel alpha_y_source um 2,8°, der zu einer numerischen Apertur von 0,05 führt, erfasst und kollimiert.

Die wichtigsten Parameter der eingesetzten gepulsten kurzkohärenten-Linien-Lichtquelle 1, s. a. Detail D44-1, im NIR, s. a. Detail D44-2, um 800 nm bis 900 nm sind dabei: 4 kHz Pulsrate und 4% relative Einschaltzeit der gepulsten kurzkohärenten-Lichtquelle 1. Die Lichtpulse werden mittels eines hier nicht dargestellten rotierenden Chopper-Rades erzeugt und weisen eine mittlere Pulszeit von 10 µs auf. Die Kohärenzlänge der Lichtquelle 1 beträgt 8 µm. Die Dauerstrichleistung der gepulsten kurzkohärenten-Lichtquelle 1 beträgt im NIR 100 mW. Die digitale Kamera 13 mit 1024 x 256 Pixeln weist eine Frame-Rate von 4 kHz auf und ist zum Lauf des Chopper-Rades streng synchronisiert.

Das kollimierte Licht gelangt auf einen hochsymmetrisch ausgebildeten und auf besser als 10 µm Glasweglängendifferenz abgeglichenen Strahlteilerwürfel 37 im Interferometer. Das Interferometer arbeitet zumindest näherungsweise am optischen Gangunterschied null. Das transmittierte Strahlenbündel gelangt im Referenzarm R nach dreifacher 90°-Umlenkung in Luft auf ein beugungsbegrenztes Referenzobjektiv 42 mit einem fest in dessen Fokusposition angeordneten Dachkantreflektor 561 mit dessen Dachkante DK in der Brennebene des Referenzobjektivs 42. Die optischen Achsen von Referenzobjektiv 42 und baugleichem Messobjektiv 66 fluchten. Der Dachkantreflektor 561 weist einen Querversatz delta_q zur optischen Achse des Referenzobjektivs 42 auf. Das eingestrahlte Licht wird um 2 delta_q mittels Dachkantreflektor 561 querversetzt und erfährt dabei am Dachkantreflektor 561 auch eine Wellenfrontinversion und trifft nach Passieren des Referenzobjektivs 42 wieder geneigt auf den Strahlteilerwürfel 37, wo das Licht teilweise reflektiert wird und in den Detektionsstrahlengang gelangt. Das am Strahlteilerwürfel 37 nach Lichteintritt reflektierte Strahlenbündel gelangt auf das Messobjektiv 66, welches das Spaltbild mit seiner NA von 0,14 beugungsbegrenzt in Linienform in den Objektraum fokussiert abbildet. Der mechanische Scan-Bereich des mechanisch bewegten Messobjektivs 106 beträgt hier +/- 1 mm Hub. Fokussiertes Licht dringt auch in die menschliche Haut 71 etwas ein kann. Es wird gestreut und breitet sich auch in Lichtung des Messobjektivs 66 aus. Dieses Messobjektiv 66 erfasst das von der menschlichen Haut 71 zurückkommende Licht und lenkt es teilweise in Transmission in Richtung Strahlteilerwürfel 37, wo es in den Detektionsstrahlengang reflektiert wird. Gescannt wird der oberflächennahe Bereich der Haut mit einer maximalen Tiefe von 100µm. Aus den Details D44-5 und D44-6 ist ersichtlich, dass die Objektive hierbei im Messvorgang entgegengesetzt schwingen, um die Rückstellkräfte zu kompensieren.

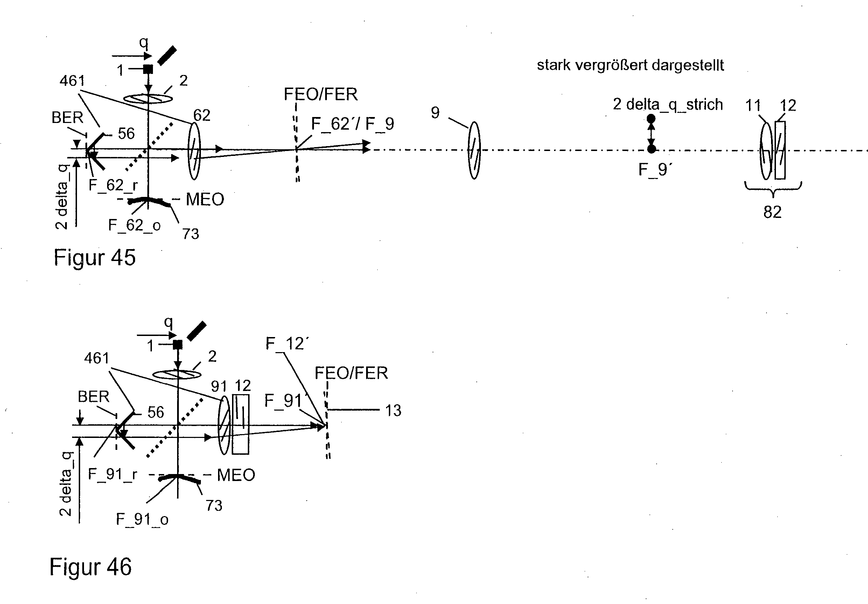

Ab Figur 45 bis Figur 54 werden einige Interferometer-Anordnungen dargestellt, die technisch und wirtschaftlich sinnvoll sind. Die Figur 45 stellt ein Michelson-Interferometer mit dem üblicherweise genutzten Ausgang und einem das Objekt abbildenden Objektiv dar, welches in einmaligem Durchgang genutzt wird. Es entstehen zwei gekippte Wellenfronten in der Fourier-Ebene FEO oder in der vorderen Brennebene F_9 des Tubus-Objektivs 9.

Die Figur 46 stellt ein Michelson-Interferometer mit üblichem Ausgang und Objekt abbildendem Objektiv dar.

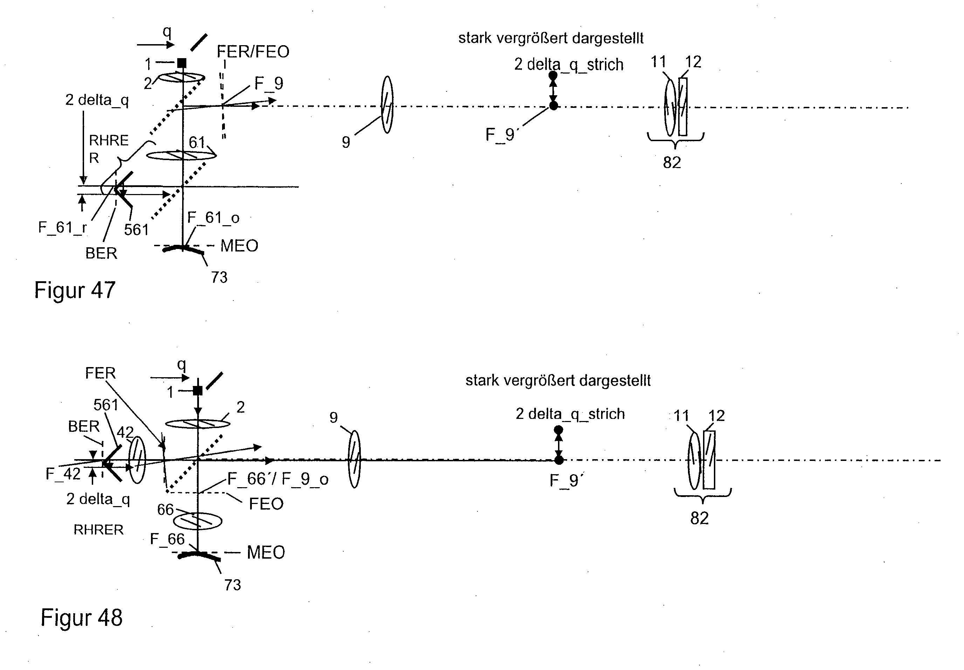

Die Figur 47 stellt ein Michelson-Interferometer mit g-Ausgang dar. Dieses ist hier in der Wirkung gleich einem Mirau-Interferometer. Das Objektiv 61 wird in zweifachem Durchgang genutzt.

Die Figur 48 stellt ein Linnik-Interferometer mit "üblichem" Ausgang dar.

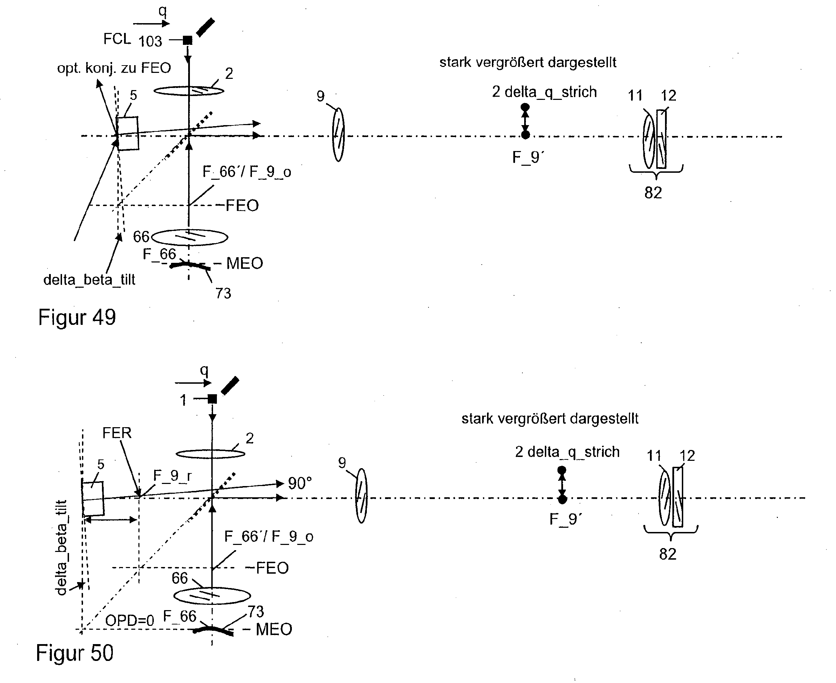

Die Figur 49 stellt ein hybrides Linnik-Interferometer dar. Wegen der ungleichen optischen Weglängen wird hierbei eine Frequenzkamm-Lichtquelle zur Kompensation eingesetzt.

Die Figur 50 stellt ein Hybrid-Linnik-Interferometer mit hier eher unerwünschten Interferenzen gleicher Neigung im Feld dar. Deshalb ist nur ein sehr kleines Feld möglich. Der hohle Dachkantreflektor 5 bildet ohne Objektiv bereits einen Hybrid-Retro-Referenz-Endreflektor und führt die Verkippung der Referenzwellenfront zur Objektwellenfront ein.

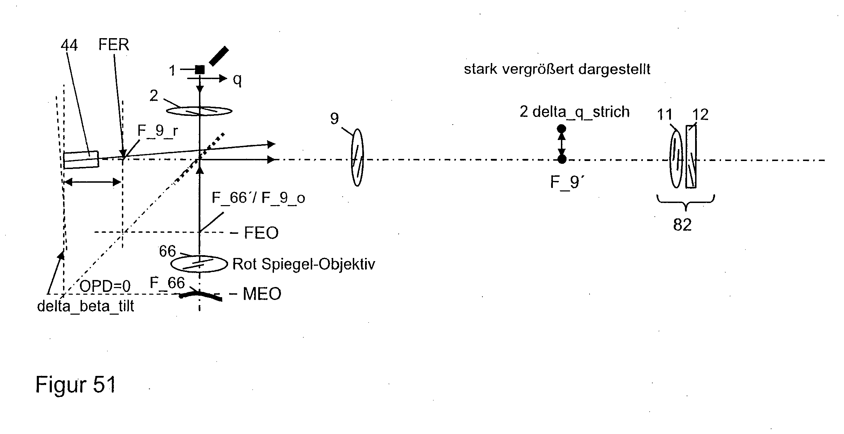

Die Figur 51 stellt ein Hybrid-Linnik-Interferometer mit Zylinder-Spiegel-Objektiv und planem Endspiegel im Referenzarm R dar.

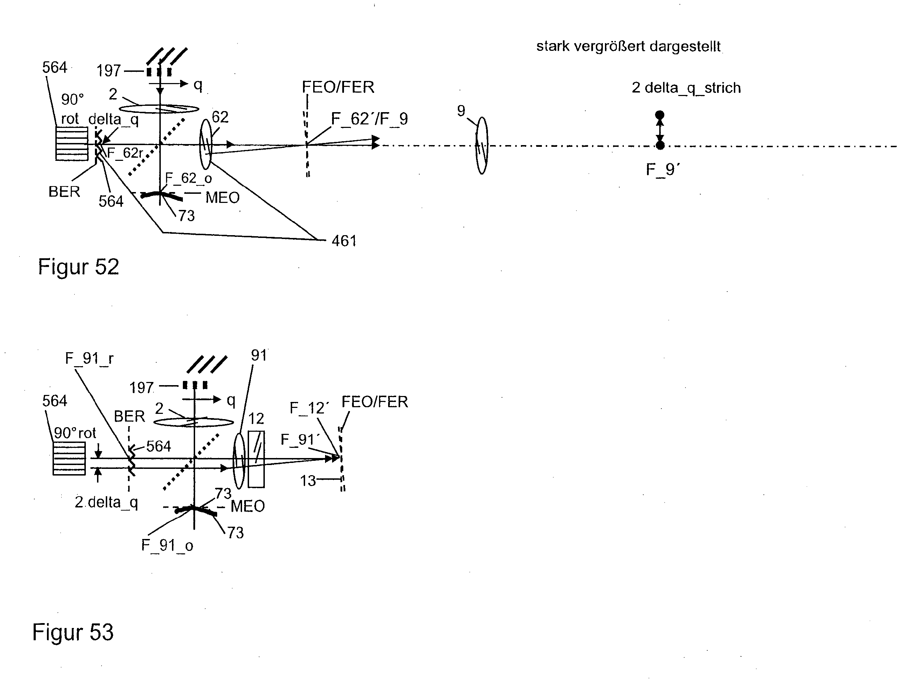

Die Figur 52 stellt ein Michelson-Interferometer mit "üblichem" Ausgang und Objektabbildendem Objektiv und einem Tubus-Objektiv dar.

Die Figur 53 stellt ein Michelson-Interferometer mit üblichem Ausgang und Objekt 73 abbildendem Objektiv dar. Alternativ ist hier auch ein Tripel-Matrix-Array im Referenzarm R einsetzbar. In diesem Fall wird die Lichtquelle als Matrix-Punktquellen-Array ausgebildet. Die Rasterkonstante im Feld ist dann von Lichtquellen-Array und Tripel-Matrix-Array gleichgemacht.

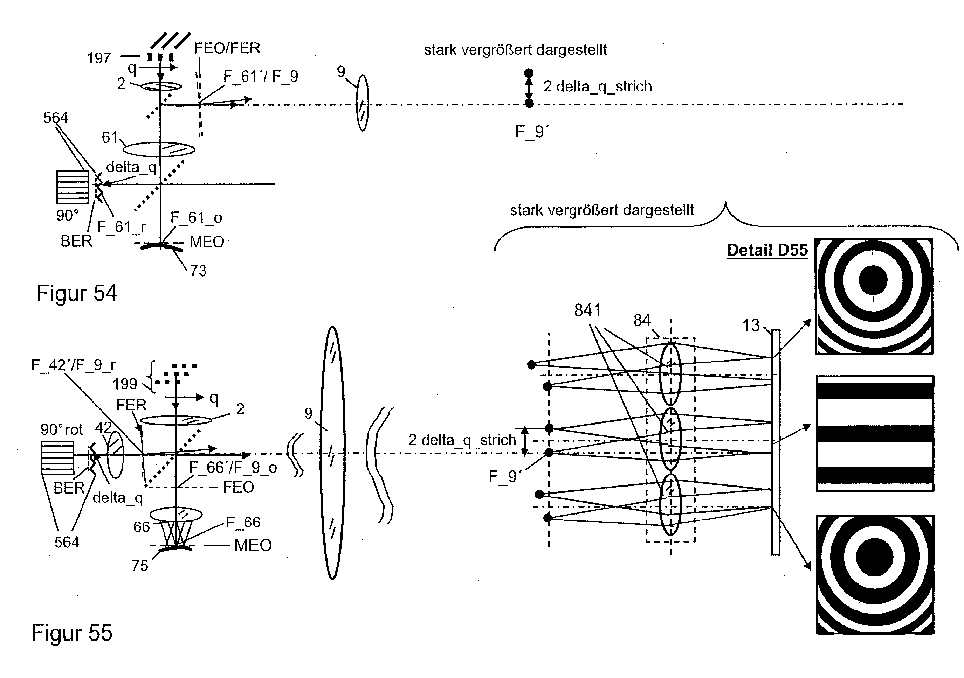

Die Figur 54 stellt ein Michelson-Interferometer mit g-Ausgang dar. Das Michelson-Interferometer ist hier in der Wirkung gleich einem Mirau-Interferometer. Das Objekt-Objektiv 61 wird zweifach genutzt. Alternativ ist hier auch ein Tripel-Matrix-Array im Referenzarm R einsetzbar. In diesem Fall wird die Lichtquelle als Matrix-Punktquellen-Array 199 ausgebildet. Die Rasterkonstante im Feld ist dann von Lichtquellen-Array und Tripel-Matrix-Array gleichgemacht.

Die Figur 55 stellt ein Linnik-Interferometer zur Formmessung einer kleinen schwachen Asphäre dar. Diese schwache Asphäre 75 wird mit einer fein ausgebildeten, gerasterten Punkt-Lichtquellen-Matrix 199 über den Objektstrahlengang des Linnik-Interferometers strukturiert beleuchtet. Die Tubuslinse 9 bildet das mehr oder weniger defokussierte Punktmuster am Ausgang des Interferometers ab. In der Brennebene mit dem Brennpunkt F_9' der Tubuslinse 9 entstehen die scharfen Bilder der Punktquellen, die über den Referenzstrahlengang abgebildet wurden. Im Bereich der Brennebene der Tubuslinse 9 mit dem Brennpunkt F_9' entstehen die mehr oder weniger fokussierten Bilder von den Punktlichtquellen der Punkt-Lichtquellen-Matrix 199, die über die schwache Asphäre 75 abgebildet wurden. Nach dem Mikrolinsen-Array 84 entstehen auf dem gerasterten Detektor 13 räumliche Interferogramme, hier stark vergrößert dargestellt, s. a. Detail D55. In den beiden äußeren räumlichen Interferogrammen ist auch der Defokus zu erkennen, der sich aus der Krümmung der schwachen Asphäre 75 ergibt, da sich diese Flächenbereiche nicht mehr im DOV befinden. Neigung der beleuchteten Flächenbereiche der schwache Asphäre 75 außerhalb der Brennebene des Linnik-Objektivs 66 und Ablage von der Brennebene der Tubuslinse 9 mit dem Brennpunkt F_9' können aus dem Interferogramm berechnet werden.

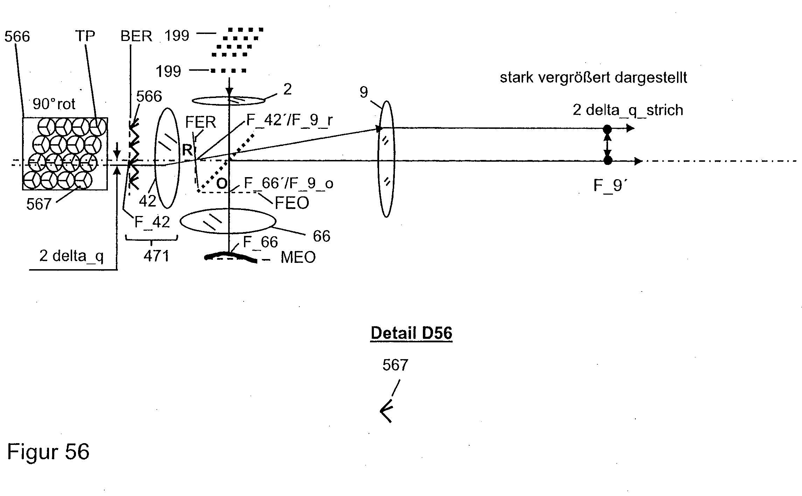

Die Figur 56 stellt ein Linnik-Interferometer mit Punktlichtquellen-Matrix-Array 199 und Hohl-Tripelspiegel-Mikro-Endreflektor-Array 566 dar. Das Hohl-Tripelspiegel-Mikro-Endreflektor-Array 566 bildet mit dem Objektiv 42 ein Voll-Retro-Referenz-Reflektor-System 471.

Es bestehen zwei gekippte Wellenfronten in der Fourier-Ebene eines dem Referenzarm R in Lichtrichtung zum Detektor 13 nachgeordneten Objektivs oder in der vorderen Brennebene F_9 des Tubus-Objektivs 9.

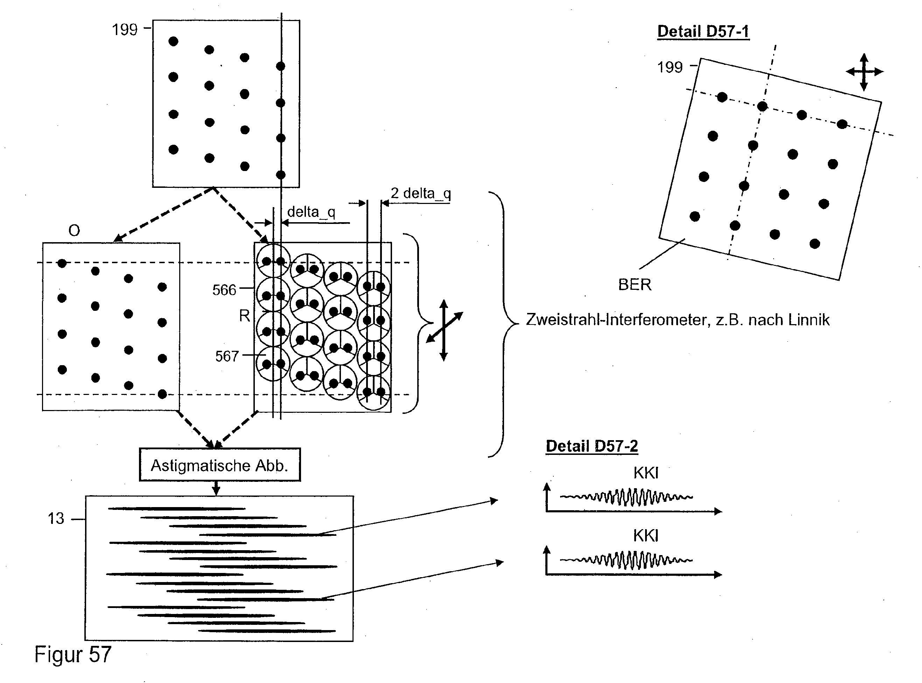

Die Figur 57 stellt ein Voll-Retro-Referenz-Endreflektor-Array mit Tripel-Reflektor dar. Hier sind nicht dargestellte Mittel zur rechnergesteuerten Lateralverschiebung dem Array zugeordnet. Das Punktlichtquellen-Array mit Lichtspots unterhalb der beugungsbegrenzter Auflösung ist lateral versetzt zum Referenz-Voll-Retro-Endreflektor-Array. Jeder Lichtquellenpunkt wird um 2delta_q lateral versetzt. Detail D57-1 stellt die fein ausgebildeten Punkt-Lichtquellen-Matrix 199 und Detail D57-2 stellt typische Kurzkohärenz-Interferogramme dar.

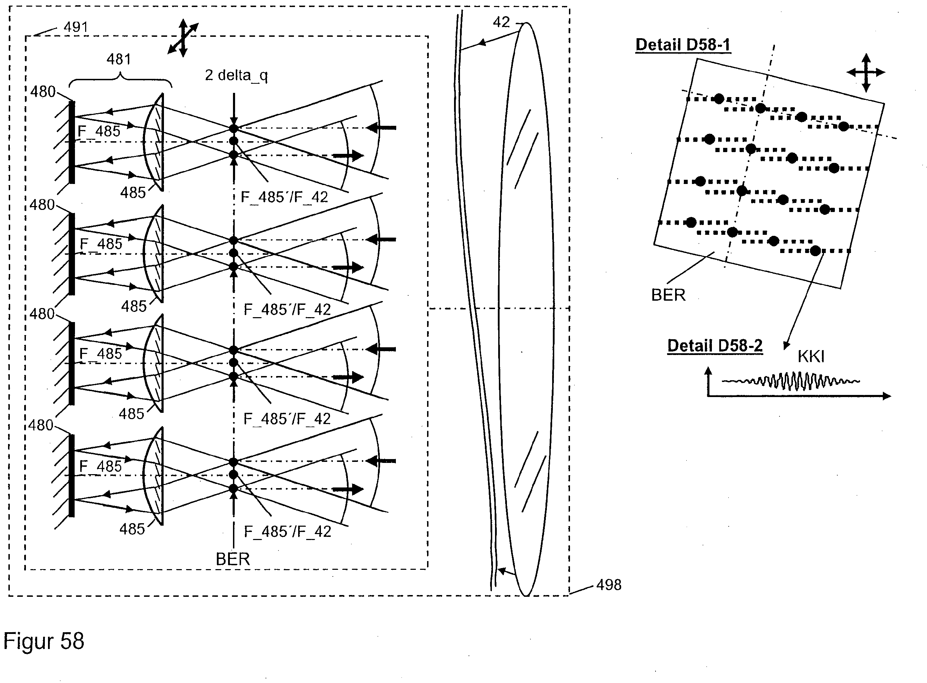

Die Figur 58 stellt ein Mikro-Endreflektor-Array-System 498 mit einem Mikro-Endreflektor-Array 491 mit Voll-Retro-Mikro-Endreflektoren 481 vom rotationssymmetrischen Linsen-Cat'eye-Typ dar. Jeder Voll-Retro-Mikro-Endreflektor ist um delta_ q versetzt. Die Anordnung ist mit einer Frequenz-Kamm-Lichtquelle 103 und einer Kompensations-Platte 569 im Objektarm O ausgebildet. Hier sind nicht dargestellte Mittel zur rechnergesteuerten Lateralverschiebung 493 zugeordnet. Detail D58-1 stellt die Messzellen auf dem gerasterten Detektor 13 dar. In der Patentschrift DE 10 2006 007 172 B4 sind die Beziehungen für das lückenlose Anordnen von länglichen Messzellen auf einem gerasterten Empfänger bei einer Drehung der länglichen Messzellen zur Empfänger-Matrix angegeben.

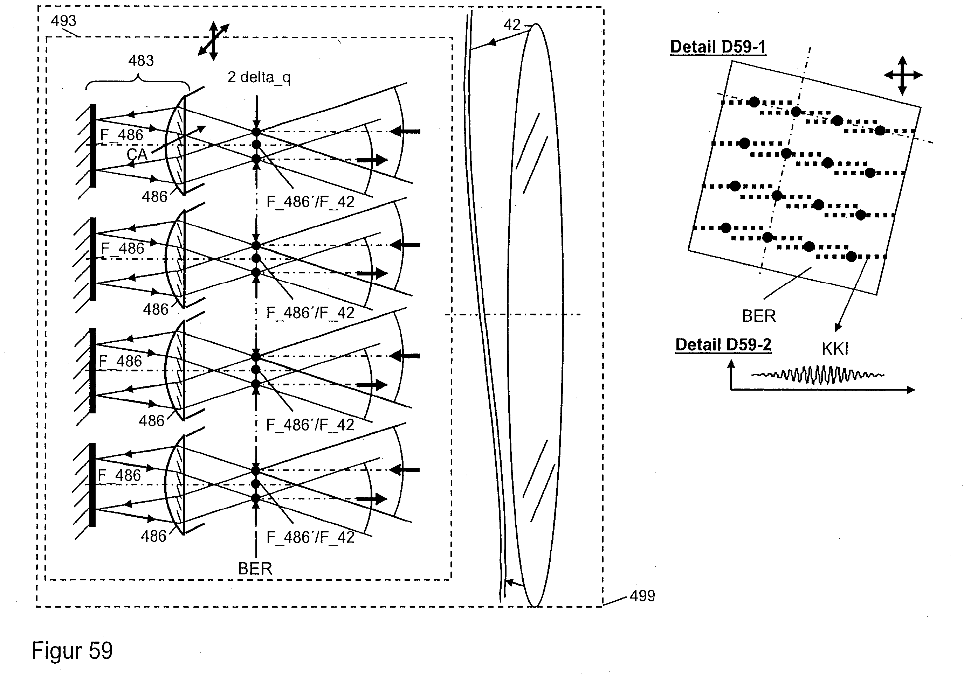

Die Figur 59 stellt ein Mikro-Endreflektor-Array-System 499 mit einem Mikro-Endreflektor-Array 493 mit Hybrid-Retro-Mikro-Endreflektoren 483 vom Zylinder-Linsen-Cat'eye-Typ dar. Jeder Hybrid-Retro-Mikro-Endreflektor 483 ist um delta_q versetzt. Dieser Ansatz wird in Kombination mit einer Frequenzkamm-Lichtquelle 103 und einer Kompensationsplatte 568 im Referenzarm angewendet. Die Mittel zur rechnergesteuerten zweidimensionalen Lateralverschiebung, um eine vollflächige Abtastung des Objekts zu erreichen, sind dem Mikro-Endreflektor-Array 493 zugeordnet, jedoch hier nicht dargestellt. Detail 59-1 stellt die Messzellen in ihrer Anordnung auf dem gerasterten Detektor 13 und Detail 59-2 ein detektiertes Kurzkohärenz-Interferogramm dar.

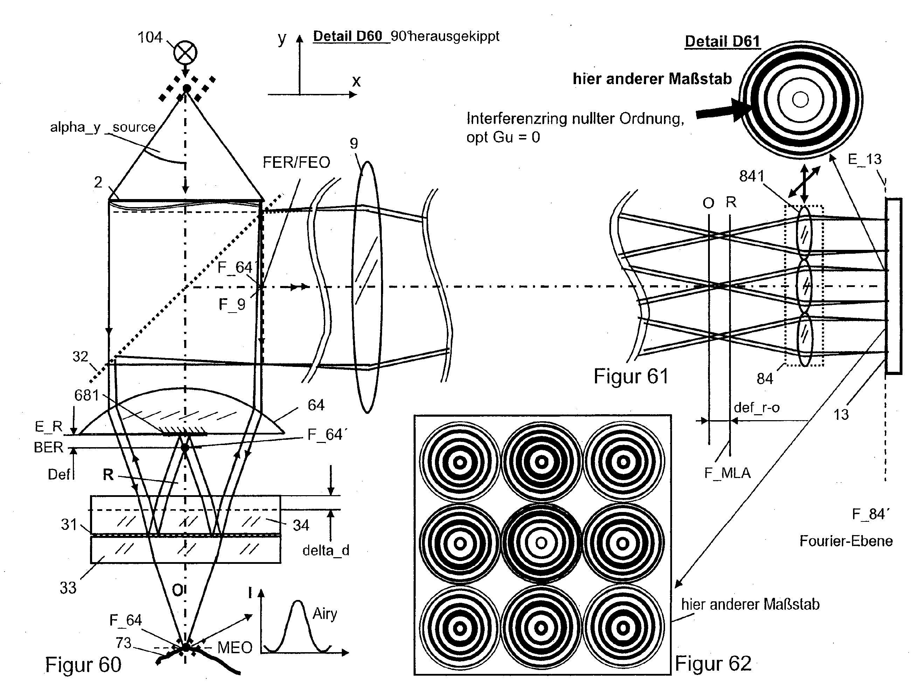

Die Figur 60 stellt ein Mirau-Interferometer dar, welches zur Mikroformmessung eingesetzt werden kann. Das Interferometer ist vorbestimmt asymmetrisch gemacht. Es besteht ein telezentrischer Strahlengang. Die Beleuchtung erfolgt als Kurzpuls. Durch die Asymmetrie ergibt sich, wie in Figur 61 da rgestellt, eine Tiefenseparierung def_r-o von Bildspots im Detektionsstrahlengang. Die Figur 62 stellt die Lichtfeld-Interferenzen mit unterschiedlichen Phasenlagen dar, die sich aus den unterschiedlichen Höhenlagen auf der rauen Metalloberfläche 73 ergeben. Das Mikrolinsen-Array 84 kann zeitlich und lateral synchronisiert mit den Spots der Beleuchtung des räumlichen Lichtmodulators 104 über die raue Metalloberfläche 73 bewegt werden, um mehr Single-shot-Daten gewinnen zu können. Die Single-shot-Daten aus mehreren Messungen können rechnergestützt mittels Merkmals-Korrelations-Algorithmik zusammengefügt werden.

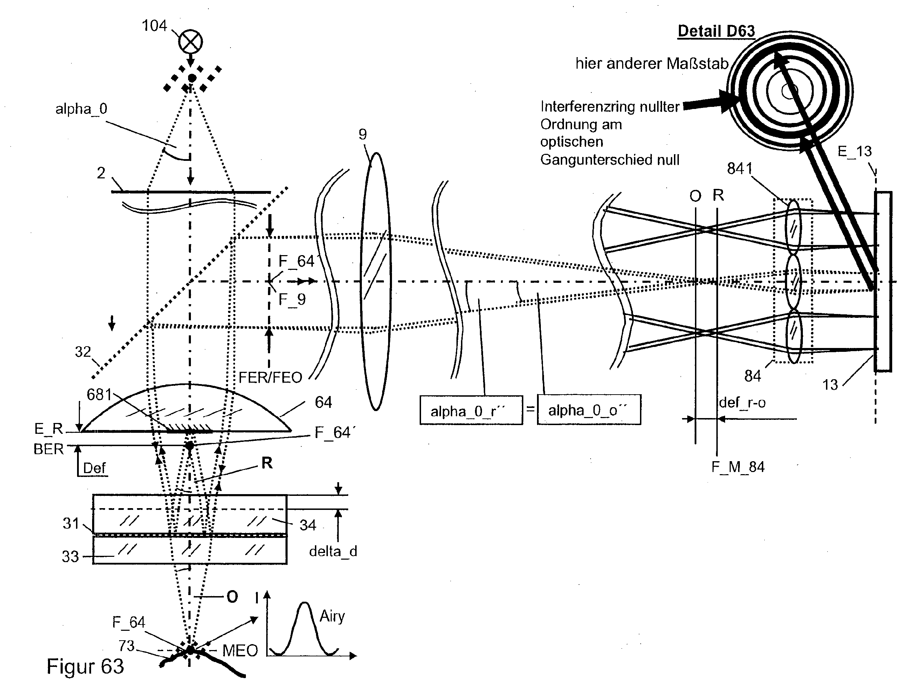

Die Figur 63 stellt das Mirau-Interferometer aus Figur 60 dar, mit einem Winkel alpha_0 aus dem Winkelspektrum der Beleuchtung, für den der optische Gangunterschied bei der Detektion null wird. Das Detail D63 stellt das Lichtfeld-Interferogramm mit einem Interferenzring nullter Ordnung dar, der hier zur Kenntlichmachung fett gezeichnet ist. Der Winkel alpha_0_o" ist dann genau der Winkel im Objektraum-Winkelspektrum, für den - für einen gegebenen Objektpunkt - der optische Gangunterschied in der Ebene E_13 auf dem gerasterten Detektor 13 null wird. Somit entsteht für diesen Objektpunkt der Interferenzring nullter Ordnung etwa in der Mitte des Winkelspektrums bzw. Interferenzringsystems. Da eine radiale Shear zu den Referenzstrahlen besteht, muss das gesamte optische System bis zur Detektion sehr gut korrigiert sein, insbesondere auch chromatisch.

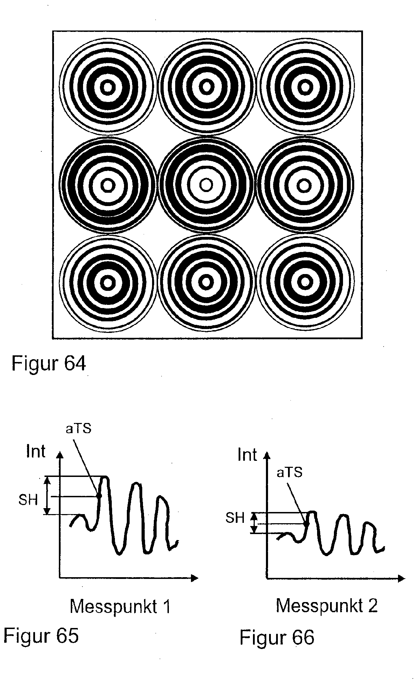

In Figur 64 ist ein Ensemble von Lichtfeld-Interferogrammen - jeweils mit einem Interferenzring nullter Ordnung - dargestellt, das von einem gerasterten Detektor 13 aufgenommen wird.

Die Figuren 65 und 66 stellen Kurzkohärenz-Wavelets mit vorbestimmt eingeführtem Chirping dar. Es wird das Design und die Montage mit Justierung auf einen möglichst großen Hub SH in Kurzkohärenz-Wavelets ausgerichtet. Zur Messung wird der Referenzpunkt aTS als alternative Triggerschwelle bestimmt. Durch den sphärischen Anteil ist das Kurzkohärenz-Wavelet nichtlinear, was bei der Auswertung besonders berücksichtigt werden muss. Jeder Messkanal auf der Grundlage eines räumlichen Interferogramms wird rechnergesteuert angelernt, mittels Teaching-Algorithmik in Verbindung mit einem Referenzmessobjekt sein eigenes Wavelet zu erkennen und auszuwerten. Der Interferometer-Aufbau muss dazu nur über der Zeit hinsichtlich der eingesetzten Komponenten und deren Lagen zueinander hochstabil aufgebaut sein.

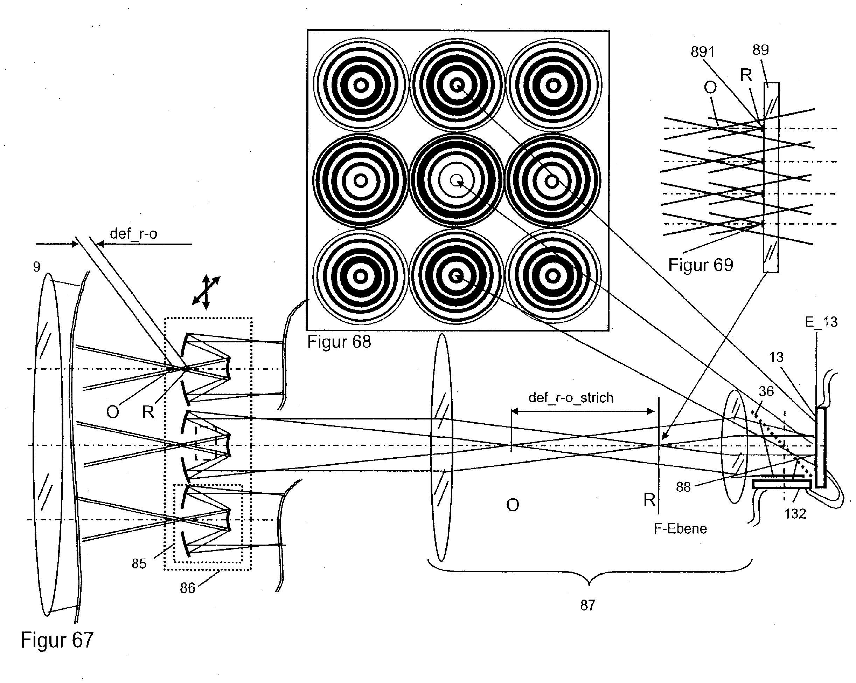

Die Figur 67 stellt einen Interferometeraufbau, der mit gepulster Beleuchtung betrieben wird, mit kleinen Spiegelobjektiven 85 als Fourier-Transformations-Komponente in einem Spiegelobjektiv-Array 86 nach dem Tubusobjektiv 9 dar. Mittels 4f-Transferstufe 87 werden die räumlichen Interferogramme auf den gerasterten Detektor 13 gebildet, dargestellt in Figur 68. In der gemeinsamen Brennebene der 4f-Transferstufe 87 ist ein Mikro-Rotsperrfilter-Array 89 mit Mikro-Rot-Sperrfiltern 891 angeordnet, um Referenzlicht im roten Spektralbereich zu sperren, siehe Figur 69, welche die Ausblendung des Referenzlichts darstellt. So können nach der 4f-Transferstufe 87 nach dem Auskoppel-Strahlteiler 36 mittels Kamera 132, welche mittels eines Rot-Durchlass-Filters 88 allein im roten Spektralbereich sensitiv gemacht ist, Winkelspektren von einzelnen Objektpunkten detektiert werden.

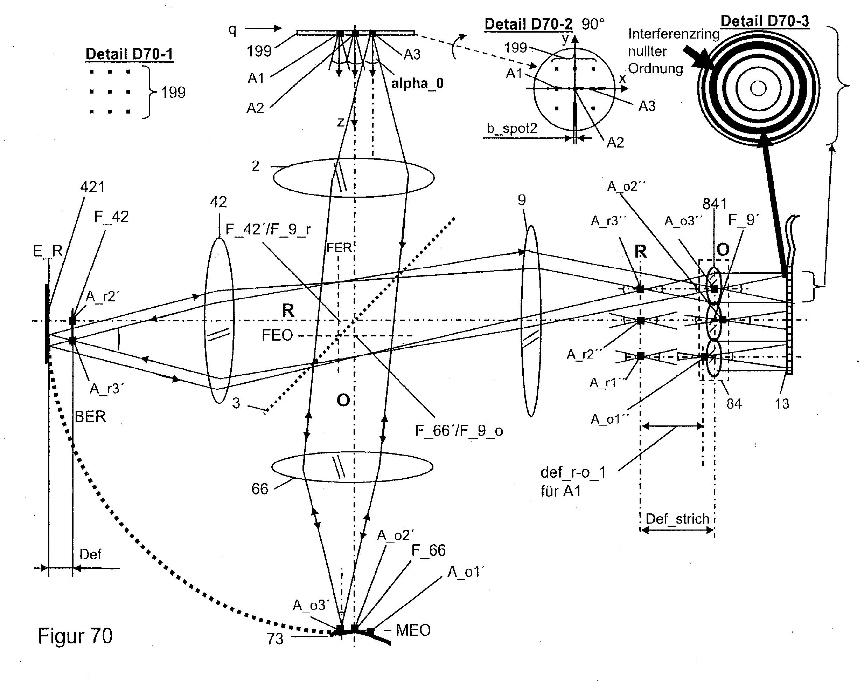

Die Figur 70 präsentiert ein Linnik-Interferometer mit einer fein ausgebildeten Punkt-Lichtquellen-Matrix 199, dargestellt im Detail D70-1, wobei Detail D70-2 die Position der Punkt-Lichtquellen-Matrix 199 darstellt. Im Referenzarm R ist eine kleine Defokussierung Def als Gerätekonstante fest eingeführt. Diese Defokussierung Def wurde durch eine kleine Verschiebung des Objektivs 42 in Richtung Strahlteiler 3 erreicht, wodurch sich die Ringanzahl ergibt, während sich beim gemeinsamen Verschieben von Interferometer-Endspiegel 421 und Objektiv 42 der optische Gangunterschied ändert, also der Streifen nullter Ordnung im Interferenzfeld also im räumlichen Interferogramm radial wandert. Das Linnik-Interferometer wurde dabei so abgeglichen, dass für den Winkel alpha_0 im Winkelspektrum der Beleuchtung sich bei der Detektion auf dem gerasterten Detektor 13 der Streifen nullter Ordnung etwa in der Mitte des räumlichen Interferogramms ergibt, dargestellt im Detail D70-3 für ein einzelnes räumliches Interferogramm. Die Tiefenseparierung def_r-o wird jeweils durch die aktuelle Objektpunktlage bestimmt und die im Referenzarm R fest eingestellte Defokussierung Def.

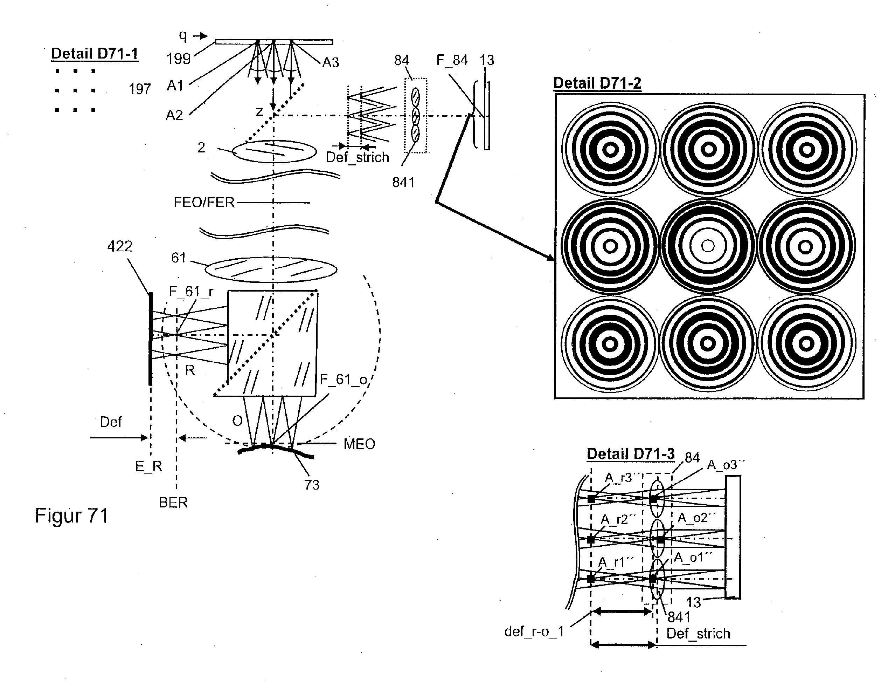

In der Figur 71 ist ein Twyman-Green-Interferometer dargestellt, bei dem die gewünschte Asymmetrie durch ungleiche optische Wege in unterschiedlichen optischen Materialien erzeugt ist. Beleuchtet wird mittels einer fein ausgebildeten Punkt-Lichtquellen-Matrix 199, s. a. Detail D71-1. Im Referenzarm R besteht am optischen Gangunterschied null eine Defokussierung Def. Diese bewirkt im Detektionsstrahlengang eine Defokussierung Def_strich. Die räumlichen Interferogramme, hier auch als Lichtfeld-Interferogramme bezeichnet, werden mittels Mikrolinsen-Array 84 auf den gerasterten Detektor 13 abgebildet. Detail D71-2 zeigt die Lichtfeld-Interferogramme, die aufgrund unterschiedlicher Höhenlagen der Objektpunkte unterschiedliche Phasen in den Lichtfeld-Interferogrammen aufweisen. Das Detail D71-3 stellt eine Anordnung mit dem Detektor 13 in der Fourier-Ebene der Mikrolinsen 841 dar. Die Bildpunkte aus dem Referenzarm und dem Objektarm sind deutlich separiert, so dass in Detail D71-2 gezeigte Interferogramme entstehen können.

In der Figur 72 ist ein Mikrolinsen-Array 84 mit den Mikrolinsen 841 dargestellt. Dieses Mikrolinsen-Array 84 ist in einem Twyman-Green-Interferometer nach dem Tubusobjektiv 9 angeordnet, wie es auch im Detail D71-3 der Figur 71 dargestellt ist. Detektiert wird die Interferenz in der Fourier-Ebene der Mikrolinsen 841 auf dem gerasterten Detektor 13. Die dort entstehenden Lichtfeld-Interferenzen sind im Detail D72 dargestellt. Bei Verwendung von kurzkohärentem Licht kann bei entsprechender Systemauslegung auch ein Ring nullter Ordnung detektiert werden. Es entstehen hier auf dem gerasterten Detektor 13 auch dezentrierte Interferenz-Ringsysteme, da ein optisch glattes Objekt mit gekrümmter Oberfläche vermessen wird, bei dem ein erfasster Teilbereich der Oberfläche etwas defokussiert ist und somit eine Neigung der angemessenen Oberfläche besteht. Die zur Interferenz kommende Wellenfront vom Objekt trägt bei Defokussierung bei der Objektbeleuchtung im Interferometer stets auch die Information über die Objektform selbst, so dass die Objektwellenfront bei der Interferenz auf dem gerasterten Detektor 13 bei einem asphärischen defokussierten Objekt auch asphärisch ist.

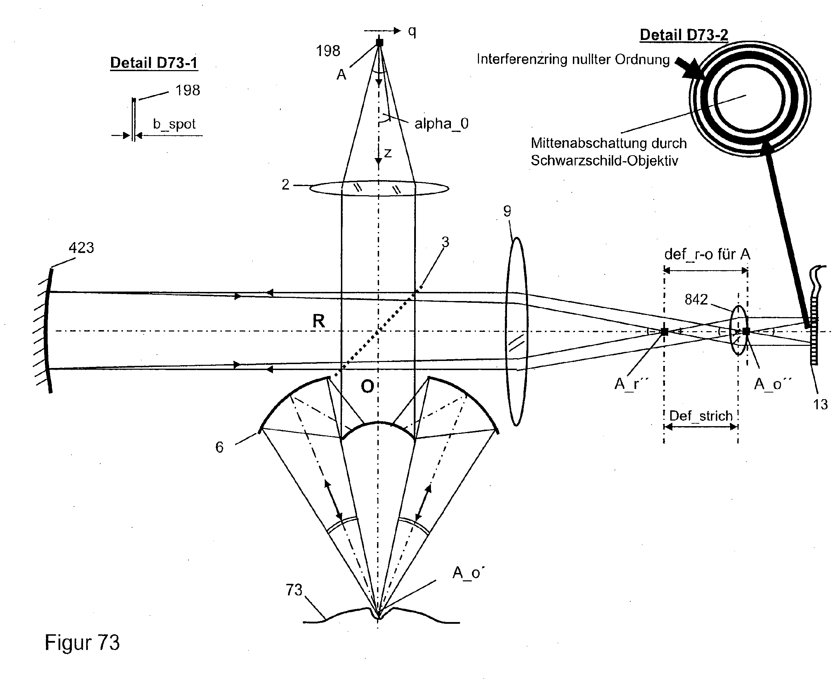

Die Figur 73 stellt ein Zweistrahlinterferometer mit einem objektabbildenden Schwarzschild-Objektiv 6 im Objektarm 0 dar. Die Punkt-Lichtquelle 188 erzeugt einen feinen Lichtspot, der in seiner Lateral-Ausdehnung b_spot unterhalb der beugungsbegrenzten Auflösung des nachfolgenden Kollimatorobjektivs 2 ist, s. a. Detail D73-1. Die Lichtquelle 188 ist mit einer Frequenzkamm-Lichtquelle, die hier nicht dargestellt ist, ausgebildet. Diese Frequenzkamm-Lichtquelle weist eine optische Verzögerungslänge Y auf, die zumindest näherungsweise gleich dem optischen Gangunterschied im Interferometer gemacht ist oder der optischen Gangunterschied im Interferometer beträgt ein ganzzahliges Vielfaches der optischen Verzögerungslänge Y. Im Referenzarm R ist ein konkaver, rotationssymmetrischer Spiegel 423 mit einer schwachen Krümmung angeordnet. Aus der Krümmung ergibt sich im Zusammenwirken mit dem Zweistrahlinterferometer die Ringanzahl auf dem gerasterten Detektor 13.

Die Geometrie des Zweistrahlinterferometers wurde dabei so auf die Frequenzkamm-Lichtquelle abgestimmt, dass sich bei der Detektion auf dem gerasterten Detektor 13 bei Position eines Objektpunkts in der Mitte des wellenoptischen Schärfentiefebereichs der Streifen nullter Ordnung etwa in der Mitte des räumlichen Interferogramms ergibt, dargestellt im Detail D73-3. Die Tiefenseparierung def_r-o wird jeweils durch die aktuelle Objektpunktlage bestimmt und die im Referenzarm R durch den konkaven, rotationssymmetrischen Spiegel 423 fest vorgegebene Spiegelkrümmung.

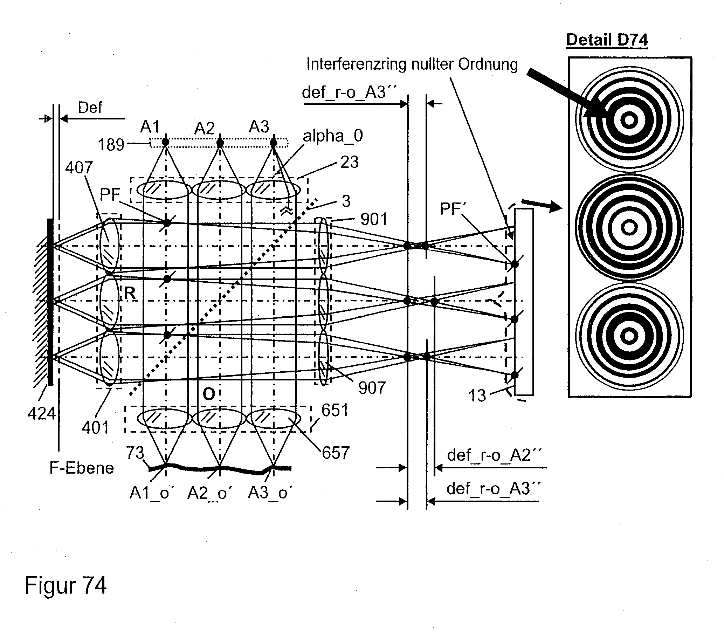

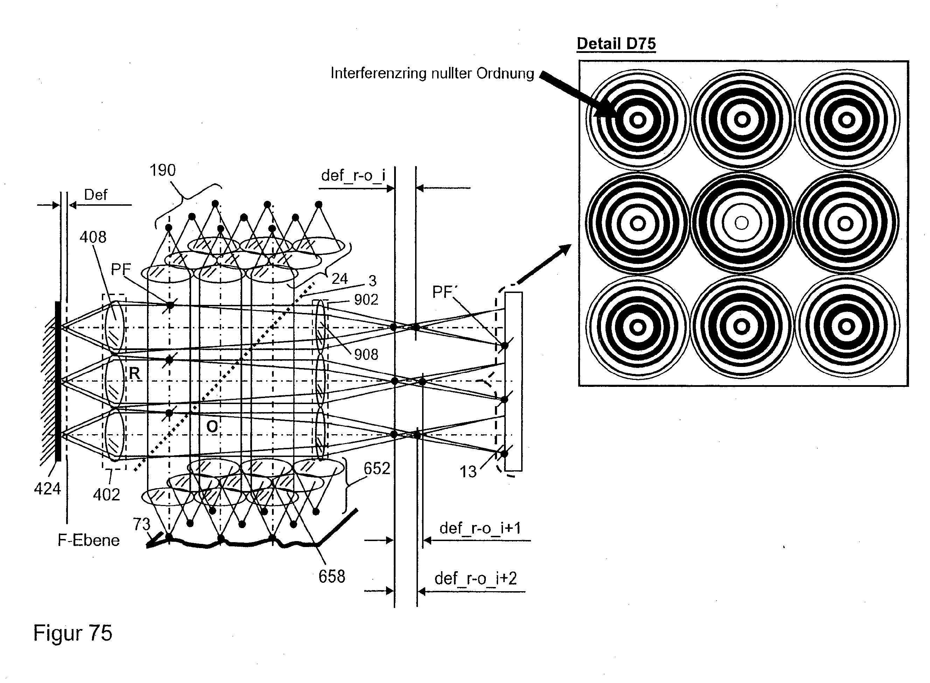

Die Figuren 74 und 75 stellen jeweils eine miniaturisierte Variante des Single-Shot-Ansatzes in Form eines vielkanaligen Linnik-Interferometers ausschließlich mit Optiken in Array-Form dar.

Figur 74 stellt dar, wie das von einer Linien-Lichtquelle 189 mit feinen Lichtspots A1, A2, A3 ausgehende Licht durch die Mikro-Kollimator-Objektive eines linienhaften Arrays 23 jeweils kollimiert wird. Dieses Licht wird hierbei mittels spektral breitbandiger leistungsstarker LEDs erzeugt, denen je ein Pinhole nachgeordnet ist. Dabei ist die Pinhole-Größe unterhalb der beugungsbegrenzten Auflösung der nachfolgenden Mikro-Kollimator-Objektive des linienhaften Arrays 23. Am Strahlteiler 3 des vielkanaligen Linnik-Interferometers entstehen jeweils Referenz- und Objektstrahlenbündel. Im Referenzarm R mit einem Mikrolinsen-Array 401 in Linienform mit rotationssymmetrischen Mikrolinsen 407 besteht eine fest eingestellte schwache Defokussierung Def. Das vielkanalige Linnik-Interferometer ist hier so justiert, dass die Foki im Referenzarm R am optischen Gangunterschied null in einer intrafokalen Position sind. Die reflektierten Lichtbündel sind schwach konvergent und treffen nach dem Strahlteiler 3 auf die Tubus-Mikrolinsen 907 des linienhaften Mikrolinsen-Arrays 901. Durch die schwache Defokussierung Def im Referenzstrahlengang ist bei einer Objektpunktlage im wellenoptischen Tiefenschärfebereich des Objektarm 0 als Folge dessen dann im Detektionsstrahlengang eine eindeutige Separierung der Foki def_r-o von Objekt- 0 und Referenzarm R gegeben. So entsteht in der Ebene der Detektion von jedem erfassten Objektpunkt im wellenoptischen Tiefenschärfebereich auf dem gerasterten Detektor 13 eine Interferenzfigur mit Ringen und dabei auch einem Interferenzring nullter Ordnung. Dazu wird zumindest näherungsweise jeweils die Fourier-Ebene der Mikrolinsen des Mikrolinsen-Arrays 401 auf den gerasterten Detektor scharf abgebildet. Das Detail D74 zeigt die entstehenden Interferenzfiguren, hier beispielhaft für die drei Kanäle dargestellt. Je nach Tiefenlage eines Objektpunkts des Objekts ergibt sich eine andere Position für den Interferenzring nullter Ordnung, die mittels Rechnerprogramm ausgewertet wird.

Figur 75 stellt in Ergänzung zu Figur 74 den Ansatz in einer flächenhaften Anordnung mit 3x3 Messkanälen dar. Das Detail D75 zeigt die Interferenzfiguren, hier beispielhaft für die neun Messkanäle dargestellt. Allein aus Darstellungsgründen wurden hier nur die neun Mikrolinsen des Beleuchtungs-Arrays 24 und des Tubus-Arrays 902 präsentiert. Je nach aktueller Tiefenlage eines Objektpunktes ergibt sich eine jeweils aufgrund des dann gegebenen optischen Gangunterschieds auch andere Position für den Interferenzring nullter Ordnung.

Eine besonders stabile Ausbildung für das vielkanalige Linnik-Interferometer ergibt sich jeweils für die Anordnungen in Figur 74 und 75, die hier nicht dargestellt ist, wenn der Strahlteiler als kompakter Strahlteilerwürfel ausgebildet ist und die Mikrolinsen-Arrays 401 beziehungsweise 402 und der ebene Interferometer-Endspiegel im Referenzarm 424 mit dem Strahlteiler in Würfelform durch Kleben oder Kitten starr verbunden sind. Der Vorteil bei dieser Anordnung besteht darin, dass sowohl im Referenzarm R als auch im Objektarm O keine zusätzlichen Glaswege bestehen. Dies ermöglicht Mikrolinsen mit einer vergleichsweise hohen numerischen Apertur einzusetzen, beispielsweise auch Mikrolinsen mit einer numerischen Apertur von 0,5.

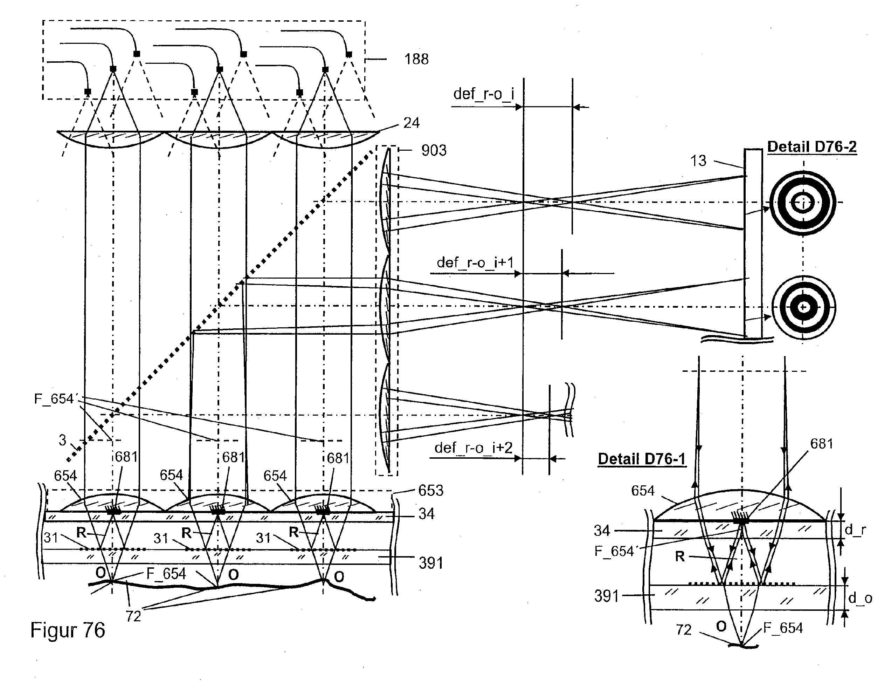

Figur 76 stellt, entsprechend Ref. [11], Abbildung 6, einen vielkanaligen Mirau-Interferometer-Ansatz dar. Dieser kann auf einer miniaturisierten Interferometer-Anordnung auch gemäß Figur 60 mit einem gemeinsamen Auskoppelstrahlteiler 32 und einem Tubus-Mikrolinsen-Array 901 gemäß Figur 74 und 75 bestehen. Auch beim vielkanaligen Mirau-Interferometer können so wie beim vielkanaligen Linnik-Interferometer die Mirau-Interferometer-Komponenten starr miteinander verbunden sein, da es zur One-shot-Signalbildung hierbei prinzipbedingt keine Verschiebung von Komponenten des Mirau-Interferometers zueinander geben muss.

Beim vielkanaligen Mirau entfällt im Vergleich zum vielkanaligen Linnik-Interferometer die Notwendigkeit der genauen Mikrolinsen-Pupillenjustierung, Fehllagen der Mikrolinsen-Pupillen können beim Linnik-Interferometer zu Wellenfrontfehlern und zu dezentrierten Interferenzringen führen.

Das Detail D76 zeigt ein einzelnes Mirau-Interferometer, also einen einzigen Messkanal, der Array-Interferometeranordnung, auch als Vielkanal-Interferometer-Anordnung zu bezeichnen, nach Figur 76. Die Strahlteilerplatte 391 und die Kompensationsplatte 34 sind aus demselben Glaswerkstoff BK7 gefertigt und weisen mit d_o und d_r nur schwach unterschiedliche geometrische Dicken auf, beispielsweise mit einer Dickendifferenz im unteren zweistelligen Mikrometerbereich. Die sich deshalb ergebende Fokusverschiebung führt zu einem, z um ebenen Referenzspiegel 681 ve rschobenen Fokus-Spot. Somit überlagert sich nach der Reflexion im Rücklauf des Lichts in der Brennebene F_654' der Fourier-Ebene, der Objektwelle stets eine schwach gekrümmte Referenzwelle. Diese Fourier-Ebene wird jeweils in jedem Messkanal auf den gerasterten Detektor 13 abgebildet. Die Abstimmung der Mirau-Interferometer mit d_o und d_r sowie der bestehenden numerischen Apertur und der Schwerpunktwellenlänge erfolgt so, dass sich für die Fokusposition der Objektpunkte in der Mitte des wellenoptischen Schärfentiefebereichs jeweils ein Ring nullter Ordnung zumindest näherungsweise am halben Radius des größten detektierbaren Ringes ergibt.

Ein Hybrid-Retro-Referenz-Mikro-Endreflektor kann ein Reflektor sein, der nur in einer einzigen ausgezeichneten Einfallsebene einen einfallenden Strahl (im geometrisch-optischen Modell) unabhängig von seinem Einfallswinkel parallel zu sich selbst (zurück-)reflektiert. Ein Hybrid-Retro-Referenz-Mikro-Endreflektor kann z.B. mittels eines miniaturisierten Dachkantspiegels, eines miniaturisierten Dachkantprismas oder eines miniaturisierten fokussierenden Systems mit refraktiver oder spiegelnder miniaturisierter Zylinderoptik und nachgeordnetem Spiegel aufgebaut werden. Die o.g. ausgezeichnete Einfallsebene steht dabei entweder senkrecht auf der Dachkante oder senkrecht zur Zylinderachse der Zylinderoptik. Dachkantspiegel stellen hierbei stets Hohl-Spiegel dar.

Ein Hybrid-Retro-Referenz-Mikro-Endreflektor kann ebenfalls als eindimensional wirkender Retro-Reflektor bzw. als (Mikro-)Retroreflektor bezeichnet werden (z.B. s. a EP 0 929 978 B1, Absatz 0038 Zeile 1 und dort Komponente 22).

Ein Voll-Retro-Referenz-Mikro-Endreflektor kann ein miniaturisierter Retro-Reflektor sein, der jeden einfallenden Strahl (im geometrisch-optischen Modell) unabhängig von seinem Einfallswinkel und dessen azimutaler Orientierung zumindest näherungsweise parallel zu sich selbst (zurück-)reflektiert. Ein Voll-Retro-Referenz-Mikro-Endreflektor kann z.B. mit einem miniaturisierten Hohl-Tripelspiegel, beziehungsweise miniaturisierten Tripelprisma oder einem miniaturisierten fokussierenden System (mit refraktiven oder spiegelnden oder auch Spiegellinsen) mit einer Spiegelfläche durch den abgewandten Fokuspunkt aufgebaut werden. Dabei ist der Abstand von einem ausgezeichneten Punkt des Voll-Retro-Referenz-Mikro-Endreflektors (Tripelpunkt, Raumeckenpunkt oder vorderer Brennpunkt des fokussierenden Systems) des hinlaufenden Strahl gleich dem des zurücklaufenden Strahls. Tripelspiegelsysteme (Hohlspiegel) werden auch als trihedrale Spiegeloptiken bezeichnet. Dagegen stellen Cat's eye-Anordnungen einen Spezialfall des Retro-Reflektors mit Fokussier-und Spiegeloptik dar.

## Patentansprüche

1. Anordnung zur robusten One-shot-Interferometrie umfassend
eine Lichtquelle (1, 101, 102, 103, 191; 192, 193, 194, 195, 196, 198, 199, LS), einen gerasterten Detektor (13) mit Empfängerelementen für elektromagnetische Strahlung, und
ein Zweistrahl-Interferometer mit Referenz- und Objektarm und mit den folgenden Komponenten:
ein der Lichtquelle nachfolgendes bzw. nachgeordnetes Lichtquellen-Objektiv (2, 21) zur zumindest näherungsweise beugungsbegrenzten Abbildung derselben,
ein das Objekt (71, 72, 73, 74) abbildendes Objektiv (6, 61, 62, 63, 64, 66, 91), wobei im Referenzarm des Zweistrahl-Interferometers ein als Retroreflektor ausgebildeter Endreflektor angeordnet ist, welcher ausgelegt ist, eine oder mehrere verkippte Referenz-Wellenfronten zu erzeugen; und das Zweistrahl-Interferometer derart ausgelegt ist, zumindest ein Paar zueinander gekippter Referenz- und Objektwellenfronten zu erzeugen, und zur Interferenz zu bringen,
wobei die Lichtquelle mindestens eine, in mindestens einer Quer-Achsen-Richtung (q) schmal ausgebildete Linien-Lichtquelle (1, 101, 102, 103, 191, 192, 193, 194, 196, LS) oder ein Linien-Lichtquellen-Array (191, 197) mit einer Vielzahl von in einer Quer-Achsen-Richtung schmal gebildeten, zumindest näherungsweise parallelen Linienspots ist, und
die Linien-Lichtquelle (1, 101, 102, 103, 192, 193, 194, 195, 196, LS) oder die Linienspots des Linien-Lichtquellen-Arrays (191, 197) in der Quer-Achsen-Richtung (q) jeweils so schmal ausgebildet ist bzw. sind, dass diese für das nachfolgende Lichtquellen-Objektiv (2, 21) in dieser Quer-Achsen-Richtung (q) zumindest näherungsweise gleich oder unterhalb dessen beugungsbegrenzter Lateral-Auflösung ist bzw. sind, wobei die Lateral-Auflösung durch die numerische Apertur des Lichtquellen-Objektivs (2, 21) und der eingesetzten Lichtwellenlänge oder der eingesetzten Lichtwellenlängen der Linien-Lichtquelle (1, 101, 102, 103, 192, 193, 194, 195, 196, LS) oder des Linien-Lichtquellen-Arrays (191, 197) bestimmt ist, und wobei die Lichtquelle (1, 101, 102, 103, 192, 193, 194, 195, 196, LS) mittels des das Objekt abbildendes Objektivs (6, 61, 62, 63, 64, 66, 91) in mindestens einer Dimension auf das Objekt beugungsbegrenzt abgebildet wird, und
wobei
(i) die Anordnung zur robusten One-shot-Interferometrie ferner ein, dem Endreflektor zugeordnetes Referenzobjektiv (4, 42) enthält, das im Referenzraum angeordnet ist, oder wobei das das Objekt abbildende Objektiv ein Objektiv (61, 62, 63, 64, 91) ist, das sowohl dem Referenz- als auch dem Objektarm zugeordnet und außerhalb des Referenzarms (R) angeordnet ist;
der Endreflektor ein Hybrid-Retro-Referenz-Endreflektor (45; 461), also ein eindimensionaler Retro-Referenz-Endreflektor ist, und
der Hybrid-Retro-Referenz-Endreflektor (45; 461) mindestens einen 90°-Dachkant-Endreflektor (5, 56, 561, 562, 57, 58, 581, 59) aufweist, wobei die Dachkante (DK) des 90°-Dachkant-Endreflektors (5, 56, 561, 562, 57, 58, 581, 59);
zumindest näherungsweise in der Brennebene des im Referenzarm angeordneten Referenzobjektivs (4, 42) oder des außerhalb des Referenzarms (R) angeordneten Objektivs (61, 62, 63, 64, 91) positioniert ist; und
zumindest näherungsweise senkrecht zur Quer-Achsen-Richtung (q) angeordnet ist, wenn im Referenzarm kein fokussierendes Referenzobjektiv angeordnet ist; oder zumindest näherungsweise senkrecht zur Quer-Achsen-Richtung (q) angeordnet ist, wenn im Referenzarm ein fokussierendes Referenzobjektiv angeordnet ist; und
ein Querversatz (delta_q) der Dachkante (DK) zumindest näherungsweise parallel zur Richtung der Quer-Achsen-Richtung (q) entweder in der Brennebene (BER) des dem Hybrid-Retro-Referenz-Endreflektor (45) zugeordneten Referenzobjektivs (4, 42) oder in der Brennebene (BER) des sowohl dem Referenz- als auch dem Objektarm zugeordneten Objektivs (61, 62, 63, 64, 91) besteht, und der Querversatz delta_q maximal gleich dem zehnten Teil der Brennweite dieses Objektivs (4, 41, 42, 44, 61, 62, 63, 64, 91) und mindestens gleich oder größer als der objektivbezogene Airy-Scheibchen-Radius für die Schwerpunktwellenlänge im detektierten räumlichen Interferogramm ist;
oder
(ii) der Endreflektor ein Hybrid-Retro-Referenz-Endreflektor ist, welcher entweder ausgebildet ist als Zylinder-Optik-Hybrid-Retro-Referenz-Endreflektor (44) mit einem Zylinder-Objektiv (41) mit einer Zylinderachse (CA) und einem Planspiegel (43) in der Brennebene des Zylinder-Objektivs (41), wobei die Zylinderachse (CA) jeweils senkrecht zur Längsrichtung der Linienlichtquelle ist, oder ausgebildet ist als Dachkantreflektor (5), wobei die Dachkante des Dachkantenreflektors (5) senkrecht zur Längsrichtung der Linienlichtquelle ist,
wobei eine Kippung (delta_beta_tilt) des Hybrid-Retro-Referenz-Endreflektors (44, 5) um eine Kipp-Achse besteht, die zumindest näherungsweise sowohl senkrecht zur Quer-Achsen-Richtung (q) als auch senkrecht zur Richtung der Lichtausbreitung ist, jeweils bezogen auf einen entfalteten Strahlengang des Zweistrahl-Interferometers, und
der Kippwinkel maximal gleich oder kleiner 6 Altgrad und mindestens gleich oder größer als eine halbe Schwerpunktwellenlänge im detektierten räumlichen Interferogramm, bezogen auf den ausgeleuchteten Pupillendurchmesser des Objektivs am Interferometerausgang (62, 91, 9) ist;
oder
(iii) wobei die Anordnung zur robusten One-shot-Interferometrie ferner ein, dem Endreflektor zugeordnetes Referenzobjektiv (4, 42) umfasst, das im Referenzraum angeordnet ist, oder wobei das das Objekt abbildende Objektiv ein Objektiv ist (61, 62, 63, 64, 91), das sowohl dem Referenz- als auch dem Objektarm zugeordnet und außerhalb des Referenzarms (R) angeordnet ist;
der Endreflektor ein Hybrid-Retro-Referenz-Endreflektor (46, 461, 499) ist, wobei der Hybrid-Retro-Referenz-Endreflektor entweder als ein Referenz-Mikro-Endreflektor-Array (564, 565) mit Hybrid-Retro-Referenz-Mikro-Endreflektoren vom DachkantSpiegel-Typ oder Dachkant-Prismen-Typ oder als ein Referenz-Mikro--Endreflektor-Array (493, 494) mit Hybrid-Retro-Referenz-Mikro- Endreflektoren vom Zylinder-Linsen-Cat'eye-Typ (483) oder vom Zylinder-Spiegel-Cat'eye-Typ (484) ausgebildet ist, und
ein Querversatz (delta_q) der Hybrid-Retro-Referenz-Mikro-Endreflektoren vom Zylinder-Cat'eye-Typ (483, 484) oder vom Dachkant-Typ im Referenz-Mikro-Endreflektor-Array (564, 565) entweder in der Brennebene (BER) des dem Hybrid-Retro-Referenz-Endreflektor (45, 46) zugeordneten, im Referenzarm (R) angeordneten Referenzobjektivs (4, 42) oder in der Brennebene (BER) des außerhalb des Referenzarms (R) angeordneten und sowohl dem Referenz- als auch dem Objektarm zugeordneten Objektivs (61, 62, 63, 64, 91) besteht, und
der Querversatz (delta_q) zumindest näherungsweise parallel zur Richtung der Quer-Achsen-Richtung (q) ist und maximal gleich dem zehnten Teil der Brennweite dieses Referenzobjektivs (4, 42) oder des außerhalb des Referenzarms angeordneten Objektivs (61, 62, 63, 64, 91) und mindestens gleich oder größer als der objektivbezogene Airy-Scheibchen-Radius für die Schwerpunktwellenlänge im detektierten räumlichen Interferogramm ist.

2. Anordnung zur robusten One-shot-Interferometrie umfassend
eine Lichtquelle (1, 101, 102, 103, 191; 192, 193, 194, 195, 196, 198, 199, LS),
einen gerasterten Detektor (13) mit Empfängerelementen für elektromagnetische Strahlung, und
ein Zweistrahl-Interferometer mit Referenz- und Objektarm und mit den folgenden Komponenten:
ein der Lichtquelle nachfolgendes bzw. nachgeordnetes Lichtquellen-Objektiv (2, 21) zur zumindest näherungsweise beugungsbegrenzten Abbildung derselben, und
ein das Objekt (71, 72, 73, 74) abbildendes Objektiv (6, 61, 62, 63, 64, 66, 91),
wobei die Lichtquelle mindestens eine Punkt-Lichtquelle (198) oder eine Punkt-Lichtquellen-Matrix (199) mit einer Vielzahl von Spots ist, wobei die Punkt-Lichtquelle (198) oder die Spots der Punkt-Lichtquellen-Matrix (199) jeweils so fein ausgebildet ist bzw. sind, dass diese für das nachfolgende Lichtquellen-Objektiv (2, 21) zumindest näherungsweise gleich oder unterhalb dessen beugungsbegrenzter Lateral-Auflösung ist bzw. sind, wobei die Lateral-Auflösung durch numerische Apertur des Lichtquellen-Objektivs (2, 21) und der eingesetzten Lichtwellenlänge oder der eingesetzten Lichtwellenlängen der Lichtquelle (198) oder der Punkt-Lichtquellen-Matrix (199) bestimmt ist,
wobei im Referenzarm des Zweistrahl-Interferometers ein als Retroreflektor ausgebildeter Endreflektor angeordnet ist, welcher ausgelegt ist, eine oder mehrere verkippte Referenz-Wellenfronten zu erzeugen und das Zweistrahl-Interferometer derart ausgelegt ist, zumindest ein Paar zueinander gekippter Referenz- und Objektwellenfronten zu erzeugen, und zur Interferenz zu bringen, und
der Endreflektor als ein Voll-Retro-Referenz-Mikro-Endreflektor-System (471, 498) mit einem Referenzobjektiv (4, 42) und mit Voll-Retro-Referenz-Mikro- Endreflektoren (481, 482, 567) in jeweils
- einem Mikro-Endreflektor-Array (491) mit Voll-Retro- Referenz-Mikro-Endreflektoren vom rotationssymmetrischen Linsen-Cat' eye-Typ (481); oder
- einem Mikro-Endreflektor-Array (492) mit Voll-Retro- Referenz-Mikro-Endreflektoren vom rotationssymmetrischen Spiegel-Cat'eye-Typ (482); oder
- einem Mikro-Tripelprismenspiegel-Endreflektor-Array (568) mit Mikro-Tripelprismenspiegel-Endreflektoren; oder
- einem Mikro-Hohl-Tripelspiegel-Endreflektor-Array (566) mit Mikro-Tripelspiegel-Endreflektoren (567)
ausgebildet ist, und
bei den Voll-Retro- Referenz-Mikro-Endreflektoren (481, 482, 567) jeweils ein Querversatz delta_q in der Brennebene des zugeordneten Referenzobjektivs (4, 42) besteht, der maximal gleich dem zehnten Teil der Brennweite des Referenzobjektivs (4, 42) und mindestens gleich oder größer als der objektivbezogene Airy-Scheibchen-Radius für die Schwerpunktwellenlänge im detektierten räumlichen Interferogramm ist.

3. Anordnung gemäß einem der vorangegangenen Ansprüche, umfassend ferner optische Mittel (12, 121, 81, 82, 83) zur Einführung von Astigmatismus in den Detektionsstrahlengang , welche dem das Objekt abbildenden Objektiv (6, 61, 62, 63, 64, 66, 91) zugeordnet oder nachgeordnet sind und welche dem gerasterten Detektor (13) vorgeordnet sind, wobei die Wellenfronten, die zur Interferenz gebracht werden, zumindest näherungsweise eine Zylinderform aufweisen und durch die optische Mittel (12, 121, 81, 82, 83) zur Einführung von Astigmatismus in den Detektionsstrahlengang gebildet sind.

4. Anordnung zur robusten One-shot-Interferometrie nach Anspruch 3, wobei
die Richtung der Längsachsen der Zylinder-Wellenfronten jeweils zumindest näherungsweise mit der Quer-Achsen-Richtung (q) im entfalteten Strahlengang des Zweistrahl-Interferometers übereinstimmend gemacht ist, und die Kippachse der mindestens einen Referenz Wellenfront jeweils zumindest näherungsweise senkrecht zur Quer-Achsen-Richtung (q) angeordnet ist; und/oder
die Lichtquelle gerastert ist, und wobei die Rasterkonstante von gerasterter Lichtquelle (191, 195, 197, 199) und gerasterten Referenz-Endreflektor-Arrays (491, 492, 493, 494, 564, 565, 566, 568) bezogen auf die Brennebene des Tubusobjektivs (9) oder auf die Brennebene des objektabbildenden Objektivs (6, 61, 62, 63, 64, 66, 67), zumindest näherungsweise gleich ist.

5. Verfahren zur robusten One-shot-Interferometrie umfassend:
Bereitstellen einer Anordnung zur robusten One-Shot-Interferometrie nach einem der Ansprüche 1 bis 4,
Einführen in der einem Detektorobjektiv (82, 91) vorgeordneten Brennebene (F_91_r, F_9') eines Querversatzes (2 delta_q_strich, 2 delta_q) von zueinander kohärenten Lichtspots bzw. Foki bzw. Bildpunkte (A_r", A_o") aus dem Referenz- (R) und Objektarm (0) und/oder Einführen einer Tiefenseparierung (def_r-o_strich, def_r-o) von zueinander kohärenten Lichtspots bzw. Foki bzw. Bildpunkte (A_r", A_o") aus dem Referenz- (R) und Objektarm (0);
Bilden und Bringen zur Interferenz in der Ebene des gerasterten Detektors (13) zumindest ein Paar Wellenfronten, je eine vom Objektarm (O) und je eine vom Referenzarm (R), wobei die Wellenfronten in dem Paar zueinander gekippt sind und/oder unterschiedlich gekrümmt sind;
Bilden mindestens eines räumlichen Interferogramms (KKI) und Detektieren des Interferogramms (KKI) mittels gerastertem Detektor (13).

6. Verfahren zur robusten One-shot-Interferometrie gemäß Anspruch 5 unter Verwendung einer Vorrichtung gemäß Anspruch 3, wobei die Richtung der Längsachsen der Zylinder-Wellenfronten jeweils zumindest näherungsweise mit der Quer-Achsen-Richtung (q) im entfalteten Strahlengang des Zweistrahl-Interferometers übereinstimmend gemacht ist, und die Kippachse der mindestens einen Referenz-Zylinder-Wellenfront jeweils zumindest näherungsweise senkrecht zur Quer-Achsen-Richtung (q) angeordnet ist.

7. Verfahren zur robusten One-shot-Interferometrie gemäß einem der Ansprüche 5 und 6 , wobei im Schnitt, der die Quer-Achsen-Richtung (q) enthält, die Fourier-Ebene (FEO) des objektabbildenden Objektivs (6, 61, 62, 63, 64, 66, 91) auf den gerasterten Detektor (13) zumindest näherungsweise scharf mit optischen Mitteln (6, 61, 62, 63, 64, 66, 91, 9, 11) abgebildet wird; und im Schnitt senkrecht dazu, der im entfalteten Strahlengang parallel zur Längsrichtung der Linienquelle (1) ist, die Messebene des Objekts (MEO) scharf mit optischen Mitteln (6, 61, 62, 63, 64, 66, 91, 9, 11, 12) auf den gerasterten Detektor (13) abgebildet wird, so dass im Detektionsstrahlengang eine astigmatische Abbildung durchgeführt wird.

8. Verfahren zur robusten One-shot-Interferometrie gemäß einem der Ansprüche 5 bis 7, wobei
bei der Abbildung in einer Zwischenbildebene (ZBE) zumindest für das Objektlicht eine konfokale Diskriminierung mittels optischer Mittel (96, 99) durchgeführt wird; und/oder
bei der Abbildung in einer Zwischenbildebene (ZBE) für das Objektlicht eine Tiefpassfilterung mittels optischer Mittel (96, 99) durchgeführt wird.

9. Verfahren zur robusten One-shot-Interferometrie gemäß Anspruch 5, wobei
die Lichtquelle eine Punkt-Lichtquelle (198) oder eine Punkt-Lichtquellen-Matrix (199) mit einer Vielzahl von Spots ist; wobei die Punkt-Lichtquelle (198) oder die Spots der Punkt-Lichtquellen-Matrix (199) jeweils so fein ausgebildet sind, dass diese für das nachfolgende Lichtquellen-Objektiv (2, 21) zumindest näherungsweise gleich oder unterhalb dessen beugungsbegrenzter Lateral-Auflösung ist bzw. sind, wobei Lateral-Auflösung durch numerische Apertur des Lichtquellen-Objektivs (2, 21) und der eingesetzten Lichtwellenlänge oder der eingesetzten Lichtwellenlängen der fein ausgebildeten Lichtquelle (198) oder der fein ausgebildeten Punkt- Lichtquellen-Matrix (199) bestimmt ist, und wobei das Verfahren umfasst:
Beleuchten von Objektpunkten des Objekts, wobei
bei optisch rauen Objekten die Objektpunkten mit zumindest näherungsweise beugungsbegrenzt scharf fokussierten Bildpunkten der Punkt-Lichtquelle (198) oder der Punkt-Lichtquellen-Matrix (199) beleuchtet werden; und
bei optisch glatten Objekten mit schwach defokussierten Bildpunkten der Punkt-Lichtquelle (198) oder der Punkt-Lichtquellen-Matrix (199) beleuchtet werden;
Durchführen einer geometrisch-optisch unscharfen Abbildung der beleuchteten Objektpunkte auf den Detektor (13) nach einer Strahlvereinigung, wobei Licht von jedem Objektpunkt einen Teilbereich mit einer Vielzahl von Detektorelementen des gerasterten Detektors (13) beleuchtet, und
Durchführen im Referenzarm (R) einer geometrisch-optisch unscharfen Abbildung von Punkten der Punkt-Lichtquelle (198) oder der Punkt-Lichtquellen-Matrix (199), wobei Licht von jedem leuchtenden Punkt der Punkt-Lichtquelle (198) oder der Punkt-Lichtquellen-Matrix (199) nach der Strahlvereinigung einen Teilbereich mit einer Vielzahl von Elementen des gerasterten Detektors (13) beleuchtet; und wobei die Teilbereichsflächen auf dem gerasterten Detektor (13) von zueinander kohärenten Strahlenbündeln aus dem Objektarm (0) und dem Referenzarm (R) zumindest näherungsweise zueinander zur Überdeckung gebracht werden;
Bilden mindestens eines räumlichen Interferogramms von kohärenter Strahlung aus dem Referenzarm (R) und dem Objektarm (0), wobei das Interferenzgebiet mindestens 30% der maximalen Ausdehnung der beiden Teilbereichsflächen beträgt, wobei
am Ausgang des Zweistrahl-Interferometers im Raum nach einem Tubusobjektiv (9) oder einem zumindest näherungsweise fokussierenden Objektiv (62,91) oder einem zumindest näherungsweise fokussierenden Objektiv (2) aus dem Beleuchtungsstrahlengang, welches auch zur Detektion mitbenutzt wird, sowohl Bildpunkte (A_r' ') von der Punkt-Lichtquelle (198) oder der Punkt-Lichtquellen-Matrix (199), die über den Referenzarm (R) abgebildet werden, als auch Bildpunkte (A_o") von der Punkt-Lichtquelle (198) oder der Punkt-Lichtquellen-Matrix (199), die über Objektarm (0) abgebildet werden, zumindest in der Tiefe voneinander separiert gebildet werden, wobei:
das Minimum die Tiefenseparierung (def_r-o) den Betrag aufweist:
Halbe Schwerpunktwellenlänge (lambda_S) im detektierten Signal vom räumlichen Interferogramm dividiert durch das Quadrat der effektiv genutzten numerischen Apertur des Tubusobjektivs (9), oder des zumindest näherungsweise fokussierenden Objektivs (62, 91) - oder des zumindest näherungsweise fokussierenden Objektivs (2) aus dem
Beleuchtungsstrahlengang, welches auch zur Detektion mitbenutzt wird,
und der Betrag des Maximums der Tiefenseparierung (def_r-o) gleich der Brennweite des Tubusobjektivs (9) oder des zumindest näherungsweise fokussierenden Objektivs (62, 91) oder des zumindest näherungsweise fokussierenden Objektivs (2) aus dem Beleuchtungsstrahlengang, welches auch zur Detektion mitbenutzt wird, ist.

10. Verfahren zur robusten One-shot-Interferometrie gemäß Anspruch 9, wobei
die Tiefenseparierung (def_r-o_strich) von Foki (A_r", A_o") von Licht aus dem Referenzarm (R) und dem Objektarm (0) im Detektionsstrahlengang bei zumindest näherungsweise gleicher optischer Weglänge in den beiden Armen des Zweistrahl-Interferometers entweder durch Nutzung unterschiedlicher Laufwege in unterschiedlich optisch dichten Medien im Referenz- und im Objektarm des Zweistrahl-Interferometers vorbestimmt gegeben ist, oder durch die Ausbildung schwach defokussierter Spots im Referenzarm auf dem Endreflektor vorbestimmt gegeben ist; und/oder
vor der Detektion eine Strahlformung mit Elementen (841) positiver oder negativer Brechkraft eines Arrays (84) durchgeführt wird, dessen Brennebenen zumindest näherungsweise in einer gemeinsamen Ebene liegen und diese Brennebenen mit der Detektionsebene des gerasterten Detektors (13) zusammenfallen oder zu dieser mittels einer optischen Transferstufe optisch konjugiert gemacht sind; und/oder
aus dem sphärischen Anteil im räumlichen Interferogramm die Ringordnungszahl bestimmt wird.

11. Verfahren zur robusten One-shot Interferometrie gemäß einem der Ansprüche 9 oder 10, wobei das Zwei-Strahlinterferometer so abgeglichen wird, dass beim optischen Gangunterschied null ein Interferenzring nullter Ordnung im Wesentlichen in der Mitte des räumlichen Interferogramms entsteht.

## Claims

1. An arrangement for robust one-shot interferometry, comprising
a light source (1, 101, 102, 103, 191; 192, 193, 194, 195, 196, 198, 199, LS), a rastered detector (13) with receiving elements for electromagnetic radiation, and
a two-beam interferometer with reference and object arms and with the following components:
a light source lens (2, 21) following or arranged downstream of the light source for at least approximately diffraction-limited imaging of the same,
a lens (6, 61, 62, 63, 64, 66, 91) imaging the object (71, 72, 73, 74),
wherein an end reflector designed as a retroreflector is arranged in the reference arm of the two-beam interferometer, said end reflector being designed to generate one or more tilted reference wavefronts; and the two-beam interferometer is designed such as to generate at least one pair of reference and object wavefronts tilted relative to one another and cause them to interfere,
wherein the light source is at least one line light source (1, 101, 102, 103, 191, 192, 193, 194, 196, LS) formed to be narrow in at least one transverse axis direction (q) or a line light source array (191, 197) having a plurality of at least approximately parallel line spots formed to be narrow in a transverse axis direction, and
the line light source (1, 101, 102, 103, 192, 193, 194, 195, 196, LS) or the line spots of the line light source array (191, 197) is or are formed to be so narrow in the transverse axis direction (q) that it is or they are at least approximately equal to or below its diffraction-limited lateral resolution for the subsequent light source lens (2, 21) in this transverse axis direction (q), wherein the lateral resolution is determined by the numerical aperture of the light source lens (2, 21) and the light wavelength or the light wavelengths of the line light source (1, 101, 102, 103, 192, 193, 194, 195, 196, LS) or the line light source arrays (191, 197) used, and wherein the light source (1, 101, 102, 103, 192, 193, 194, 195, 196, LS) is imaged onto the object in a diffraction-limited manner in at least one dimension by means of the lens (6, 61, 62, 63, 64, 66, 91) imaging the object, and wherein
(i) the arrangement for robust one-shot interferometry also includes a reference lens (4, 42) assigned to the end reflector and arranged in the reference arm, or wherein the lens imaging the object comprises a lens (6, 61, 62, 63, 64, 66, 91) associated with both the reference and the object arm and arranged outside the reference arm (R);
the end reflector is a hybrid retro reference end reflector (45; 461), i.e. a one-dimensional retro reference end reflector, and the hybrid retro reference end reflector (45; 461) has at least one 90° roof end reflector (5, 56, 561, 562, 57, 58, 581, 59), wherein the roof (DK) of the 90° roof end reflector (5, 56, 561, 562, 57, 58, 581, 59):
is positioned at least approximately in the focal plane of a reference lens (4, 42) arranged in the reference arm or of the lens (61, 62, 63, 64, 91) arranged outside the reference arm (R); and
is arranged at least approximately perpendicular to the transverse axis direction (q) if no focusing reference lens is arranged in the reference arm; or is arranged at least approximately perpendicular to the transverse axis direction (q) if a focusing reference lens is arranged in the reference arm; and
there is a transverse offset (delta_q) of the roof (DK) at least approximately parallel to the direction of the transverse axis direction (q) either in the focal plane (BER) of the reference lens (4, 42) associated with hybrid retro reference end reflector (45) or in the focal plane (BER) of the lens (61, 62, 63, 64, 91) associated with both the reference arm and
the object arm, and the transverse offset delta_q is at most equal to one tenth of the focal length of this lens (4, 41, 42 , 44, 61, 62, 63, 64, 91) and at least equal to or greater than the lens-related Airy disk radius for the centroid wavelength in the detected spatial interferogram;
or
(ii) the end reflector is a hybrid retro reference end reflector which is either designed as a cylinder optics hybrid retro reference end reflector (44) with a cylinder lens (41) with a cylinder axis (CA) and a plane mirror (43) in the focal plane of the cylinder lens (41), wherein the cylinder axis (CA) is respectively designed to be perpendicular to the longitudinal direction of the line light source, or is designed as a roof reflector (5), wherein the roof of the roof reflector (5) is perpendicular to the longitudinal direction of the line light source,
wherein there is a tilt (delta_beta_tilt) of the hybrid retro reference end reflector (44, 5) about a tilt axis that is at least approximately perpendicular to both the transverse axis direction (q) and perpendicular to the direction of light propagation, respectively based on an unfolded beam path of the two-beam interferometer, and
the tilt angle is maximally equal to or smaller than 6 degrees and at least equal to or greater than half the centroid wavelength in the detected spatial interferogram, based on the illuminated pupil diameter of the lens at the interferometer output (62, 91, 9);
or
(iii) wherein the arrangement for robust one-shot interferometry further comprises a reference lens (4, 42) associated with the end reflector and arranged in the reference arm, or wherein the lens imaging the object comprises a lens (6, 61, 62, 63 , 64, 66, 91) associated with both the reference and the object arm and arranged outside the reference arm (R);
the end reflector is a hybrid retro reference end reflector (46, 461, 499), wherein
the hybrid retro reference end reflector is designed either as a micro reference end reflector array (564, 565) with hybrid retro micro reference end reflectors of the roof mirror type or roof prism type or as a micro reference end reflector array (493, 494) with hybrid retro micro reference end reflectors of the cylinder lens cat'eye type (483) or of the cylinder mirror cat'eye type (484), and
there is a transverse offset (delta_q) of the hybrid retro micro reference end reflectors of the cylinder cat' eye type (483, 484) or of the roof type in the micro reference end reflector array (564, 565) in either the focal plane (BER) of the reference lens (4, 42) associated with the hybrid retro reference end reflector (45, 46) and arranged in the reference arm (R) or in the focal plane (BER) of the lens (61, 62, 63, 64, 91) arranged outside the reference arm (R) and associates with both the reference and the object arm, and
the transverse offset (delta_q) is at least approximately parallel to the direction of the transverse axis direction (q) and maximally equal to one tenth of the focal length of this reference lens (4, 42) or of the lens (61, 62, 63, 64, 91) arranged outside the reference arm and is at least equal to or greater than the lens-related Airy disk radius for the centroid wavelength in the detected spatial interferogram.

2. An arrangement for robust one-shot interferometry, comprising
a light source (1, 101, 102, 103, 191; 192, 193, 194, 195, 196, 198, 199, LS),
a rastered detector (13) with receiving elements for electromagnetic radiation, and
a two-beam interferometer with reference and object arms and with the following components:
a light source lens (2, 21) following or arranged downstream of the light source for at least approximately diffraction-limited imaging of the same, and
a lens (6, 61, 62, 63, 64, 66, 91) imaging the object (71, 72, 73, 74), wherein the light source is at least one point light source (198) or a point light source matrix (199) with a plurality of spots, wherein the point light source (198) or the spots of the point light source matrix (199) is or are respectively designed to be so fine that it is or they are at least approximately equal to or below the diffraction-limited lateral resolution for the subsequent light source lens (2, 21), wherein the lateral resolution is determined by the numerical aperture of the light source lens (2, 21) and the light wavelength or the light wavelengths of the light source (198) or the point light source matrix (199) used,
wherein an end reflector designed as a retroreflector is arranged in the reference arm of the two-beam interferometer, said end reflector being designed to generate one or more tilted reference wavefronts, and the two-beam interferometer is designed such as to generate at least one pair of reference and object wavefronts tilted relative to one another and cause them to interfere, and the end reflector is designed as a full retro micro reference end reflector system (471, 498) with a reference lens (4, 42) and with full retro micro reference end reflectors (481, 482, 567) in respectively
- one micro endreflector array (491) with full retro-micro-reference endreflectors of the rotationally symmetric lens cat'eye type (481); or
- one micro endreflector array (492) with full retro-micro-reference endreflectors of the rotationally symmetric mirror cat'eye type (482); or
- one micro triple prism mirror end reflector array (568) with micro- triple prism mirror end reflectors; or
- one micro hollow triple mirror end reflector array (566) with micro triple mirror end reflectors (567)
and
in the full retro micro reference end reflectors (481, 482, 567) there is a transverse offset delta_q in the focal plane of the associated reference lens (4, 42), which is maximally equal to one tenth of the focal length of the reference lens (4, 42) and is at least equal to or greater than the lens-related Airy disk radius for the centroid wavelength in the detected spatial interferogram.

3. The arrangement according to one of the preceding claims, further comprising optical means (12, 121, 81, 82, 83) for introducing astigmatism into the detection beam path, which are associated with or arranged downstream of the lens (6, 61, 62, 63, 64, 66, 91) imaging the object and which are arranged upstream of the rastered detector (13), wherein the wavefronts caused to interfere have at least approximately a cylinder shape and are formed by the optical means (12, 121, 81, 82, 83) for introducing astigmatism into the detection beam path.

4. The arrangement for robust one-shot interferometry according to claim 3, wherein
the direction of the longitudinal axes of the cylindrical wavefronts is made at least approximately in accordance with the transverse axis direction (q) in the unfolded beam path of the two-beam interferometer, and the tilting axis of the at least one reference wavefront is arranged at least approximately perpendicular to the transverse axis direction (q); and/or
the light source is rastered, and wherein the raster constant of rastered light source (191, 195, 197, 199) and rastered reference end reflector arrays (491, 492, 493, 494, 564, 565, 566, 568) based on the focal plane of the tube lens (9) or the focal plane of the lens (6, 61, 62, 63, 64, 66, 67) imaging the object is at least approximately the same.

5. A method for robust one-shot interferometry, comprising:
providing an arrangement for robust one-shot interferometry according to one of claims 1 to 4,
introducing, into the focal plane (F_91_r, F_9') upstream of a detector lens (82, 91), a transverse offset (2 delta_q_strich, 2 delta_q) of mutually coherent light spots or foci or image points (A_r", A_o") from the reference (R) and
object arm (O) and/or introducing a depth separation (def_r-o_strich, def_r-o) of mutually coherent light spots or foci or image points (A_r", A_o") from the reference (R) and object arm (O);
forming and making to interfere, in the plane of the rastered detector (13), at least one pair of wavefronts, one each from the object arm (O) and one each from the reference arm (R), wherein the wavefronts in the pair are tilted with respect to one another and/or are differently curved;
forming at least one spatial interferogram (KKI) and detecting the interferogram (KKI) by means of the rastered detector (13).

6. The method for robust one-shot interferometry according to claim 5 using a device according to claim 3, wherein the direction of the longitudinal axes of the cylindrical wavefronts is made at least approximately in accordance with the transverse axis direction (q) in the unfolded beam path of the two-beam interferometer, and the tilt axis of the at least one reference cylindrical wavefront is arranged at least approximately perpendicular to the transverse axis direction (q).

7. The method for robust one-shot interferometry according to one of claims 5 and 6, wherein
in the section containing the transverse axis direction (q), the Fourier plane (FEO) of the object-imaging lens (6, 61, 62, 63, 64, 66, 91) is imaged at least approximately sharp onto the rastered detector (13) with optical means (6, 61, 62, 63, 64, 66, 91, 9, 11); and in a section perpendicular to it, which is parallel to the longitudinal direction of the line source (1) in the unfolded beam path, the measuring plane of the object (MEO) is imaged sharply onto the rastered detector (13) with optical means (6, 61, 62, 63, 64, 66, 91, 9, 11, 12), so that astigmatic imaging is carried out in the detection beam path.

8. The method for robust one-shot interferometry according to one of claims 5 to 7, wherein
during imaging in an intermediate image plane (ZBE), a confocal discrimination is carried out at least for the object light by means of optical means (96, 99); and/or
during imaging in an intermediate image plane (ZBE), low-pass filtering is carried out for the object light by means of optical means (96, 99).

9. The method for robust one-shot interferometry according to claim 5, wherein the light source is a point light source (198) or a point light source matrix (199) with a plurality of spots; wherein the point light source (198) or the spots of the point light source matrix (199) is or are respectively designed to be so fine that it is or they are at least approximately equal to or below the diffraction-limited lateral resolution for the subsequent light source lens (2, 21), wherein the lateral resolution is determined by the numerical aperture of the light source lens (2, 21) and the light wavelength or the light wavelengths of the finely designed light source (198) or the finely designed point light source matrix (199) used, and wherein the method comprises:
illuminating object points of the object, wherein
in the case of optically rough objects, the object points are illuminated with at least approximately diffraction-limited sharply focused image points of the point light source (198) or the point light source matrix (199); and
in the case of optically smooth objects, are illuminated with weakly defocused image points of the point light source (198) or the point light source matrix (199);
carrying out a geometrically-optically blurred imaging of the illuminated object points to the detector (13) after beam convergence, wherein light from each object point illuminates a partial area with a plurality of detector elements of the rastered detector (13), and
carrying out, in the reference arm (R), a geometrically-optically blurred imaging of points of the point light source (198) or the point light source matrix (199), wherein light from each luminous point of the point light source (198) or the point light source matrix (199) illuminates a partial area with a plurality of elements of the rastered detector (13); and wherein the partial area surfaces on the rastered detector (13) are brought to overlap at least approximately with one another by mutually coherent beams of rays from the object arm (O) and the reference arm (R);
forming at least one spatial interferogram of coherent radiation from the reference arm (R) and the object arm (O), wherein the interference area is at least 30% of the maximum extent of the two partial areas, wherein
at the output of the two-beam interferometer in space after a tube lens (9) or an at least approximately focusing lens (62, 91) or an at least approximately focusing lens (2) from the illumination beam path, which is also used for detection, both image points (A_r") from the point light source (198) or
the point light source matrix (199), which are imaged via the reference arm (R), as well as image points (A_o") from the point light source (198) or the point light source matrix (199), which are imaged via the object arm (O), are formed separately from one another at least in depth, wherein:
the minimum the depth separation (def_r-o) has the amount:
half the centroid wavelength (lambda_S) in the detected signal from the spatial interferogram divided by the square of the effectively used numerical aperture of the tube lens (9), or the at least approximately focusing lens (62, 91) - or the at least approximately focusing lens (2) from the illumination beam path, which is also used for detection,
and the amount of the maximum of the depth separation (def_r-o) equal to the focal length of the tube lens (9) or the at least approximately focusing lens (62, 91) or the at least approximately focusing lens (2) from the illumination beam path, which is also used for detection.

10. The method for robust one-shot interferometry according to claim 9, wherein
the depth separation (def_r-o_strich) of foci (A_r", A_o") of light from the reference arm (R) and the object arm (O) in the detection beam path with at least approximately the same optical path length in the two arms of the two-beam interferometer is given in a predetermined manner either by use of different paths in media of different optical densities in the reference and in the object arm of the two-beam interferometer, or is given in a predetermined manner by the formation of weakly defocused spots in the reference arm on the end reflector; and/or
prior to detection, beam shaping is carried out with elements (841) of positive or negative refractive power of an array (84) whose focal planes lie at least approximately in a common plane and these focal planes coincide with the detection plane of the rastered detector (13) or are made optically conjugate with respect to it by means of an optical transfer stage; and/or
the ring order number is determined from the spherical proportion in the spatial interferogram.

11. The method for robust one-shot interferometry according to claim 9 or 10, wherein the two-beam interferometer is adjusted such that with an optical path difference of zero an interference ring of zero order arises substantially in the center of the spatial interferogram.

## Revendications

1. Agencement d'interférométrie robuste à interférogramme unique comprenant une source lumineuse (1, 101, 102, 103, 191 ; 192, 193, 194, 195, 196, 198, 199, LS),
un détecteur tramé (13) avec des éléments récepteurs pour rayonnement électromagnétique, et
un interféromètre à deux ondes avec bras de référence et d'objet et avec les composants suivants :
un objectif de source lumineuse (2, 21) suivant ou disposé en aval de la source lumineuse pour la représentation au moins approximativement limitée par diffraction de celle-ci,
un objectif (6, 61, 62, 63, 64, 66, 91) représentant l'objet (71, 72, 73, 74), dans lequel un réflecteur terminal réalisé en tant que rétro-réflecteur est disposé dans le bras de référence de l'interféromètre à deux ondes, lequel est conçu pour générer un ou plusieurs fronts d'onde de référence à défaut d'alignement angulaire ; et l'interféromètre à deux ondes est conçu pour générer au moins une paire de fronts d'onde de référence et d'objet basculés l'un par rapport à l'autre,
et pour amener à l'interférence,
dans lequel la source lumineuse est au moins une source lumineuse linéaire (1, 101, 102, 103, 191, 192, 193, 194, 196, LS) réalisée de manière étroite dans au moins une direction d'axes transversaux (q) ou un réseau de sources lumineuses linéaires (191, 197) avec une pluralité de spots linéaires au moins approximativement parallèles, formés de manière étroite dans une direction d'axes transversaux, et
la source lumineuse linéaire (1, 101, 102, 103, 191, 192, 193, 194, 196, LS) ou
les spots linéaires du réseau de sources lumineuses linéaires (191, 197) est ou
sont réalisée/réalisés chacun dans la direction d'axes transversaux (q) de manière si étroite que celle/ceux-ci est ou sont au moins approximativement égale/égaux ou en dessous de sa/leur résolution latérale limitée par diffraction pour l'objectif de source lumineuse suivant (2 ,21) dans cette direction d'axes transversaux (q), dans lequel la résolution latérale est déterminée par l'ouverture numérique de l'objectif de source lumineuse (2, 21) et de la longueur d'onde lumineuse utilisée ou des longueurs d'onde lumineuses utilisées de la source lumineuse linéaire (1, 101, 102, 103, 191, 192, 193, 194, 196, LS) ou du réseau de sources lumineuses linéaires (191, 197), et dans lequel la source lumineuse (1, 101, 102, 103, 191, 192, 193, 194, 196, LS) est représentée sur l'objet de manière limitée par diffraction au moyen de l'objectif (6, 61, 62, 63, 64, 66, 91) représentant l'objet dans au moins une dimension, et
dans lequel
(i) l'agencement d'interférométrie robuste à interférogramme unique contient en outre un objectif de référence (4, 42) associé au réflecteur terminal qui est disposé dans l'espace de référence, ou dans lequel l'objectif représentant l'objet est un objectif (61, 62, 63, 64, 91) qui est associé aussi bien au bras de référence qu'au bras d'objet et disposé en dehors du bras de référence (R) ;
le réflecteur terminal est un rétro-réflecteur terminal hybride de référence (45 ; 461), c'est-à-dire un rétro-réflecteur terminal de référence unidimensionnel, et le rétro-réflecteur terminal hybride de référence (45 ; 461) présente au moins un réflecteur terminal à arête de toit à 90° (5, 56, 561, 562, 57, 58, 581, 59), dans lequel l'arête de toit (DK) du réflecteur terminal à arête de toit à 90° (5, 56, 561, 562, 57, 58, 581, 59) :
est positionnée au moins approximativement dans le plan focal de l'objectif de référence (4, 42) disposé dans le bras de référence ou de l'objectif (61, 62, 63, 64, 91) disposé en dehors du bras de référence (R) ; et
est disposée au moins approximativement perpendiculairement à la direction d'axes transversaux (q) lorsqu'aucun objectif de référence focalisant n'est disposé dans le bras de référence ; ou est disposée au moins approximativement perpendiculairement à la direction d'axes transversaux (q) lorsqu'un objectif de référence focalisant est disposé dans le bras de référence ; et
un décalage transversal (delta_q) de l'arête de toit (DK) existe au moins approximativement parallèlement à la direction d'axes transversaux (q) soit dans le plan focal (BER) de l'objectif de référence (4, 42) associé au rétro-réflecteur terminal hybride de référence (45), soit dans le plan focal (BER) de l'objectif (61, 62, 63, 64, 91) associé aussi bien au bras de référence qu'au bras d'objet, et le décalage transversal delta q est au maximum égal au dixième de la distance focale de cet objectif (4, 41, 42, 44, 61, 62, 63, 64, 91) et au minimum supérieur ou égal au rayon de disque d'Airy lié à l'objectif pour la longueur d'onde d'intensité dans l'interférogramme spatial détecté ;
ou
(ii) le réflecteur terminal est un rétro-réflecteur terminal hybride de référence qui soit est réalisé en tant que rétro-réflecteur terminal hybride de référence optique cylindrique (44) avec un objectif cylindrique (41) avec un axe cylindrique (CA) et un miroir plan (43) dans le plan focal de l'objectif cylindrique (41), dans lequel l'axe cylindrique (CA) est à chaque fois perpendiculaire à la direction longitudinale de la source lumineuse linéaire, soit est réalisé en tant que réflecteur à arête de toit (5), dans lequel l'arête de toit du réflecteur à arête de toit (5) est perpendiculaire à la direction longitudinale de la source lumineuse linéaire,
dans lequel un basculement (delta_beta_tilt) du rétro-réflecteur terminal hybride de référence (44, 5) autour d'un axe de basculement existe, lequel est au moins approximativement aussi bien perpendiculaire à la direction d'axes transversaux (q) que perpendiculaire à la direction de la propagation lumineuse, à chaque fois par rapport à une trajectoire du faisceau déployée de l'interféromètre à deux ondes, et
l'angle de basculement est au maximum inférieur ou égal à 6 degrés et au moins supérieur ou égal à une demi-longueur d'onde d'intensité dans l'interférogramme spatial détecté, par rapport au diamètre de pupille éclairé de l'objectif à la sortie de l'interféromètre (62, 91, 9) ;
ou
(iii) dans lequel l'agencement d'interférométrie robuste à interférogramme unique contient en outre un objectif de référence (4, 42) associé au réflecteur terminal qui est disposé dans l'espace de référence, ou dans lequel l'objectif représentant l'objet est un objectif (61, 62, 63, 64, 91) qui est associé aussi bien au bras de référence qu'au bras d'objet et disposé en dehors du bras de référence (R) ;
le réflecteur terminal est un rétro-réflecteur terminal hybride de référence (46, 461, 499), dans lequel
le rétro-réflecteur terminal hybride de référence est réalisé soit en tant que réseau de micro-réflecteurs terminaux de référence (564, 565) avec des micro-rétro-réflecteurs terminaux hybrides de référence du type à miroir triangulaire ou du type à prisme triangulaire, soit en tant que réseau de micro-réflecteurs terminaux de référence (493, 494) avec des micro-rétro-réflecteurs terminaux hybrides de référence du type Cat'eye à lentille cylindrique (483) ou du type Cat'eye à miroir cylindrique (484), et
un décalage transversal (delta_q) des micro-rétro-réflecteurs terminaux hybrides de référence du type Cat'eye cylindrique (483, 484) ou du type à arête de toit dans le réseau de micro-réflecteurs terminaux de référence (564, 565) existe soit dans le plan focal (BER) de l'objectif de référence (4, 42) disposé dans le bras de référence (R), associé au rétro-réflecteur terminal hybride de référence (45, 46), soit dans le plan focal (BER) de l'objectif (61, 62, 63, 64, 91) disposé en dehors du bras de référence (R) et associé aussi bien au bras de référence qu'au bras d'objet, et
le décalage transversal delta_q est au moins approximativement parallèle à la direction d'axes transversaux (q) et au maximum égal au dixième de la distance focale de cet objectif de référence (4, 42) ou de l'objectif (61, 62, 63, 64, 91) disposé en dehors du bras de référence et au moins supérieur ou égal au rayon de disque d'Airy lié à l'objectif pour la longueur d'onde d'intensité dans l'interférogramme spatial détecté.

2. Agencement d'interférométrie robuste à interférogramme unique comprenant une source lumineuse (1, 101, 102, 103, 191 ; 192, 193, 194, 195, 196, 198, 199, LS),
un détecteur tramé (13) avec des éléments récepteurs pour rayonnement électromagnétique, et
un interféromètre à deux ondes avec bras de référence et d'objet et avec les composants suivants :
un objectif de source lumineuse (2, 21) suivant ou disposé en aval de la source lumineuse pour la représentation au moins approximativement limitée par diffraction de celle-ci, et
un objectif (6, 61, 62, 63, 64, 66, 91) représentant l'objet (71, 72, 73, 74), dans lequel la source lumineuse est au moins une source lumineuse ponctuelle (198) ou une matrice de sources lumineuses ponctuelles (199) avec une pluralité de spots, dans lequel la source lumineuse ponctuelle (198) ou les spots de la matrice de sources lumineuses ponctuelles (199) est ou sont réalisée/réalisés à chaque fois de manière si fine que celle/ceux-ci est ou sont pour l'objectif de source lumineuse suivant (2, 21) au moins approximativement égale/égaux à ou
en dessous de sa/leur résolution latérale limitée par diffraction, dans lequel la résolution latérale est déterminée par l'ouverture numérique de l'objectif de source lumineuse (2, 21) et de la longueur d'onde lumineuse utilisée ou des longueurs d'onde lumineuses utilisées de la source lumineuse (198) ou de la matrice de sources lumineuses ponctuelles (199),
dans lequel un réflecteur terminal réalisé en tant que rétro-réflecteur est disposé dans le bras de référence de l'interféromètre à deux ondes, lequel est conçu pour générer un ou plusieurs fronts d'onde de référence à défaut d'alignement angulaire et l'interféromètre à deux ondes est conçu pour générer au moins une paire de fronts d'onde de référence et d'objet basculés l'un par rapport à l'autre,
et pour amener à l'interférence, et
le réflecteur terminal est réalisé en tant que système de micro-rétro-réflecteurs terminaux de référence entiers (471, 498) avec un objectif de référence (4, 42) et avec des micro-rétro-réflecteurs terminaux de référence entiers (481, 482, 567) à chaque fois dans
- un réseau de micro-réflecteurs terminaux (491) avec des micro-rétro-réflecteurs terminaux de référence entiers du type Cat'eye à lentille à symétrie de rotation (481) ; ou
- un réseau de micro-réflecteurs terminaux (492) avec des micro-rétro-réflecteurs terminaux de référence entiers du type Cat'eye à miroir à symétrie de rotation (482) ; ou
- un réseau de micro-réflecteurs terminaux à miroir prismatique triple (568) avec des micro-réflecteurs terminaux à miroir prismatique triple ; ou
- un réseau de micro-réflecteurs terminaux à miroir triple creux (566) avec des micro-réflecteurs terminaux à miroir triple (567),
et
dans le cas des micro-rétro-réflecteurs terminaux de référence entiers (481, 482, 567), un décalage transversal delta q existe à chaque fois dans le plan focal de l'objectif de référence associé (4, 42), lequel est au maximum égal au dixième de la distance focale de l'objectif de référence (4, 42) et au moins supérieur ou
égal au rayon de disque d'Airy lié à l'objectif pour la longueur d'onde d'intensité dans l'interférogramme spatial détecté.

3. Agencement selon une des revendications précédentes, comprenant en outre des moyens optiques (12, 121, 81, 82, 83) pour l'introduction d'astigmatisme dans la trajectoire du faisceau de détection, qui sont associés ou disposés en aval de l'objectif représentant l'objet (6, 61, 62, 63, 64, 66, 91) et qui sont disposés en amont du détecteur tramé (13), dans lequel les fronts d'onde qui ont amené à l'interférence présentent au moins approximativement une forme cylindrique et sont formés par les moyens optiques (12, 121, 81, 82, 83) pour l'introduction d'astigmatisme dans la trajectoire du faisceau de détection.

4. Agencement d'interférométrie robuste à interférogramme unique selon la revendication 3, dans lequel
la direction des axes longitudinaux des fronts d'onde cylindriques est rendue concordante à chaque fois au moins approximativement avec la direction d'axes transversaux (q) dans la trajectoire du faisceau déployée de l'interféromètre à deux ondes, et l'axe de basculement de l'au moins un front d'onde de référence est disposé à chaque fois au moins approximativement perpendiculairement à la direction d'axes transversaux (q) ; et/ou
la source lumineuse est tramée, et dans lequel le pas du réseau de la source lumineuse tramée (191, 195, 197, 199) et des réseaux de réflecteurs terminaux de référence tramés (491, 492, 493, 494, 564, 565, 566, 568) par rapport au plan focal de l'objectif à barillet (9) ou au plan focal de l'objectif représentant l'objet (6, 61, 62, 63, 64, 66, 67) est au moins approximativement égal.

5. Procédé d'interférométrie robuste à interférogramme unique comprenant :
mise à disposition d'un agencement d'interférométrie robuste à interférogramme unique selon une des revendications 1 à 4,
introduction dans le plan focal (F_91_r, F-9') disposé en amont d'un objectif de détecteur (82, 91) d'un décalage latéral (2 delta_q_strich, 2 delta_q) de points lumineux ou foyers ou points d'image (A_r", A_o") cohérents l'un par rapport à l'autre provenant du bras de référence (R) et d'objet (O) et/ou introduction d'une séparation de profondeur (def_r-o_strich, def_r-o) de points lumineux ou
foyers ou points d'image (A_r", A_o") cohérents l'un par rapport à l'autre provenant du bras de référence (R) et d'objet (O) ;
formation et amenée à l'interférence dans le plan du détecteur tramé (13) d'au moins une paire de fronts d'onde, à chaque fois une par le bras d'objet (O) et à chaque fois une par le bras de référence (R), dans lequel les fronts d'onde dans la paire sont basculés l'un par rapport à l'autre et/ou sont courbés différemment ;
formation d'au moins un interférogramme spatial (KKI) et détection de l'interférogramme (KKI) au moyen du détecteur tramé (13).

6. Procédé d'interférométrie robuste à interférogramme unique selon la revendication 5 avec utilisation d'un dispositif selon la revendication 3,
dans lequel la direction des axes longitudinaux des fronts d'onde cylindriques est rendue concordante à chaque fois au moins approximativement avec la direction d'axes transversaux (q) dans la trajectoire du faisceau déployée de l'interféromètre à deux ondes, et l'axe de basculement de l'au moins un front d'onde cylindrique de référence est disposé à chaque fois au moins approximativement perpendiculairement à la direction d'axes transversaux (q).

7. Procédé d'interférométrie robuste à interférogramme unique selon une des revendications 5 et 6, dans lequel dans la coupe qui contient la direction d'axes transversaux (q), le plan de Fourier (FEO) de l'objectif représentant l'objet (6, 61, 62, 63, 64, 66, 91) est représenté sur le détecteur tramé (13) au moins approximativement de manière nette avec des moyens optiques (6, 61, 62, 63, 64, 66, 91, 9, 11) ; et dans la coupe perpendiculaire à celle-ci qui est parallèle à la direction longitudinale de la source linéaire (1) dans la trajectoire du faisceau déployée, le plan de mesure de l'objet (MEO) est représenté de manière nette avec des moyens optiques (6, 61, 62, 63, 64, 66, 91, 9, 11, 12) sur le détecteur tramé (13) de sorte qu'une représentation astigmate est réalisée dans la trajectoire du faisceau de détection.

8. Procédé d'interférométrie robuste à interférogramme unique selon une des revendications 5 à 7, dans lequel
lors de la représentation dans un plan d'image intermédiaire (ZBE) au moins pour la lumière d'objet, une discrimination confocale est réalisée au moyen de moyens optiques (96, 99) ; et/ou
lors de la représentation dans un plan d'image intermédiaire (ZBE) pour la lumière d'objet, une filtration passe-bas est réalisée au moyen de moyens optiques (96, 99).

9. Procédé d'interférométrie robuste à interférogramme unique selon la revendication 5, dans lequel
la source lumineuse est une source lumineuse ponctuelle (198) ou une matrice de sources lumineuses ponctuelles (199) avec une pluralité de spots ; dans lequel
la source lumineuse ponctuelle (198) ou les spots de la matrice de sources lumineuses ponctuelles (199) sont réalisés à chaque fois de manière si fine que celle/ceux-ci est ou sont pour l'objectif de source lumineuse suivant (2, 21) au moins approximativement égale/égaux à ou en dessous de sa/leur résolution latérale limitée par diffraction, dans lequel la résolution latérale est déterminée par l'ouverture numérique de l'objectif de source lumineuse (2, 21) et de la longueur d'onde lumineuse utilisée ou des longueurs d'onde lumineuses utilisées de la source lumineuse réalisée finement (198) ou de la matrice de sources lumineuses ponctuelles réalisée finement (199), et dans lequel le procédé comprend :
éclairage de points de l'objet, dans lequel
dans le cas d'objets rugueux optiquement, les points d'objet sont éclairés avec des points d'image focalisés de manière nette au moins approximativement de manière limitée par diffraction de la source lumineuse ponctuelle (198) ou de la matrice de sources lumineuses ponctuelles (199) ; et
dans le cas d'objets lisses optiquement, sont éclairés avec des points d'objet défocalisés de manière faible de la source lumineuse ponctuelle (198) ou de la matrice de sources lumineuses ponctuelles (199) ;
réalisation d'une représentation floue géométriquement-optiquement des points d'objet éclairés sur le détecteur (13) après une réunion de rayons, dans lequel de la lumière de chaque point d'objet éclaire une région partielle avec une pluralité d'éléments de détecteur du détecteur tramé (13), et
réalisation dans le bras de référence (R) d'une représentation floue géométriquement-optiquement de points de la source lumineuse ponctuelle (198) ou de la matrice de sources lumineuses ponctuelles (199), dans lequel de la lumière de chaque point éclairant de la source lumineuse ponctuelle (198) ou
de la matrice de sources lumineuses ponctuelles (199) éclaire après la réunion de rayons une région partielle avec une pluralité d'éléments du détecteur tramé (13) ; et dans lequel les faces de région partielle sur le détecteur tramé (13) sont amenées à la superposition au moins approximativement l'une par rapport à l'autre par des faisceaux de rayons cohérents l'un par rapport à l'autre provenant du bras d'objet (O) et du bras de référence (R) ;
formation d'au moins un interférogramme spatial de rayonnement cohérent provenant du bras de référence (R) et du bras d'objet (O), dans lequel le domaine d'interférence se monte à au moins 30 % de l'étendue maximale des deux faces de région partielle, dans lequel
aussi bien des points d'image (A_r") de la source lumineuse ponctuelle (198) ou
de la matrice de sources lumineuses ponctuelles (199) qui sont représentés au-dessus du bras de référence (R), que des points d'image (A_o") de la source lumineuse ponctuelle (198) ou de la matrice de sources lumineuses ponctuelles (199) qui sont représentés au-dessus du bras d'objet (O), sont formés de manière séparée l'un de l'autre au moins en profondeur à la sortie de l'interféromètre à deux ondes dans l'espace après un objectif à barillet (9) ou un objectif au moins approximativement focalisant (62, 91) ou un objectif au moins approximativement focalisant (2) provenant de la trajectoire du faisceau d'éclairage qui est également utilisée pour la détection, dans lequel :
le minimum de la séparation de profondeur (def_r-o) présente le montant :
demi-longueur d'onde d'intensité (lambda_S) dans le signal détecté par l'interférogramme spatial divisée par le carré de l'ouverture numérique utilisée réellement de l'objectif à barillet (9), ou de l'objectif au moins approximativement focalisant (62, 91), ou de l'objectif au moins approximativement focalisant (2) provenant de la trajectoire du faisceau d'éclairage qui est également utilisée pour la détection,
et le montant du maximum de la séparation de profondeur (def_r-o) est égal à la distance focale de l'objectif à barillet (9) ou de l'objectif au moins approximativement focalisant (62, 91) ou de l'objectif au moins approximativement focalisant (2) provenant de la trajectoire du faisceau d'éclairage qui est également utilisée pour la détection.

10. Procédé d'interférométrie robuste à interférogramme unique selon la revendication 9, dans lequel
la séparation de profondeur (def_r-o_strich) de foyers (A_r", A_o") de lumière provenant du bras de référence (R) et du bras d'objet (O) dans la trajectoire du faisceau de détection dans le cas de trajet optique au moins approximativement égal dans les deux bras de l'interféromètre à deux ondes est donnée de manière prédéterminée soit par l'utilisation de différents parcours dans des milieux à densité optique différente dans le bras de référence et d'objet de l'interféromètre à deux ondes, soit est donnée de manière prédéterminée par la représentation de spots défocalisés de manière faible dans le bras de référence sur le réflecteur terminal ; et/ou
une formation de rayons avec des éléments (841) de pouvoir réfringent positif ou négatif d'un réseau (84) dont les plans focaux se situent au moins approximativement dans un plan commun est réalisée avant la détection, et ces plans focaux coïncident avec le plan de détection du détecteur tramé (13) ou sont rendus conjugués optiquement à celui-ci au moyen d'une étape de transfert optique ; et/ou
le nombre ordinal d'anneaux est déterminé à partir de la portion sphérique dans l'interférogramme spatial.

11. Procédé d'interférométrie robuste à interférogramme unique selon une des revendications 9 ou 10, dans lequel l'interféromètre à deux ondes est aligné de sorte qu'en cas de différence de vitesse optique nulle, un anneau d'interférence d'ordre nul apparaît essentiellement au milieu de l'interférogramme spatial.
